# EUROPEAN PATENT SPECIFICATION

(11) **EP 4 344 686 B1**
(45) Date of publication and mention of the grant of the patent: **02.07.2025**
(21) Application number: 23200023.2
(22) Date of filing: 27.09.2023
(51) Int. Cl.: A61F 9/008

(54) **OPHTHALMIC LASER TREATMENT DEVICE**
OPHTHALMISCHES LASERBEHANDLUNGSGERÄT
DISPOSITIF DE TRAITEMENT AU LASER OPHTALMIQUE

(30) Priority: 28.09.2022 JP 2022154613; 28.09.2022 JP 2022154614; 05.07.2023 JP 2023110986; 31.08.2023 JP 2023141781; 31.08.2023 JP 2023141782
(43) Date of publication of application: 03.04.2024
(73) Proprietor: Nidek Co., Ltd., Gamagori-shi, Aichi, 443-0038 (JP)
(72) Inventor: TSUMURA, Takanori, Gamagori-shi, Aichi, 443-0038 (JP); ICHIKAWA, Naoki, Gamagori-shi, Aichi, 443-0038 (JP); MASUNAGA, Kohei, Gamagori-shi, Aichi, 443-0038 (JP); MIZUTA, Shota, Gamagori-shi, Aichi, 443-0038 (JP); TSUKAMOTO, Teruki, Gamagori-shi, Aichi, 443-0038 (JP)
(74) Representative: Viering, Jentschura & Partner mbB Patent- und Rechtsanwälte

(56) References cited:
- JP-A- 2017 086 154
- JP-A- 2022 059 854
- US-A1- 2002 165 525
- US-A1- 2006 224 147
- US-A1- 2011 245 815
- US-A1- 2014 094 783

## Description

### TECHNICAL FIELD

The present disclosure relates to an ophthalmic laser treatment device that treats tissue of a patient's eye by irradiating the tissue with treatment laser light.

### BACKGROUND

Techniques have been proposed to assist in treating patient's eyes using treatment laser light. For example, an ophthalmic laser treatment device disclosed in JP 2014-233469 A displays a target area to be irradiated with the treatment laser light on a display screen, and also displays an area that is actually irradiated with the treatment laser light (a post-irradiated area) when the treatment laser light is irradiated. The target irradiation area and the irradiated area are displayed for the purpose of allowing the operator to check the progress of the treatment. The ophthalmic laser therapeutic device disclosed in JP 2017-086154 is also considered relevant prior art knowledge.

### SUMMARY

For treating a patient's eye's tissue with a treatment laser light, a contact lens having a reflective surface to reflect the treatment laser light is sometimes used. For example, a contact lens having a reflective surface is sometimes used to treat a ring-shaped trabecular meshwork that exists in the area where the cornea and iris of the eyeball are in contact with each other. In this case, the operator needs to adjust the aiming point for the treatment laser light by observing the tissue reflected on the reflective surface of the contact lens while holding the contact lens. Therefore, the operator's skill is required to accurately adjust the aiming point. In view of the above, there has been a demand for a technique that can appropriately support treatment with a treatment laser light when a contact lens having a reflective surface is used.

A typical objective of the present disclosure is to provide an ophthalmic laser treatment device that is configured to appropriately support treatment with a treatment laser light when a contact lens having a reflective surface is used.

The present disclosure provides an ophthalmic laser treatment device that irradiates a tissue of a patient's eye with treatment laser light each time an instruction for performing laser irradiation is input. The device includes: a laser irradiation optical system that irradiates the patient's eye with treatment laser light; an observation optical system that allows an operator to observe an observation image of the patient's eye through an eyepiece; an internal display unit that is disposed in the observation optical system and is configured to display an image to the operator through the eyepiece; and a control unit that is configured to execute: a irradiation plan acquisition step to, when irradiating the patient's eye with treatment laser light using a contact lens having a reflective surface that reflects the treatment laser light in a direction intersecting an optical axis, acquire an irradiation plan that defines an order for irradiating a plurality of irradiation spots with the treatment laser light, the plurality of irradiation spots being scheduled to be irradiated with the treatment laser light; and an aiming guide display step to control the internal display unit to display an aiming guide in accordance with a progress of the irradiation plan to assist the operator in adjusting an aiming spot for the treatment laser light in an appropriate position.

According to the ophthalmic laser treatment device in the present disclosure, it is possible to appropriately support treatment with a treatment laser light when a contact lens having a reflective surface is used.

An ophthalmic laser treatment device exemplified in the present disclosure irradiates the tissue of a patient's eye with treatment laser light each time an instruction for executing laser irradiation is input. The ophthalmic laser treatment device includes a laser irradiation optical system, an observation optical system, an internal display unit, and a control unit. The laser irradiation optical system irradiates a patient's eye with a treatment laser light. The observation optical system allows an operator to observe an observation image of the patient's eye through an eyepiece. The internal display unit is provided in the observation optical system and displays images to the operator via the eyepiece. The control unit manages various controls of the ophthalmic laser treatment device. The control unit executes an irradiation plan acquisition step and an aiming guide display step. In the irradiation plan acquisition step, when irradiating the patient's eye with the treatment laser beam using a contact lens having a reflective surface that reflects the treatment laser light in a direction intersecting the optical axis, the control unit acquires an irradiation plan in which the order of irradiation of treatment laser light is set for a plurality of irradiation spots to be irradiated. In the aiming guide display step, the control unit controls the internal display unit to display an aiming guide that assists the operator in adjusting the aiming point of the laser light adjusted by the operator to an appropriate position in accordance with progress of the irradiation plan.

According to the ophthalmic laser treatment device in the present disclosure, an aiming guide for assisting the operator in adjusting the aiming point is displayed on the internal display unit according to progress of the irradiation plan. Therefore, the operator checks the aiming guide while observing the patient's eye through the eyepiece (in other words, without taking his eyes off the eyepiece). Then, using the confirmed aiming guide as a reference, the aiming point for the treatment laser light can be adjusted. Therefore, the operator can more appropriately adjust the aiming point for the treatment laser light.

In addition, if a treatment method that allows observation of treatment scars caused by treatment laser light is used, the operator can confirm the position of the treatment scars formed by previous laser irradiation, and then can adjust the aiming point of the treatment laser light for subsequent processes. However, a treatment method by which it is difficult to confirm the treatment scar caused by the treatment laser light (for example, a treatment method performed with a lower power of the treatment laser light than the power with which the treatment scars was formed) may be used. In this case, it would be more difficult to accurately adjust the aiming point to each of the plurality of irradiation spots at which the treatment laser lights plan to be irradiated. However, by adjusting the aiming point with reference to the aiming guide, the operator can easily adjust the aiming point more accurately even when it is difficult to confirm the treatment scar caused by the treatment laser light. It should be noted that the technology in this present disclosure is also very useful when treatment scars caused by treatment laser light can be observed.

The center of the display area in the internal display unit may be aligned with the optical axis of the observation optical system (hereinafter, may be referred to as an "observation optical axis"). In this case, the image is displayed on the display area of the internal display unit with the observation optical axis of the observation optical system as a reference. Therefore, the operator can appropriately perform treatment while recognizing the image presented at an appropriate position in the visual field.

Further, the optical axis of the treatment laser light irradiated by the laser irradiation optical system may be also aligned with the optical axis of the observation optical system. In this case, the control unit can display the image at an appropriate angle in an appropriate direction with the optical axis of the treatment laser light as a center. Furthermore, when the laser irradiation optical system irradiates the patient's eye with aiming laser light (hereinafter, may be sometimes referred to as "aiming light") to make the operator aware of the planned irradiation position for the treatment laser light, the optical axis of the aiming laser light may be also aligned with the optical axis of the observation optical system. In this case, since the operator visually recognizes the aiming light at the center of the observation field and the display area of the internal display unit, it is easier to adjust the irradiation position of the treatment laser light based on the aiming light and the displayed content.

The control unit may control the internal display unit to display a target location guide as the aiming guide on the internal display unit. The target location guide serves as a reference with which an arc-shaped or annular shaped treatment target location observed by the operator through the observation optical system is aligned. The operator can adjust the aiming point for the treatment laser light to an appropriate position by performing a various type of adjustments so as to cause the arc-shaped or annular-shaped treatment target location observed via the observation optical system to be aligned with the displayed target location guide.

The target location guide may include a straight-line portion that is in parallel with a tangential direction that is in contact with an appropriate position of the next aiming spot in the arc-shaped or annular-shaped treatment target location when viewed from the line of sight of the operator in the observation optical system. Focusing on a part of the circumference (i.e., an area having an angular range of less than 180 degrees when viewed from the center of the circle), the slope of the tangent line is uniquely determined at a specific point on the circumference. The angular range of the arc-shaped or annular-shaped treatment target location observed through the reflective surface of the contact lens is less than 180 degrees. Therefore, by aligning the treatment target location with the target location guide so that the tangential direction of the arc-shaped or annular-shaped treatment target location (for example, trabecular meshwork, etc.) is aligned with the direction of the straight-line portion of the target location guide, the aiming point for the treatment laser can be accurately adjusted. In addition, when the operator observes an arc-shaped or annular-shaped treatment target location through the observation optical system, the curvature of the treatment target location to be observed is changed according to the size of the patient's eye, the magnification of the observation optical system (may be referred to as a "observation magnification"), etc. However, the operator can align the tangential direction of the treatment target location with the direction of the straight-line portion of the target location guide, regardless of the observed curvature of the treatment target location. Therefore, the operator can more easily adjust the aiming point to an appropriate position.

The target location guide may include an arc-shaped portion corresponding to the arc-shaped arrangement of a plurality of irradiation spots determined under the irradiation plan. In this case, by aligning the treatment target location with the target location guide so that the arc shape drawn by the treatment target location matches the shape of the arc-shaped portion of the target location guide, the rotation angle of the reflective surface of the contact lens and the aiming point of the treatment laser light can be easily, accurately adjusted.

The control unit may change the curvature of the arc-shaped portion of the target location guide displayed on the internal display unit according to the magnification of the observation optical system. That is, the control unit may decrease the curvature of the arc-shaped portion of the target location guide as the magnification of the observation optical system increases. In this case, the arc-shaped portion of the target location guide changes appropriately in accordance with the difference in appearance of the treatment target location that changes depending on the observation magnification. Therefore, the aiming point can be adjusted more easily.

The control unit may switch between displaying and non-displaying of at least a portion (for example, a straight portion or an arc-shaped portion) of the target location guide on the internal display unit in accordance with an instruction input by the operator. In this case, when actually emitting the treatment laser light toward the irradiation spot, at least a portion of the target location guide is prevented from interfering with the treatment laser light.

Note that it is also possible to change the configuration of the target location guide. For example, the target location guide includes a straight-line portion that is, when viewed in a direction along line of sight of the operator in the observation optical system, parallel to the appropriate direction of reflection of the treatment laser light to the next irradiation spot by the reflective surface of the contact lens. In this case, the operator adjusts the rotation angle of the reflective surface of the contact lens so that the arc-shaped or annular-shaped treatment target location approaches the straight-line portion of the target location guide in a perpendicular direction. As a result, the rotation angle of the reflective surface can be adjusted appropriately.

The control unit determines the angle of the target location guide to be displayed on the internal display unit according to the progress of the irradiation plan, and then displays the target location guide at the determined angle. In this case, since the target location guide is displayed on the internal display unit at an appropriate angle according to the progress of the irradiation plan, the aiming point can be more appropriately adjusted.

In detail, at the start of treatment according to the irradiation plan, the control unit may determine the angle of the target location guide according to the position or direction of the irradiation spot that is in the first irradiation order determined by the irradiation plan.. Each time laser irradiation is executed (for example, each time a laser irradiation execution instruction is input), the control unit changes the angle of the displayed target location guide in accordance with the forward direction and the advancing angle of the irradiation spot. In this case, each time the laser irradiation is performed, the target location guide is displayed with an appropriate angle according to the next irradiation spot.

In addition, in a contact lens that has a reflective surface (may be referred to as a "goniolens"), there is a part rotatable lens where each time the user operates the contact lens with the user's finger, the part of the lens including the reflective surface rotates relative to the grip that is gripped by the user at a specified angle. When a partially rotatable lens is used, the control unit may acquire information about the specified rotation angle (that is, the angle of rotation by one operation) of the used partially rotatable lens. The control unit may change the angle of the displayed target location guide in the forward direction of the irradiation spot at a specified angle each time a predetermined number of laser irradiations set to be performed within one specified rotation angle are completed. In this case, each time the laser irradiation within one specified rotation angle range is completed, the target location guide is rotated to an angle corresponding to the next specified rotation angle range. Therefore, even when treatment is performed using a partially rotatable lens, the aim position can be adjusted more appropriately.

The control unit may change the displaying manner of the target location guides based on information regarding the contact lenses being used. For example, if the contact lens being used is a contact lens that can be rotated as a whole by the operator, the control unit 60 may rotate the target location guide 71A by the advancing angle of the irradiation spot each time the laser irradiation is performed. In addition, when the contact lens being used is a partially rotatable lens, the control unit may rotate the target location guide by a specified rotation angle each time the laser irradiation is completed a predetermined number of times. In this case, since the display of the target location guides is appropriately changed according to the contact lens being used, the aiming position of the treatment laser light can be easily adjusted.

In addition, even when using a contact lens that is entirely rotated by the operator rather than using a partially rotatable lens, the target location guide may be rotated by the specified rotation angle each time a predetermined number of laser irradiations are completed. In this case, by using the displayed target location guide, the operator can easily proceed with treatment using the same procedure as when using a partially rotatable lens. Further, the control unit may change the displaying method of the target location guide according to settings input by a user (for example, an operator).

The control unit may display, on the internal display unit, an angle guide, as an aiming guide, indicative of a rotation angle of the reflective surface of the contact lens suitable for the progress of the irradiation plan. In this case, the operator refers to the angle indicated by the angle guide and adjusts the rotation angle of the reflective surface of the contact lens, thereby adjusting the aiming point of the treatment laser light while the angle of the reflective surface is being appropriately adjusted. Therefore, the aiming point of the treatment laser light can be adjusted more easily.

The angle guide may include at least one of a shape that follows the appropriate angle of the arc-shaped or annular-shaped treatment target location of the patient's eye or a shape that follows an appropriate reflection direction of the laser light toward the next irradiation spot caused by the reflective surface of the contact lens. The control unit may determine the angle of the angle guide to be displayed on the internal display unit according to the progress of the irradiation plan, and display the angle guide at the determined angle. When the angle guide (for example, an arc-shaped schematic diagram of the treatment target location or an arc-shaped line along the treatment target location) that follows an appropriate angle of the treatment target location is displayed, the operator can easily adjust the rotation angle of the reflective surface of the contact lens by adjusting the angle of the treatment target location to be aligned with the angle guide. In addition, if the angle guide with a shape that follows an appropriate reflection direction of the treatment laser light is displayed, the operator can easily adjust the rotation angle of the reflective surface of the contact lens by adjusting the reflection direction of the treatment laser light to follow the direction of the angle guide.

Note that it is also possible to change the specific feature of the angle guide. For example, a contact lens schematic diagram showing an appropriate rotation angle of the contact lens to irradiate the next irradiation spot with the treatment laser light may be displayed as an angle guide. In this case, the operator can easily adjust the rotation angle of the contact lens by adjusting the rotation angle of the contact lens included in the observation field to the angle of the contact lens schematic diagram displayed as an angle guide.

In detail, at the start of treatment according to the irradiation plan, the control unit may determine the angle of the angle guide according to the position or direction of the irradiation spot that is in the first irradiation order determined by the irradiation plan.. Each time laser irradiation is executed (for example, each time a laser irradiation execution instruction is input), the control unit changes the angle of the displayed angle guide in accordance with the forward direction and the advancing angle of the irradiation spot. In this case, each time the laser irradiation is performed, the angle guide is displayed with an appropriate angle according to the next irradiation spot.

Furthermore, when a partially rotatable lens is used, the control unit may acquire information about the specified rotation angle (that is, the angle of rotation by one operation) of the used partially rotatable lens. The control unit may change the angle of the displayed angle guide in the forward direction of the irradiation spot at a specified angle each time a predetermined number of laser irradiations set to be performed within one specified rotation angle are completed. In this case, each time the laser irradiation within one specified rotation angle range is completed, the angle guide is rotated to an angle corresponding to the next specified rotation angle range. Therefore, even when treatment is performed using a partially rotatable lens, the aim position can be adjusted more appropriately.

The control unit may change the displaying manner of the angle guides based on information regarding the contact lenses being used. For example, if the contact lens being used is a contact lens that can be rotated as a whole by the operator, the control unit may rotate the angle guide by the advancing angle of the irradiation spot each time the laser irradiation is performed. In addition, when the contact lens being used is a partially rotatable lens, the control unit may rotate the angle guide by a specified rotation angle each time the laser irradiation is completed a predetermined number of times. In this case, since the display of the angle guide is appropriately changed according to the contact lens being used, the aiming position of the treatment laser light can be easily adjusted.

A part of the contact lens (for example, an inner wall, etc.) has a plurality of interval indicators (may be referred to as "indexes") placed at regular intervals to serve as a reference for the interval between spots to which irradiation with treatment laser light is performed. The angle guide may include a interval indicator schematic diagram that mimics the interval indicator of a contact lens. In this case, in addition to easily adjusting the rotation angle of the reflective surface of the contact lens using the angle guide, the operator can also adjust a plurality of aiming points by adjusting the aiming points of the treatment laser light while grasping the interval indicator schematic diagram. In particular, when the contact lens to be used is provided with the interval indicators, the operator can adjust the aiming point while comparing the interval indicators of the contact lens with the interval indicator schematic diagram displayed on the display unit. Thus, the aiming point can be adjusted more easily.

A spot arrangement guide map showing the arrangement of a plurality of consecutive irradiation spots including the next irradiation spot among the plurality of irradiation spots in the irradiation plan may be added to the angle guide. The control unit may shift the next irradiation spot to, among the plurality of irradiation spots included in the spot arrangement guide diagram, the irradiation spot adjacent in the forward direction determined by the irradiation plan each time the laser irradiation is performed. In this case, by grasping the position of the next irradiation spot displayed on the spot arrangement guide image, the operator can more appropriately adjust the next aiming point.

In addition, when an interval indicator schematic diagram is used, it is more preferable to display a spot arrangement guide diagram such that the spot arrangement guide diagram is aligned with reference intervals indicated by the interval indicator schematic diagram. In this case, the operator can more appropriately adjust the next aiming point by grasping the positional relationship of the next irradiation spot shown in the spot arrangement guide image relative to the interval indicator schematic image.

As described above, a partially rotatable contact lens may be used as a type of the contact lens. In the partially rotatable contact lens, a part including the reflective surface rotates at a specified angle relative to a grip that is held by a user each time the user makes an operation it one time by his/her finger. The control unit may perform a rotation recommendation step for recommending the operator to rotate the reflective surface of the contact lens by a specified angle each time a predetermined number of times of laser irradiations are completed within a single specified rotation angle range. In this case, especially when performing treatment using the partially rotatable lens, the operator can appropriately grasp the timing at which the reflective surface needs to be rotated by the specified angle.

Note that a specific method for recommending rotation of the reflective surface by the specified angle can be selected as appropriate. For example, the control unit may recommend rotation of the reflective surface using at least one of sound and vibration. Further, the control unit may recommend rotation of the reflective surface by controlling the internal display unit (for example, by rotating an image displayed on the internal display unit by a specified angle).

The control unit may control the internal display unit to display, as the irradiation guide, an outer circumferential guide along a circumference of the observation field observed by the operator through the observation optical system. The outer circumferential guide may indicate at least one of a rotation angle of the reflective surface of the contact lens suitable for the progress of the irradiation plan or a direction to the irradiation spot to which the laser light should be applied. In this case, the operator can easily grasp at least one of the appropriate rotation angle of the reflective surface or the direction to the irradiation spot by visually recognizing the outer circumferential guide displayed along the outer circumference of the observation field.

Note that the outer circumferential guide may be displayed in an arc shape or an annular shape along the outer circumference of the observation field. The center of the arc or annular outer circumferential guide may be aligned with the optical axis of the observation optical system. In this case, the direction indicated by the outer circumferential guide coincides with the direction from the center of the observation field of view. Therefore, the operator can appropriately grasp the direction indicated by the outer circumferential guide.

The outer circumferential guide may include a next aiming guide that indicates the direction to the next irradiation spot among the plurality of irradiation spots in the irradiation plan. Each time the laser irradiation is performed, the control unit may shift the position of the next aiming guide to a position corresponding to an irradiation spot that is determined in the irradiation plan and is located adjacent in a forward direction. In this case, the operator can appropriately grasp the direction to the next irradiation spot using the next aiming guide that is shifted along the outer circumference of the observation field.

The outer circumferential guide may include an irradiation completion guide that indicates the direction to an irradiation spot, among a plurality of irradiation spots in the irradiation plan, that has already been irradiated with the treatment laser light. The control unit may change the displayed next aiming guide to the irradiation completion guide each time the laser irradiation is executed. In this case, the operator can appropriately adjust the next aiming point using the next aiming guide by grasping the direction in which the laser irradiation has been completed. It also becomes easier to recognize the progress of treatment.

The outer circumferential guide may include a pre-irradiation guide that indicates the direction to an irradiation spot, among a plurality of irradiation spots in the irradiation plan, that is scheduled to be irradiated with treatment laser light later than the next time. Each time the laser irradiation is completed, the control unit replaces the pre-irradiation guide displayed in the direction of the irradiation spot in the next irradiation order determined in the irradiation plan with the next aiming guide. In this case, the operator can appropriately adjust the next aiming position using the next aiming guide by grasping the direction of the spot that is scheduled to be irradiated with the treatment laser light after the next time. It also becomes easier to recognize the progress of treatment.

The outer circumferential guide may include a section aiming guide. The section aiming guide may indicate a direction to an irradiation section that is a section formed of a plurality of irradiation spots onto which the laser light is applied while the reflective surface of the contact lens is being fixed at a rotation angle. The section aiming guide may also indicate a direction to a rotation angle of the reflective surface that is suitable for irradiation with the laser light onto the irradiation section. Each time the same number of laser irradiations as the number of the plurality of irradiation spots included in the irradiation section are completed, the control unit may shift the position of the section aiming guide to a position adjacent in the forward direction determined by the irradiation plan. In this case, the operator can easily grasp the direction to the plurality of irradiation sections or the direction to the reflective surface for irradiating the irradiation spots in the irradiation section with the treatment laser light using the section aiming guide. Therefore, it becomes easier to adjust the angle of the reflective surface of the contact lens.

The control unit may display the section aiming guide for each range defined by a specified angle in the partially rotatable lens. The control unit may shift the position of the section aiming guide to an adjacent position in the forward direction determined by the irradiation plan each time the same number of laser irradiations as the number of the plurality of irradiation spots included within one specified angle range are completed. In this case, the section aiming guide is appropriately displayed in accordance with the specifications of the partially rotatable lens and the progress of treatment. Therefore, even when the partially rotatable lens is used, the section aiming guide appropriately assists in adjusting the aiming point.

Note that it is also possible to change the specific configuration of the aiming guide. For example, the control unit may control the internal display unit to display an image of the treatment target location irradiated with aiming light. The treatment target location is captured by the imaging unit when the treatment laser light was emitted last time. The operator can adjust a next aiming point by grasping the location previously irradiated with the treatment laser light (that is, the region where the aiming light appears in the displayed image). Therefore, it becomes easier to irradiate the treatment laser light more appropriately.

Note that when displaying at least one of the above-described angle guide or the outer circumferential guide as an aiming guide, the ophthalmic laser treatment device may appropriately assist an operator in adjusting the aiming point even if the aiming guide is displayed by a display unit other than the internal display unit (for example, a display unit located outside of the observation optical system). In this case, the ophthalmic laser treatment device may also be defined as follows. An ophthalmic laser treatment device that irradiates a tissue of a patient's eye with a treatment laser light each time an instruction to perform irradiation with a treatment laser light is input, the ophthalmic laser treatment device including: a laser irradiation optical system that irradiates the patient's eye with the treatment laser light; an observation optical system that allows the operator to observe an observation image of the patient's eye through an eyepiece, a display unit that displays the image, and a control unit. The control unit is configured to perform an irradiation plan acquisition step and an aiming guide display step. At the irradiation plan acquisition step, the control unit acquires an irradiation plan defining an irradiation order in which the treatment laser light is applied to a plurality of irradiation spots that are scheduled to be irradiated by using a contact lens having a reflective surface that reflects the treatment laser light in a direction intersecting an optical axis. At the aiming guide display step, the control unit controls the display unit to display an aiming guide (at least one of an angle guide or an outer circumferential guide) in accordance with the progress of the irradiation plan. The aiming guide is displayed to assist the operator in adjusting the aiming spot for the treatment laser light to an apparatus position.

Furthermore, the ophthalmic laser treatment device may execute only a process of recommending an operator to rotate the reflective surface of the partially rotatable lens, without executing a process of displaying the aiming guide. In this case, the ophthalmic laser treatment device may also be defined as follows. An ophthalmic laser treatment device that irradiates a tissue of a patient's eye with a treatment laser light each time an instruction to perform irradiation with the treatment laser light is input, the laser irradiation optical system including: a laser irradiation optical system that irradiates the patient's eye with a therapeutic laser beam; an observation optical system that allows an operator to observe an observation image of the patient's eye through an eyepiece; and a control unit. In the ophthalmic laser treatment device, a partially rotatable lens is used as the contact lens having a reflective surface that reflects the treatment laser light in a direction intersecting an optical axis. The partially rotatable lens has a portion including the reflective surface and a grip that is gripped by the operator. The portion including the reflective surface is rotated by a specified angle relative to the grip each time the operator operates the partially rotatable lens one time. The control unit is configured to perform an irradiation plan acquisition step and a rotation recommendation step. At the irradiation plan acquisition step, the control unit is configured to acquire an irradiation plan defining an irradiation order in which the treatment laser light is applied onto a plurality of irradiation spots that are scheduled to be irradiated with the treatment laser light using the partially rotatable lens. At the rotation recommendation step, the control unit is configured to recommend the operator to rotate the reflective surface of the contact lens by a specified angle each time laser irradiation onto a specified angle area a specified number of times is completed.

The ophthalmic laser treatment device reflects treatment laser light on the reflective surface of the contact lens, and irradiates an annular-shaped or arc-shaped treatment target location of a patient's eye with the treatment laser light. The ophthalmic laser treatment device includes a laser irradiation optical system and a superimposing display unit. The laser irradiation optical system emits the treatment laser light. The superimposing display unit superimposes a circumferential chart indicating angles in a circumferential direction with the optical axis of the treatment laser light as a center on an observation image including at least a portion of the treatment target location. In this case, by visually recognizing the circumferential direction chart superimposed on the observation image, the operator can appropriately grasp the direction of the treatment target location with respect to the optical axis of the treatment laser light.

For example, the contact lens may be temporarily removed from the patient's eye during treatment on multiple spots. In this case, the operator should circle the treatment target location in the direction in which the spot irradiated with the treatment laser light immediately before removing the contact lens (the angle in the circumferential direction centered on the optical axis of the treatment laser light) is located. It can be properly understood by referring to the circumferential chart. Therefore, when reattaching a contact lens that has been removed to the patient's eye, the contact lens should be adjusted while referring to the circumferential chart so that the aiming point of the treatment laser light can be adjusted appropriately in the direction where the next spot will be located. The angle can be adjusted. Therefore, the operator can easily and appropriately adjust the aiming point of the treatment laser light.

The center of the circumferential chart superimposed on the observation image may coincide with the passing position of the optical axis of the treatment laser light in the observation image. In this case, the direction of the treatment target location with respect to the center of the optical axis of the treatment laser light can be more appropriately recognized using the circumferential chart.

The circumferential chart may include a plurality of indicators arranged at equal angles in a circumferential direction with the optical axis of the treatment laser light as a center. In this case, the plurality of indicators arranged at equal angles makes it easier for an operator to recognize the direction to the treatment target location more appropriately.

The superimposing display unit may display symbols (for example, numbers up to 12, etc.) each of which can be displayed in at least one of the directions of the dial of the analog clock at appropriate positions on the circumferential chart. In this case, the operator can appropriately grasp the direction of the treatment target location with respect to the optical axis of the treatment laser light, in the same sense as when grasping the time using an analog clock.

The superimposing display unit may fixedly display the circumferential chart at a predetermined position within the image display area. In this case, the circumferential chart is fixedly displayed at a predetermined position regardless of the state of the observation image, and thus the direction to the treatment target location with the optical axis of the treatment laser light as a center can be recognized more accurately. In this case, the relative positional relationship between the optical axis of the optical system for making the operator visually recognize the observation image (for example, the observation optical system described later or the optical system of the observation imaging unit) and the optical axis of the treatment laser light may be fixed. For example, the optical axis of the observation optical system and the optical axis O of the treatment laser light may always coincide with each other.

Various display units can be used as a superimposing display unit that displays the circumferential chart in a superimposed manner on the observation image. For example, the ophthalmic laser treatment device may include an observation optical system that causes the operator to observe an observation image including at least a portion of the treatment target location through an eyepiece. The superimposing display unit may be an internal display unit that displays images to an operator through an eyepiece. Further, the ophthalmic laser treatment device may include an observation and imaging unit that captures an observation image of the patient's eye. The superimposing display unit (for example, a monitor) may superimpose a circumferential chart on the observation image captured by the observation and imaging unit. The configuration of the superimposing display unit may be a simple configuration in which it is impossible to change the image displayed superimposed on the observation image.

The superimposing display unit may be configured to switch between superimposed display mode and non-display mode. When there is no need for the operator to grasp the direction of the treatment target location, the circumferential chart is not displayed so that the operator can concentrate on observing the observation image. For example, the control unit may determine whether the circumferential chart is to be displayed based on whether a treatment mode set by the operator is an annular treatment mode where treatment on an annular-shaped or arc-shaped treatment target location is performed. In this case, a field of view in accordance to the set treatment mode is appropriately provided to the operator.

An ophthalmic laser treatment device exemplified in the present disclosure irradiates the tissue of a patient's eye with treatment laser light each time an instruction for executing laser irradiation is input. The ophthalmic laser treatment device includes a laser irradiation optical system, an observation optical system, an internal display unit, and a control unit. The laser irradiation optical system irradiates a patient's eye with a treatment laser light. The observation optical system allows an operator to observe an observation image of the patient's eye through an eyepiece. The internal display unit is provided in the observation optical system and displays images to the operator via the eyepiece. The control unit manages various controls of the ophthalmic laser treatment device. The control unit executes an irradiation plan acquisition step and a spot interval guide display step. At the irradiation plan acquisition step, the control unit acquires an irradiation plan for irradiating a patient's eye with treatment laser light using a contact lens having a reflective surface that reflects the treatment laser light in a direction intersecting the optical axis. At the spot interval guide display step, the control unit controls the internal display unit to display a spot interval guide indicating appropriate intervals between a plurality of irradiation spots on which irradiation with treatment laser light is scheduled to be performed in accordance with the progress of the irradiation plan.

According to the ophthalmic laser treatment device according to the present disclosure, the spot interval guide indicating appropriate intervals between a plurality of irradiation spots is displayed on the internal display unit according to the progress of the irradiation plan. Therefore, the operator checks the spot interval guide while observing the patient's eye through the eyepiece (that is, without taking his eyes off the eyepiece), and adjusts the plurality of aiming points for the treatment laser light using the confirmed spot interval guide as a reference. Therefore, the operator can easily cause the interval between the irradiation spots, to which treatment laser light is applied, to be an appropriate interval. Note that the spot interval guide can also be treated as one example of the aiming guide that assists the operator in adjusting the aiming point for the treatment laser light to an appropriate position.

In addition, if a treatment method that allows observation of treatment scars caused by treatment laser light is used, the operator can confirm the position of the treatment scars formed by previous laser irradiation, and then can adjust the aiming point of the treatment laser light for subsequent processes. However, a treatment method by which it is difficult to confirm the treatment scar caused by the treatment laser light (for example, a treatment method performed with a lower power of the treatment laser light than the power with which the treatment scars was formed) may be used. In this case, it would be even more difficult to adjust the interval between the irradiation spots onto which the treatment laser light is applied to be an appropriate interval. However, by adjusting multiple aiming points with reference to the spot interval guide, the operator can easily adjust the intervals of the irradiation spots more accurately even when it is difficult to recognize the treatment scar caused by the treatment laser light. It should be noted that the technology in this present disclosure is also very useful when treatment scars caused by treatment laser light can be observed.

The control unit may change the entire spot interval guide displayed on the internal display unit according to the magnification of the observation optical system. That is, the control unit may increase the size of the spot interval guide as the magnification of the observation optical system increases. In this case, even if the observation magnification is changed, a spot interval guide with a size suitable for adjusting the intervals between the plurality of irradiation spots to be appropriate intervals is displayed on the internal display unit.

A part of the contact lens (for example, an inner wall, etc.) has a plurality of interval indicators (may be referred to as "indexes") placed at regular intervals to serve as a reference for the interval between spots to which irradiation with treatment laser light is performed. The control unit may cause the intervals of at least some of the plurality of indicators included in the spot interval guide to coincide with the intervals of the interval indicators of the contact lens. In this case, the operator adjusts the aiming points while referring to the position and the direction of the interval indicators observed through the observation optical system and the position and the direction of the spot interval guide displayed on the internal display. Thus, the operator can appropriately adjust the aiming points for the treatment laser light.

Depending on the type of contact lens, the aspect of the interval indicators provided on the contact lens (for example, the interval and number of interval indicators, etc.) may differ. Therefore, the control unit may change the displaying method of the spot interval guide (for example, the intervals and the number of the plurality of indicators in the spot interval guide) based on information regarding the contact lens being used. In this case, since an appropriate spot interval guide is displayed according to the interval indicator of the contact lens being used, the treatment can smoothly proceed.

The control unit may cause the intervals of at least some of the plurality of indicators included in the spot interval guide to coincide with appropriate intervals of the plurality of irradiation spots. In this case, the operator can easily cause the intervals of the irradiation spot for the treatment laser light to be appropriate intervals by aligning movement distance from the former aiming spot to the next aiming spot with the interval of the indicators included in the spot interval guide 90. Therefore, it becomes easier to perform treatment in accordance with the treatment plan more appropriately.

The spot interval guide may include a shape extending along an appropriate direction of reflection of the treatment laser light to the next irradiation spot by the reflective surface of the contact lens. The control unit may determine the angle of the spot interval guide to be displayed on the internal display unit according to the progress of the irradiation plan, and displays the spot interval guide at the determined angle. In this case, the operator can easily adjust the rotation angle of the reflective surface of the contact lens by adjusting the reflection angle of the treatment laser light to follow the direction indicated by the spot interval guide. Furthermore, since the spot interval guide is displayed on the internal display unit at an appropriate angle according to the progress of the irradiation plan, the aiming point can be more appropriately adjusted.

It should be noted that if the interval indicators are formed on the contact lens, the operator can easily adjust the rotation angle of the reflective surface of the contact lens by adjusting the rotation angle so that the interval indicator is located ahead of the direction indicated by the shape of the spot interval guide.

The control unit may display the spot interval guide at a position spaced apart from the aiming point of the treatment laser light in the display area of the internal display unit. The control unit may display ends of the plurality of indicators included in the spot interval guide that are close to the aiming point for the treatment laser light along a curve line that extends approximately along the curve of the arc-shaped treatment location of the patient's eye. In this case, the spot interval guide is displayed at a position different from the aiming point for the treatment laser light (that is, the spot interval guide does not overlap with the aiming point). Thus, the operator can set the irradiation position of the treatment laser light by appropriately recognizing the state of the tissue at the aiming point. Furthermore, by arranging the end of each of the plurality of indicators close to the aiming point along a curve line (i.e., in an arc shape), when the spot interval guide is placed at an appropriate position relative to the next aiming point, the distance between the aim point side end of each of the plurality of indicators and the arc-shaped treatment location decreases. As a result, by referring to the spot interval guide, the operator can more appropriately adjust the next aiming point of the treatment laser light.

Note that although the shape of the curve of the arc-shaped treatment site of a patient's eye varies slightly depending on the patient's eye, it does not vary significantly from patient to patient's eye. Therefore, the aiming point side end of each of the plurality of indicators may be displayed along a predetermined curve that is determined based on the average shape of the treatment area.

The control unit may display a next aiming indicator for aligning the position of the next irradiation spot in the spot interval guide with a position corresponding to the optical axis of the treatment laser light emitted by the laser irradiation optical system.. Each time the laser irradiation is executed, the control unit may shift the position of the next aiming index in the spot interval guide by one appropriate interval between the plurality of irradiation spots in the forward direction determined by the irradiation plan. In this case, the operator sets the position on the tissue to which the next treatment lase light is applied while considering the position of the next irradiation indicator on the optical axis of the treatment laser light (if the aiming light is emitted, the optical axis of the aiming light).

Among the plurality of indicators in the spot interval guide, the indicators other than the next aiming indicator 90A are post-irradiated indicators corresponding to the spots to which the treatment laser light has been applied or pre-irradiated indicators corresponding to the spots to which the treatment laser light has not been applied. The control unit may display each of the next aiming indicator, the post-irradiated indicator, and the pre-irradiated indicator in a different manner (that is, in a manner that makes each of these indicators distinguishable for the operator). In this case, the operator can easily grasp the positional relationship between the next aiming indicator, the post-irradiated indicator, and the pre-irradiated indicator, making it easier to proceed with the treatment more smoothly.

Each time the laser irradiation is executed, the control unit may shift the spot interval guide by one appropriate interval between the plurality of irradiation spots in a direction opposite to the forward direction determined by the irradiation plan. In this case, the operator adjusts the aiming point every time so that the position of the plurality of indicators that are shifted each time the treatment laser light is emitted is fixed on the observation image observed through the observation optical system. Thus, the aiming point can be adjusted more easily.

In addition, if the contact lens is provided with an interval indicator, the operator can easily adjust each of the plurality of aiming spots by appropriately adjusting a positional relationship between the interval indicators in the observation image and the plurality of indicators of the spot interval guide that is shifted each time laser light irradiation is performed. Even if an interval indicator is not formed in the contact lens, the operator can appropriately adjust each of the plurality of aiming points by appropriately adjusting the positional relationship between the characteristic parts present in the tissue included in the observation image and the plurality of indicators of the spot interval guide. For example, the operator identifies one characteristic region on the observation image, and each time the spot interval guide is shifted by one appropriate interval of the irradiation spot (that is, each time the laser irradiation is performed), at least one of a shifting instruction to the device or rotation operation of the contact lens may be performed such that a specific indicator among the indicators (for example, the most left side indicator or the second left side indicator) is aligned with the specified characteristic location. In other words, if the spot interval guide includes multiple indicators, each of the multiple aim positions can be adjusted appropriately by matching at least one of the multiple indicators (i.e., the specific indicator mentioned above) to the characteristic location. Note that when the relative position of the device with respect to the patient's eye moves, the position of the optical axis of the treatment laser light moves, and therefore the aiming point of the treatment laser light moves. Further, when the contact lens is rotated, the angle of the reflective surface of the contact lens changes, and thus the aiming point of the treatment laser light moves. In other words, in order to move the aiming point for the treatment laser light, the operator may perform at least one of a method of moving the relative position of the device with respect to the patient's eye or a method of rotating the contact lens.

The control unit may display at least one of the plurality of indicators included in the spot interval guide by arranging the indicators along a straight-line. For example, the control unit may display the centers of gravity of the plurality of indicators included in the spot interval guide by arranging the centers along a straight-line. Further, the control unit may display ends of the plurality of indicators included in the spot interval guide that are away from the aiming point for the treatment laser light by arranging the ends along a straight-line. The control unit may move the spot interval guide along a linear direction in which at least some of the plurality of indicators are arranged each time irradiation with the treatment laser light is performed. In this case, the operator can proceed with the treatment more appropriately by matching the direction in which the plurality of index elements are arranged with the movement direction in which the aiming point of the treatment laser light is moved to the next aiming point. For example, the reflective surface of a contact lens may include an edge or the like perpendicular to a direction extending outward from the central axis of the lens. In this case, the operator aligns the direction of the edge of the contact lens reflected in the observation image with the linear direction in which multiple index elements are arranged, and aims the treatment laser light along the direction of the edge. It is also possible to move it. In addition, even if the contact lens that was in contact with the patient's eye is once removed from the patient's eye, the operator can orient the edge of the contact lens in the observation image to a straight-line on which multiple index elements are lined up. It is also possible to easily restore the position and direction of the contact lens by adjusting the contact lens to the correct direction.

When an instruction to omit irradiation with the treatment laser light onto the next irradiation spot defined in the irradiation plan (hereinafter, referred to as a "skip instruction") is input, the control unit may shift the spot interval guide by one appropriate interval between the plurality of irradiation spots in the forward direction determined by the irradiation plan or a direction opposite to the forward direction. For example, if a non-irradiation area where irradiation with the treatment laser light should not be performed, the operator inputs the skip instruction to skip the irradiation with the treatment laser light for the non-irradiation area. Thus, the operator restarts treatment from the irradiation spot that is planned to be irradiated next. Therefore, it becomes easier for the treatment to proceed more smoothly.

The shape of the treatment target location of the patient's eye onto which the treatment laser light is applied may be arcuate or annular. The control unit may rotate the entire spot interval guide. In this case, by rotating the entire spot interval guide according to the shape of the arc-shaped or annular-shaped treatment target location, adjustment of the aiming position can be appropriately performed using the spot interval guide regardless of the difference in the circumferential position of the irradiation spot.

An irradiation section refers to an angle range of an arc area where laser irradiation will be performed specified times M (M>=2) based on the spot interval guide (that is, laser irradiation for each of a specified number of the irradiation spots). The control unit may rotate the spot interval guide in the forward direction as determined by the irradiation plan by setting one rotation angle of the spot interval guide to be the angle of the irradiation section. In this case, treatment with the treatment laser light for each irradiation section can be smoothly performed on the arc-shaped or annular-shaped treatment target location.

Further, the control unit may rotate the spot interval guide in the forward direction determined by the irradiation plan each time irradiation with the treatment laser light is executed. It is assumed that a treatment laser light is applied onto each of N equally spaced irradiation spots within the range of R degrees (R<=360) in an annular or arcuate treatment target location. In this case, the angle at which the spot interval guide is rotated each time irradiation with the treatment laser light is performed may be R/N degrees. In addition, the angle at which the spot interval guide is rotated each time laser irradiation is executed may be the rotation angle of the contact lens (a unit angle T) required to shift the aiming point for treatment laser light to the next irradiation spot. In this case, the operator can appropriately shift the aiming spot for the laser light to the next irradiation spot without adjusting the position of the optical axis of the laser light by simply rotating the contact lens at the unit angle as long as the optical path of the laser light does not deviate from the reflective surface of the contact lens. Note that the unit angle T is smaller than R/N degrees. The details will be described later.

Hereinafter, a pattern in which the relative position of the device with respect to the patient's eye is mainly shifted when adjusting the aiming point for each of a plurality of irradiation spots included in one irradiation section (hereinafter, referred to as a "shifting adjustment pattern") will be described below.

In the shifting adjustment pattern, if laser light irradiation onto all the irradiation spots in the irradiation section under treatment is not completed, the control unit may shift the spot interval guide in a direction opposite to the forward direction determined by the irradiation plan by one appropriate interval between the plurality of irradiation spots each time laser light irradiation is performed. In this case, the control unit may execute only a process of shifting the spot interval guide in parallel without rotating the plurality of irradiation spots. The control unit may rotate the entire spot interval guide in the forward direction by the angle of one irradiation section each time laser light irradiation onto all the plurality of irradiation spots in the irradiation section under treatment is completed. In this case, the operator can be appropriately assisted in performing, in accordance with the progress of the irradiation plan, laser light irradiation multiple times within the single irradiation section in which the irradiation position can be adjusted only by moving the device. Furthermore, irradiation with the treatment laser light onto each of the plurality of irradiation sections is automatically and appropriately assisted according to the progress of the irradiation plan.

The angle of the irradiation section can be selected as appropriate. For example, it is assumed that the range of R degree (R<=360) in a ring-shaped or arc-shaped treatment target location (for example, trabecular meshwork, etc.) is divided into S irradiation sections, and the aiming point is adjusted within each irradiation section using the shifting adjustment pattern. In this case, the control unit may rotate the entire spot interval guide in the forward direction by the angle of one irradiation section (i.e., R/S degrees) each time laser light irradiation onto all the plurality of irradiation spots in the irradiation section under treatment is completed. As a result, the laser irradiation is appropriately performed in each of the plurality of irradiation sections. **In** the shifting adjustment pattern, each irradiation section has an angular range in which treatment laser light irradiated can be performed a prescribed number of times (multiple times) based on the spot interval guide while the angle of the spot interval guide is fixed.

Note that, when the laser light irradiation onto all the plurality of irradiation spots in the irradiation section under treatment is completed, the control unit may execute only a process of rotating the entire spot interval guide by the angle of the single irradiation section without executing a process of shifting the spot interval guide by the appropriate interval when executing the laser light irradiation.

Next, a pattern where the operator mainly executes a rotational operation of the contact lens when adjusting the aiming point for each of the plurality of irradiation spots included in one irradiation section will be described (hereinafter, referred to as a "rotation adjustment pattern").

**In** the rotation adjustment pattern, if laser light irradiation onto all the irradiation spots in the irradiation section under treatment is not completed, the control unit may shift the spot interval guide in a direction opposite to the forward direction determined by the irradiation plan by one appropriate interval between the plurality of irradiation spots each time laser light irradiation is performed. Furthermore, the control unit may rotate the contact lens by a rotation angle (hereinafter, may be referred to as a "unit angle") required to shift the aiming spot for the laser light to the next irradiation spot in the forward direction each time laser light irradiation is performed. **In** this case, irradiation with the laser light multiple times in the single irradiation section is appropriately performed according to the progress of the irradiation plan by adjusting the aiming point by rotating the contact lens. Further, the control unit may adjust the angle of the entire spot interval guide to the angle corresponding to the next irradiation section (i.e., for the next irradiation section) by rotating the entire spot interval guide in the forward direction each time laser light irradiation onto all the plurality of irradiation spots within the irradiation section under treatment is completed. In this case, irradiation with the treatment laser light onto each of the plurality of irradiation sections is automatically and appropriately assisted according to the progress of the irradiation plan.

In addition, if the laser irradiation onto all the plurality of irradiation spots in the irradiation section under treatment is not completed, the control unit may execute only a process of rotating the spot interval guide by the unit angle each time laser light irradiation is executed without executing a process of shifting the spot interval guide by the appropriate interval between the plurality of irradiation spots. Even in this case, the operator can easily adjust the aiming spot to the next irradiation spot by rotating the contact lens.

It should be noted that, when the relative position of the device with respect to the patient's eye is not moved, the rotation angle (i.e., the unit angle) of the contact lens required to move the aiming point for the treatment laser light to the next irradiation spot may change depending on various conditions. Here, it is assumed that laser irradiation is performed N times at equal intervals within the range of R degrees (R<=360) in an annular-shaped or arc-shaped treatment target location. In addition, it is assumed that a virtual approximate circle (that is, a circle through which a plurality of planned irradiation spots passes) passing through a annular-shaped or arc-shaped treatment target location. When the patient's eye is viewed in a direction along the visual axis, if the treatment laser light reflected by the reflective surface of the contact lens always intersects the center of the approximate circle, the unit angle will be R/N degrees. In this case, when the patient's eye is viewed in a direction along the visual axis, the radius of gyration of the treatment laser light is equal to the radius of the approximate circle.

However, if the aiming point is shifted to the next irradiation spot only by rotating the contact lens without moving the relative position of the device with respect to the patient's eye, the laser light does not always intersect the center of the approximate circle. That is, in the rotation adjustment pattern, the treatment laser light is rotated around a reflection position on the reflective surface of the contact lens. In this case, when the patient's eye is viewed in a direction along the visual axis, the radius of gyration of the treatment laser light is longer than the radius of the above-described approximate circle. As a result, the unit angle T in the rotation adjustment pattern when the eye to be examined is viewed from the direction along the visual axis becomes smaller than R/N degrees. Therefore, the unit angle T in the rotation adjustment pattern may be set, within a range smaller than R/N degrees, in accordance with the turning radius of the laser light when viewed in the visual axis direction of the patient's eye (i.e., a distance between the reflective position in the reflective surface of the contact lens and the position at which the laser light is applied.

The angle at which the entire spot interval guide is rotated when irradiation to all the plurality of irradiation spots in the irradiation section under treatment with the treatment laser light is completed can also be selected as appropriate. For example, assume that the range of R degree (R<=360) in an annular or arcuate treatment region is divided into S irradiation sections, and each irradiation section is irradiated with the treatment laser light M times. During treatment in one irradiation section, when irradiation with the treatment laser light is completed M times, the total value of the rotation angle of the spot interval guide is (T x (M - 1)) degree. Further, the angular range of one irradiation section is R/S degrees. Therefore, when irradiation with the treatment laser light onto all the plurality of irradiation spots in the irradiation section under treatment is completed, the control unit may rotate the spot interval guide by (R/S-T*(M-1)) degrees. In this case, the entire angle of the spot interval guide is adjusted appropriately to the angle corresponding to the next irradiation section.

In addition, the control unit may only rotate the aiming spot for the laser light by the rotation angle of the contact lens required to shift the aiming point to the next irradiation spot (i.e., the above-described unit angle T) without shifting the spot interval guide by the appropriate interval between the plurality of spot interval guide each time the laser light irradiation is performed.

For example, if irradiation with the treatment laser light has not been completed on all the plurality of irradiation spots in the irradiation section under treatment, the control unit may rotate the aiming spot for the laser light by the rotation angle (the above-described unit angle) required to shift the aiming point to the next irradiation spot without shifting the spot interval guide by the appropriate interval each time the laser light irradiation is performed. The control unit may adjust the angle of the entire spot interval guide to the angle corresponding to the next irradiation section (i.e., for the next irradiation section) by rotating the entire spot interval guide in the forward direction each time laser light irradiation onto all the plurality of irradiation spots within the irradiation section under treatment is completed. In this case, irradiation with the treatment laser light onto each of the plurality of irradiation sections is automatically and appropriately assisted according to the progress of the irradiation plan.

In the rotation adjustment pattern, when a skip instruction is input, the control unit may shift the spot interval guide in the forward direction determined in the irradiation plan or in a direction opposite to the forward direction by the appropriate interval between the plurality of irradiation spots Furthermore, when the skip instruction is input, the control unit may rotate the spot interval guide in the forward direction by the rotation angle of the contact lens required to shift the aiming spot to the next irradiation spot. In this case, the operator can skip irradiation with the treatment laser light to the non-irradiated area.

As mentioned above, it may be also possible to rotate the spot interval guide by the unit angle T each time laser irradiation is performed without shifting the spot interval guide by one appropriate interval between multiple irradiation spots. **In** this case, when the skip instruction is input, the control unit may rotate the spot interval guide by the unit angle T without performing laser irradiation.

When the control unit receives an instruction to rotate the entire spot interval guide to the next irradiation section before the laser irradiation onto all the plurality of irradiation spots in the irradiation section under treatment is completed, the entire spot interval guide may be rotated by an angle corresponding to the range where the treatment has been done within the irradiation section being treated. In this case, even if the treatment for the irradiation section is not completely completed, the operator can move forward to treatment for the next irradiation section by rotating the entire spot interval guide. Therefore, it becomes easier for the treatment to proceed more smoothly.

Note that the method for calculating the angle when rotating the spot interval guide to the next irradiation section can be selected as appropriate. For example, the angle between two adjacent irradiation spots is A degrees when viewed from the center of a circle through which multiple planned irradiation spots pass, and the number of the irradiation spots for which treatment has been done within the irradiation section under treatment is m. The angle corresponding to the range in which the treatment has been done within the irradiation section being treated may be calculated by "A (degrees) x m (number)". The number of irradiation spots "m" for which treatment has been done may also include the number of irradiation spots for which irradiation with the treatment laser light was skipped in accordance with the above-described skip instruction.

When displaying the next aiming indicator for aligning the position of the next irradiation spot in the spot interval guide, the control unit may set an end of the indicators in a direction opposite to the forward direction of treatment to the position of the next irradiation indicator each time the plurality of indicators are rotated to the next irradiation section. In this case, the position of the next aiming indicator is appropriately changed according to the progress of the irradiation plan, making it easier for the treatment to proceed more smoothly. Furthermore, when displaying each of the next aiming indicator, the post-irradiated indicator, and the pre-irradiated indicator in a distinguishable manner, the control unit may set all the indicators except the next irradiation indicator as the pre-irradiated indicators each time the plurality of indicators are rotated to the next irradiation section.

Note that it is also possible to change the specific method for rotating the entire spot interval guide (for example, a plurality of indicators in the spot interval guide). For example, the control unit may rotate the spot interval guide by the angle of the irradiation section each time a rotation instruction is input by a user (for example, a operator or an assistant). Further, the rotation angle of the spot interval guide may be set by a user.

### BRIEF DESCRIPTION OF DRAWINGS

FIG. 1 is an external view of an ophthalmological laser treatment device.
FIG. 2 is a side view of an optical system of the ophthalmic laser treatment device.
FIG. 3 is a diagram of the optical system of the ophthalmic laser treatment device when viewed from an upper side.
FIG. 4 is a schematic cross-sectional view of a patient's eye and a contact lens.
FIG. 5 is a diagram schematically showing an example of an observation location observed through a reflective surface of the contact lens.
FIG. 6 is an exploded perspective view of the contact lens that is a partially rotated lens.
FIG. 7 is a view of the contact lens provided with an interval index when viewed from a side opposite to the side that is in contact with the patient's eye.
FIG. 8 is a diagram showing an example of an irradiation plan displayed on a control box.
FIG. 9 is an explanatory diagram for explaining a method of adjusting an aiming point of an irradiation spot.
FIG. 10 is a diagram showing an example of an operator's observation field of view during treatment by the ophthalmic laser treatment device according to a first embodiment.
FIG. 11 is a flowchart of treatment control processing executed by the ophthalmic laser treatment device according to the first embodiment.
FIG. 12 is a diagram showing an example of transition of the operator's observation field of view during treatment by an ophthalmic laser treatment device according to a second embodiment.
FIG. 13 is a flowchart of treatment control processing executed by the ophthalmic laser treatment device according to the second embodiment.
FIG. 14 is a diagram showing an example of an operator's observation field of view during treatment by an ophthalmic laser treatment device according to a third embodiment.
FIG. 15 is an enlarged view of an angle guide shown in FIG. 13.
FIG. 16 is a flowchart of treatment control processing executed by the ophthalmic laser treatment device according to the third embodiment.
FIG. 17 is a diagram showing an example of transition of an operator's observation field of view during treatment by an ophthalmic laser treatment device according to a fourth embodiment.
FIG. 18 is a diagram showing an example of transition of the operator's observation field of view during treatment by the ophthalmic laser treatment device according to the fourth embodiment.
FIG. 19 is a flowchart of treatment control processing executed by the ophthalmic laser treatment device according to the fourth embodiment.
FIG. 20 is a diagram showing an example of an operator's observation field of view during treatment by an ophthalmic laser treatment device according to a fifth embodiment.
FIG. 21 is a diagram showing an example of transition of a part of the operator's observation field during treatment by the ophthalmic laser treatment device according to the fifth embodiment.
FIG. 22 is a diagram showing an example of transition of a part of the operator's observation field during treatment by the ophthalmic laser treatment device according to the fifth embodiment.
FIG. 23 is a flowchart of treatment control processing executed by the ophthalmic laser treatment device according to the fifth embodiment.
FIG. 24 is a diagram showing an example of transition of a part of the operator's observation field during treatment by the ophthalmic laser treatment device according to the sixth embodiment.
FIG. 25 is a diagram showing an example of transition of a part of the operator's observation field during treatment by the ophthalmic laser treatment device according to the sixth embodiment.
FIG. 26 is a diagram schematically showing an example of transition of an optical path of the treatment laser light according to the sixth embodiment.
FIG. 27 is a flowchart of treatment control processing executed by the ophthalmic laser treatment device according to the sixth embodiment.
FIG. 28 is a diagram showing an example of an operator's observation field of view during treatment by an ophthalmic laser treatment device according to a seventh embodiment.
FIG. 29 is a diagram showing an example of a modification of a spot interval guide.
FIG. 30 is a diagram showing a modification example of the spot interval guide of the fourth embodiment.

### DETAILED DESCRIPTION

### <Embodiment>

Hereinafter, a plurality of typical embodiments according to the present disclosure will be described with reference to the drawings. An ophthalmic laser treatment device 1 in this embodiment can perform treatment of a patient's eye E by irradiating the patient's eye E with treatment laser light.

### <Overall configuration>

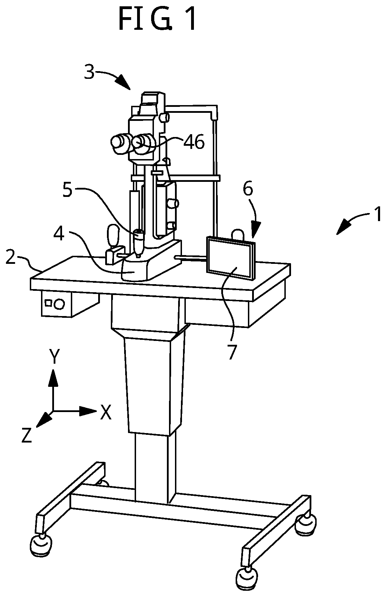

The configuration of the ophthalmic laser treatment device 1 will be described with reference to FIGS. 1 to 3. As shown in FIG. 1, the ophthalmic laser treatment device 1 according to this embodiment includes a table 2, a main body 3, and a control box 6. The main body 3 and the control box 6 are disposed on the table 2.

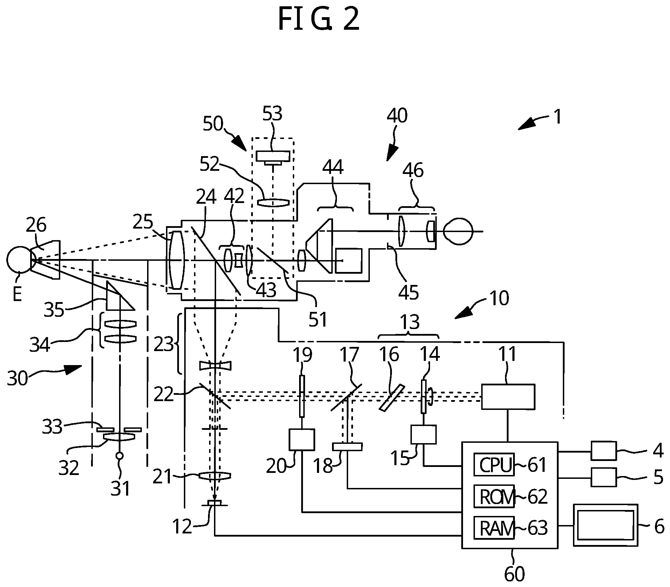

The main body 3 includes various components such as a laser irradiation optical system 10, an illumination optical system 30, an observation optical system 40, an internal display unit 50, and a control unit 60 (see FIG. 2), which will be described later. Further, the main body 3 includes a base 4 and a joystick 5 (i.e., an operation lever). The base 4 is a displacement machine having a displacement mechanism, and moves at least one of the laser irradiation optical system 10, the observation optical system 40, the internal display unit 50, etc. in a vertical direction (Y direction in FIG. 1) and in a horizontal direction. (in X direction in FIG. 1), and in a front-back direction (in Z direction in FIG. 1). The displacement machine changes the positional relationship between the patient's eye and the laser irradiation optical system 10 in an up-down direction, left-right direction, and front-back direction. The base 4 can further rotate at least one of the laser irradiation optical system 10, the observation optical system 40, the internal display unit 50, etc. in the horizontal direction about an axis extending in the vertical direction. The operator operates the joystick 5 to move or rotate the laser irradiation optical system 10, the observation optical system 40, the internal display unit 50, etc., thereby adjusting the observation position of the patient's eye E and the irradiation position of the laser beam (the treatment laser light and aiming beam). Note that the joystick 5 in this embodiment (for example, the upper end of the joystick 5) is provided with an operation button for operation by a operator. In this embodiment, the operation button of the joystick 5 is used as trigger input means for inputting a trigger for irradiating the treatment laser light. Note that a foot switch or the like operated by the operator's foot may be used as trigger input means for inputting a trigger for executing laser irradiation.

The control box 6 includes an external display 7 provided outside of the observation optical system 40 (see FIG. 2). The external display 7 can display various images. A touch panel type operation section is formed on a surface of the external display 7 of the control box 6. The control box 6 displays various parameters related to treatment on the external display 7 and receives inputs of various instructions from a user.

### <Laser irradiation optical system>

As shown in FIG. 2, the laser irradiation optical system 10 includes in this embodiment includes a treatment laser light source 11, an aiming light source 12, an energy adjustment unit 13, a beam splitter 17, a photodetector 18, a safety shutter 19, a collimator lens 21, a dichroic mirror 22, an expander lens 23, a dichroic mirror 24, and an objective lens 25.

The treatment laser light source 11 emits treatment laser light for treating the tissue of the patient's eye E. As an example, in the laser light source 11 in this embodiment, neodymium-doped YAG (yttrium aluminum garnet) crystal (Nd:YAG) is used as the laser rod. Further, a wavelength conversion element (not shown) is configured to convert infrared laser light (wavelength: 1064 nm) emitted by the laser light source 11 into visible laser light (wavelength: 532 nm).

The aiming light source 12 emits aiming laser light (hereinafter, simply referred to as "aiming light") indicative of the position to be irradiated with the treatment laser light (that is, the position of the irradiation spot). In this embodiment, a light source that emits visible laser light with a wavelength of 635 nm (red) is used as the aiming light source 12. However, the wavelength of the aiming light may be changed as appropriate.

The energy adjustment unit 13 adjusts the amount of energy of the treatment laser light irradiated onto the tissue of the patient's eye E. The energy adjustment unit 13 in this embodiment includes a half-wave wavelength plate 14 and a polarizing plate 16. The half-wave plate 14 is rotated by a motor 15 about the optical axis of the treatment laser light. The polarizing plate 16 is arranged at Brewster's angle. The amount of energy of the treatment laser light is adjusted by the combination of the 1/2 wavelength plate 14 and the polarizing plate 16.

The beam splitter 17 reflects a portion of the treatment laser light toward the photodetector 18. The photodetector 18 detects an amount of energy of the treatment laser light by receiving the treatment laser light reflected by the beam splitter 17. The safety shutter 19 is moved between a position on the optical axis of the treatment laser light and a position outside of the optical axis by a shutter driving unit (for example, a solenoid) 20. When the safety shutter 19 is located on the optical axis of the treatment laser light, the safety shutter 19 blocks irradiation of the patient's eye E with the treatment laser light.

The collimator lens 21 converts the aiming light emitted by the aiming light source 12 into a parallel light beam. The dichroic mirror 22 has a light axis coaxial with the optical axes of the treatment laser light and the aiming light and is configured to multiplex the treatment laser light and the aiming light. The dichroic mirror 22 in this embodiment reflects the treatment laser light and transmits the aiming light, thereby combining the treatment laser light and the aiming light.

The expander lens 23 expands the beam of laser light (treatment laser light and aiming light) combined by the dichroic mirror 22. The laser beam expanded by the expander lens 23 is reflected by the dichroic mirror 24 and transmitted through the objective lens 25. In this embodiment, the laser light transmitted through the objective lens 25 is irradiated onto the tissue of the patient's eye E via the contact lens 26 attached to the patient's eye E. The dichroic mirror 24 reflects almost all the reflected light of the treatment laser light reflected by the patient's eye E so that the reflected light is less likely to enter the operator's eye. Note that the irradiation optical system 10 may be provided with a mechanism for adjusting the spot size of the laser light irradiated onto the tissue.

### <Illumination optical system>

The illumination optical system 30 illuminates the observation location including the treatment target site of the tissue. The illumination optical system 30 in this embodiment includes a lamp 31, a lens 32, an aperture 33, a lens group 34, and a prism 35. For example, a white light emitting element or the like may be used as the lamp 31. Note that the illumination optical system 30 may include a slit plate or the like for illuminating the observation location with slit light.

### <Observation optical system>

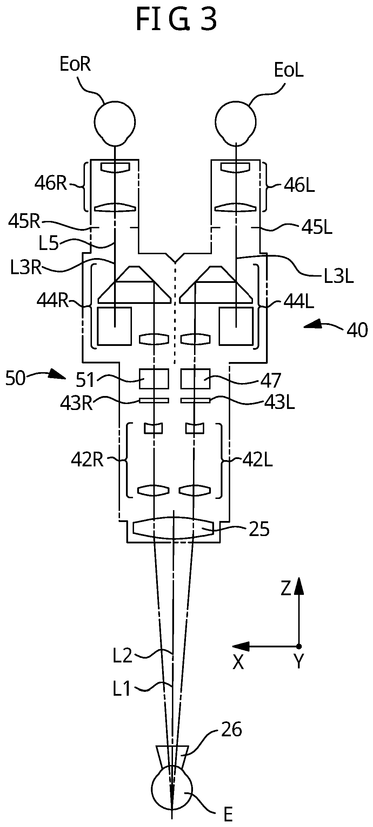

As shown in FIGS. 2 and 3, the observation optical system 40 is observation means for allowing the operator to observe the patient's eye E, and includes an optical axis L3 (see FIG. 3). As shown in FIG. 3, the observation optical system 40 in this embodiment has an optical axis L3R for presenting an observation image to the operator's right eye EoR and an optical axis L3R for presenting an observation image to the operator's left eye EoL. and an optical axis L3L. The observation optical system 30 in this embodiment may be binoculars. The observation optical system 40 in this embodiment includes an objective lens 25, a variable magnification optical system 42 (42R, 42L), a protective filter 43 (43R, 43L), a half mirror 47, an erecting prism group 44 (44R, 44L), and a visual field aperture 45 (45R, 45L), an eyepiece 46 (46R, 46L), and the like. The operator looks through the eyepiece 46 and checks the observation location of the patient's eye E, the spot of the aiming light (in other words, the reflected light (return light) of the aiming light reflected by the patient's eye E), etc. In this embodiment, an observation surface (object surface) disposed at the tip of the objective lens 41 and a field stop 45 disposed inside the device are in an optically conjugate positional relationship via the objective lens 41. That is, at the position of the field stop 45, an observation image of the patient's eye E is formed as an aerial image (a virtual image). Note that in this embodiment, the magnification of the observation image observed by the operator is changed by the variable magnification optical system 42. The observation optical system 40 is provided with an encoder (not shown) for obtaining the magnification of the observation image by the variable magnification optical system 42.

### <Internal display unit>

As shown in FIG. 2, the internal display unit 50 is disposed in the observation optical system 40 and displays images to the operator via the eyepiece 46. The internal display unit 50 includes a display 53, a lens 52, and a half mirror 51. Various images are displayed on the display 53. In this embodiment, an LCD (with a backlight) is used as the display 53. Specifically, in this embodiment, a color LCD capable of displaying 1600 (H) x 1200 (V) is used as the display 53. As shown in FIG. 3, the half mirror 51 is arranged on the optical axis L3R. Specifically, the half mirror 51 is arranged between the protective filter 43R and the erecting prism group 44R.

Presentation light (display light) emitted from the display 53 travels along the optical axis L5 (see FIG. 3). Specifically, the presentation light emitted from the display 53 passes through the lens 52 and is then reflected by the half mirror 51 in a direction toward the erecting prism group 44R. The half mirror 51 in this embodiment is combining means for combining the optical observation image observed by the observation optical system 40 and the image displayed on the display 53. The half mirror 51 in this embodiment has an axis coaxial with the optical axis L5 and the optical axis L3R. The presentation light reflected by the half mirror 51 passes through the erecting prism group 44R, the field stop 45R, and the eyepiece lens 46R in this order, and is focused on the fundus of the operator's eye looking through the eyepiece lens 46R. The internal display unit 50 functions as a so-called head-up display (HUD).

In this embodiment, the display 53 and the field stop 45R are in an optically conjugate positional relationship. In other words, the image displayed on the display 53 is formed as an aerial image at the position of the field stop 45R. Note that the method of displaying the image to the operator through the eyepiece lens 46 is not necessarily limited to the method exemplified in this disclosure. For example, an LCD (liquid crystal panel) without a backlight is arranged as an internal display unit at the position of the field stop 45R (i.e., on the optical axis L3R), and the control unit 60 may control the transmittance of each cell constituting the LCD to present presentation information to the operator.

In this embodiment, the center of the display area in the internal display unit 50 is aligned with the observation optical axis of the observation optical system 40. Therefore, an image is displayed in the display area of the internal display unit 50 with the observation optical axis of the observation optical system 40 as a reference. Therefore, the operator can appropriately perform treatment while recognizing the image presented at an appropriate position in the observation field through the eyepiece lens 46.

Further, in this embodiment, the optical axis of the treatment laser light irradiated by the laser irradiation optical system 10 is also aligned with the optical axis of the observation optical system 40 and the center of the display area in the internal display unit 50. Therefore, the internal display unit 50 can display an image at an appropriate angle in an appropriate direction with the optical axis of the treatment laser light as a center. Furthermore, in this embodiment, the optical axis of the aiming light irradiated by the laser irradiation optical system 10 is also aligned with the optical axis of the observation optical system 40 and the center of the display area in the internal display unit 50. Therefore, since the operator visually recognizes the aiming light at the center of the observation field of view and the center of the display area of the internal display unit 50, the irradiation position of the treatment laser light is easily adjusted based on the aiming light and the displayed contents by the internal display unit 50.

Although not shown in the drawings, the observation optical system 40 in this embodiment incorporates an imaging optical system for capturing an observation image of the patient's eye E and the like. The imaging optical system includes a half mirror, an imaging lens, and an imaging element. The half mirror is disposed on either a left-side observation optical path or a right-side observation optical path disposed in the observation optical system 40. Light that enters the half mirror from the observation location or the like via the objective lens 25 is reflected by the half mirror, and enters the imaging element via the imaging lens. As a result, an observation image is captured by the imaging optical system.

### <Control unit>

The control unit 60 manages various controls of the laser treatment device 1. The control unit 60 in this embodiment includes a CPU (processor) 61, a ROM 62, a RAM 63, a nonvolatile memory 65, and the like. The CPU 61 controls each component of the laser treatment device 1. The ROM 62 stores various programs, initial values, and the like. The RAM 63 temporarily stores various information. The nonvolatile memory 65 is a non-transitory storage medium that is configured to retain stored contents even if the power supply turned off. For example, a USB memory that is removably attached to the control unit 60, a flash ROM built into the control unit 60, or the like may be used as the nonvolatile memory 65. In this embodiment, the control unit 60 is connected to the base unit 4, the joystick 5, the control box 6, the laser light source 11, the aiming light source 12, the motor 15, the photodetector 18, the shutter drive unit 20, the lamp 31, and the display device. 53 etc. are connected.

### <Treatment manner for trabecular meshwork>

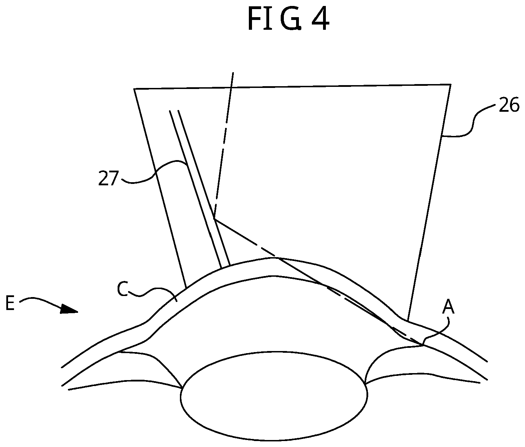

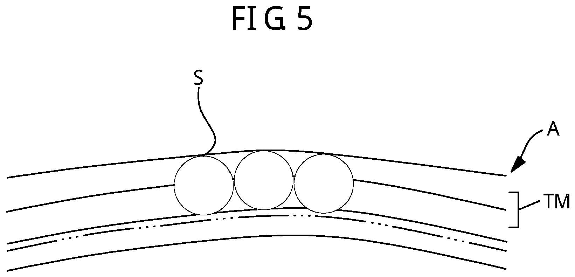

An example of a treatment manner for trabecular meshwork performed by the ophthalmic laser treatment device 1 in this embodiment will be described with reference to FIGS. 4 and 5. In this embodiment, the trabecular meshwork, which is a ring-shaped (partially, arc-shaped) tissue of the patient's eye E, is the target site for treatment. For example, a selective laser trabeculoplasty (SLT) is a treatment method that involves irradiating the trabecular meshwork at the angle of the patient's eye E with treatment laser light in order to increase the drainage of aqueous humor in the patient's eye E. In SLT, the treatment laser light is irradiated multiple times over the entire circumference or part of the annular-shape trabecular meshwork.

As shown in FIG. 4, in the treatment of trabecular meshwork in this embodiment, the contact lens 26 is attached to the cornea C of the patient's eye E. As the contact lens 26, for example, a gonioscope or a Goldmann three-sided mirror for observing the corner A of the patient's eye E may be used. The contact lens 26 is provided with a reflective surface (i.e., a reflective mirror) 27. The corner A is observed through the reflective surface 27. Therefore, as shown in FIG. 5, in this embodiment, the entire corner A (the entire circumference) is not observed at the same time, but a portion of the corner A is observed in a fan shape (a sector shape). The range of the fan-shaped portion of the annular corner A that is observed through the reflective surface 27 falls within an angular range of less than 180 degrees with the center of the annular corner A as a reference. In addition, the reflective surface 27 of the contact lens 26 reflects the treatment laser light and aiming light in a direction intersecting the optical axis extending from the objective lens 25 (see FIGS. 2 and 3) toward the patient's eye E, thereby, irradiating the trabecular meshwork TM, which is the treatment target site, is irradiated with the treatment laser light and the aiming light. That is, in this embodiment, the trabecular meshwork is treated by irradiating the trabecular meshwork TM at the corner A with the treatment laser light, as illustrated in FIG. 5.

By adjusting the rotation angle of the contact lens 26 (that is, the angle of the rotation direction about the axis of the contact lens 26), the operator can adjust a reflective direction of the treatment laser light by the reflective surface 27 of the contact lens 26 as viewed from the operator's line of sight in the observation optical system 40. In addition, the operator operates the joystick 5 to move the base 4 to adjust the aiming point of the treatment laser light and the aiming light with respect to the tissue of the patient's eye E to a target spot S to be treated. After the adjustment of the aiming point is completed, the operator inputs an instruction to irradiate the treatment laser light by operating the operation button of the joystick 5 or the foot switch, thereby irradiating the spot S with the treatment laser light. Note that in SLT, treatment laser light is emitted with a lower output (energy and irradiation time) than in argon laser trabeculoplasty (ALT) in order not to cause thermal degeneration of the tissue. Therefore, it is difficult for the operator to visually recognize changes in the condition of the treated area (for example, treatment scars, etc.) before and after the surgery. In addition, as a SLT procedure, there has been known that the spot size of the treatment laser light and the aiming light is fixed to a predetermined size (for example, 400 µm, etc.), and the treatment laser light is intermittently emitted so that multiple irradiation spots are adjacent to each other along the trabecular meshwork TM. FIG. 5 schematically shows part of a procedure in which treatment laser light is intermittently emitted so that a plurality of irradiation spots are arranged adjacent to each other. Note that in SLT, it is difficult to visually observe treatment scars. That is, the irradiated spot (spot S) irradiated with the treatment laser light is not visually observed by the operator as shown in FIG. 5.

### <Contact lens>

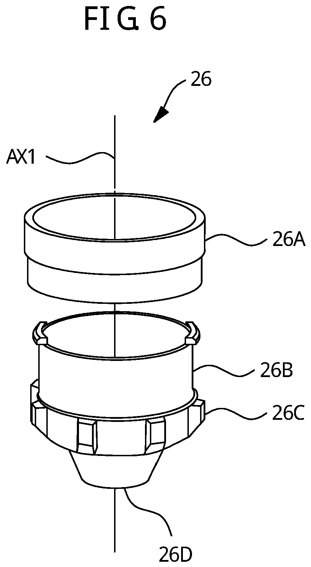

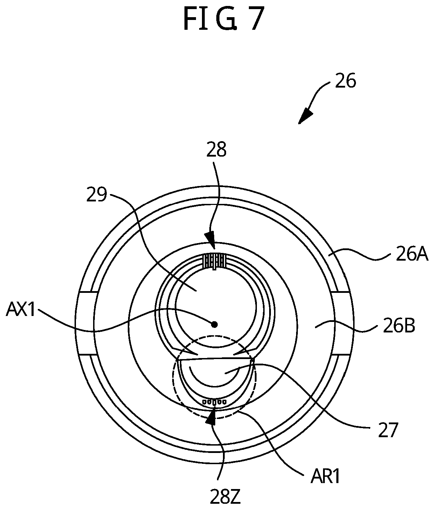

An example of the contact lens 26 used in the treatment of trabecular meshwork in this embodiment will be described with reference to FIGS. 6 and 7. The contact lens 26 shown in FIGS. 6 and 7 is a partially-rotatable-type lens. In the partially-rotatable-type lens, each time the lens is operated by the user's (operator's) finger, the portion including the reflective surface 27 rotates by a specified angle with respect to a grip 26A held by the user. That is, when the user performs one operation with a finger, a portion including the reflective surface 27 rotates in the circumferential direction about the axis AX1 independently of other portions.

Specifically, the contact lens 26 shown in FIG. 6 includes an annular grip 26A and a rotation base 26B. The grip 26A has a substantially cylindrical shape and is gripped by a plurality of fingers of the user. The rotation base 26B has a cylindrical portion with an outer diameter approximately equal to the inner diameter of the substantially cylindrical grip 26A. The tip of the rotation base 26B (a lower side of the paper in FIG. 6) serves as a contact portion 26D that comes into contact with the patient's eye. A transparent window 29 (see FIG. 7) is formed in the contact portion 26D. A reflective surface 27 (see FIG. 7) is fixed inside the rotation base 26B. The grip 26A is attached to the outer side of the cylindrical portion of the rotation base 26B. The rotation base 26B can rotate relative to the grip 26A. A plurality of protrusions 26C that protrude outward are provided on a portion of the outer periphery of the rotation base 26B that is exposed to the outside when the grip 26A is attached. In this embodiment, a plurality of (10 in this example shown in FIG. 6) protrusions 26C are provided at equal intervals in the circumferential direction at each of the aforementioned predetermined angles (36 degrees in the example shown in FIG. 6). The user can rotate the rotation base 26B provided with the reflective surface 27 with respect to the grip 26A by placing fingers on at least one of the protrusions 26C. Specifically, the user grips and fixes the grip 26A with multiple fingers (for example, index finger and thumb) and rotates only the rotation base 26B by hooking the remaining fingers (for example, the middle finger) on the protrusion 26C (the rotation axis is axis AX1). Therefore, as compared to the reflective surface that is rotated by rotating the entire contact lens, fewer finger movements are required during operation. As a result, the contact lens 26 is easily held in a stable manner even when the reflective surface 27 is rotated.

In the contact lens 26 shown in FIG. 6, the shape is designed so that the rotation angle of the rotation base 26B due to one rotation operation by the user's finger has a specified value (a specified angle). As an example, in the contact lens 26 shown in FIG. 6, the rotation base 26B rotates 36 degrees with respect to the grip 26A by a single operation. Therefore, the rotation base 26B rotates with respect to the grip 26A once by rotating ten times in the same direction. It should be noted the specified angle can be changed. For example, a partially rotatable lens in which the rotating base rotates 45 degrees with respect to the grip by a single operation may be used.

FIG. 7 is a view of the contact lens 26 on a side opposite to the patient's eye E. As shown in FIG. 7, interval indicators 28 is formed on a part of the inner wall of the contact lens 26 (in the example shown in FIG. 7, the inner wall of the rotation base 26B). A plurality of interval indicators 28 are arranged at regular intervals to serve as a guideline for intervals between spots irradiated with treatment laser light. In the example shown in FIG. 7, five interval indicators that extend linearly are arranged at equal intervals. The interval indicators 28 are formed on an inner wall of the contact lens 26 opposite to a side of the inner wall where the reflective surface 27 is provided. When the contact lens 26 is viewed from a side of the contact lens 26 opposite to the patient's eye E, a reflected image 28Z of at least one of the interval indicators 28 is reflected on the reflective surface 27. Further, during the treatment, a reflected image of the treatment target site (in this embodiment, the trabecular meshwork) of the patient's eye E is also reflected (in other words, viewed) on the reflective surface 27. Furthermore, a reflected image of the aiming light irradiated onto the treatment target location is also reflected on the reflective surface 27 . Therefore, while grasping the positional relationship between the treatment target location reflected on the reflective surface 27 and the aiming light, the operator can adjust the aiming point of the treatment laser light to each of the plurality of spots in the treatment target location one by one using the reflected image 28Z of the interval indicators 28 as a guide. Note that the diagram of the observation field illustrated in each drawing in the present disclosure is a diagram showing an area corresponding to the area AR1 shown in FIG. 7 that is observed in an enlarged manner. Note that the reflective surface 27 is formed with a size that does not cause the reflected image (reflected image 28Z) of the interval indicators 28 to be missing. In this embodiment, the irradiation of the treatment laser light to the entire circumference of the trabecular meshwork TM is performed by rotating the reflective surface 27 about the axis AX1 one time while maintaining a state where the center of the pupil of the patient's eye E and the center of the light-transmitting window 29 are substantially aligned with each other regardless of the type of the contact lens 26 (for example, regardless of whether the interval indicators 28 are provided and whether the contact lens 26 is a partially rotatable lens). At this time, the area AR1 is made to follow the displacement of the reflective surface 27 as appropriate. Therefore, the operator operates the joystick 5 while irradiating the entire periphery of the trabecular meshwork TM with the treatment laser light to rotate the region AR1 about the axis AX1.

Note that the contact lens 26 shown in FIGS. 6 and 7 is only one of contact lenses that can be used in treatment with the ophthalmic laser treatment device 1 in this embodiment. Therefore, it is also possible to use a contact lens different from the contact lens 26 shown in FIGS. 6 and 7 for treatment in the ophthalmic laser treatment device 1 in this embodiment. For example, it is possible to use a contact lens for treatment that includes the reflective surface 27 and the interval indicators 28 but is not a partially rotatable lens (that is, the reflective surface 27 is rotated by rotating the entire lens). It is also possible to use a partially rotatable lens without the interval indicators 28 for treatment. It is also possible to use a contact lens that does not include the interval indicators 28 and is not a partially rotatable lens.

In addition, if a contact lens that requires the entire body to be rotated in order to rotate the reflective part is used, the operator grasps the contact lens with multiple fingers and rotates the entire contact lens while keeping the contact portion in contact with the patient's eye E. In this case, there is a limit to the angle at which the operator can rotate the entire contact lens without changing his/her grip (without having to re-grip the contact lens). Furthermore, when the operator changes his/her grip of the contact lens, the positional relationship between the patient's eye E and the laser irradiation optical system 10 may easily change. On the contrary, in the partially rotating contact lens 26 illustrated in FIGS. 6 and 7, the reflective surface can be rotated with each specified angle without changing operator's grip of the contact lens.

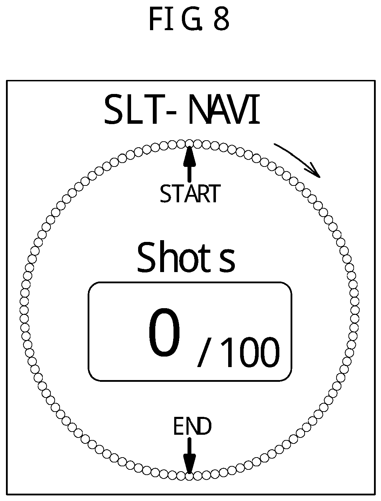

One example of a laser irradiation plan will be described with reference to FIG. 8. FIG. 8 is a diagram showing an example of an irradiation plan displayed on a control box. In this embodiment, the irradiation plan is prepared by operating the control box 6. In the irradiation plan, when the patient's eye E is irradiated with the treatment laser light using the contact lens 26 having the reflective surface 27, an irradiation order for a plurality of irradiation spots to which the treatment laser light is to be irradiated is determined. In the irradiation plan in this embodiment, an irradiation range of the treatment laser light (that is, a range in a circumferential direction in which a plurality of irradiation spots are arranged) in an annular treatment target location (in this embodiment, a trabecular meshwork TM), the irradiation spot to which the treatment laser light is irradiated first (i.e., the irradiation spot at the "START" position shown in FIG. 8), the number of irradiation spots (the number of denominators of "Shots" shown in FIG. 8), and the irradiation order (including an irradiation direction) of the treatment laser light for each of the irradiation spots. In FIG. 8, an arrow in a circumferential direction indicates the direction of the irradiation order. In this embodiment, each time a single treatment laser light is irradiated, the irradiation spot adjacent to the irradiation spot where the irradiation has been completed becomes a next irradiation spot to which the treatment laser light is irradiated next, and "1" is added to the numerator of "Shots" shown in FIG. 8. In addition, each time a single treatment laser light is irradiated, a displaying manner of a spot (an irradiated spot) corresponding to a spot where an irradiation has been completed among multiple irradiation spots arranged in an arc shape or an annular shape (the annular shape in FIG. 8) is changed to be different from that of other spots. The multiple irradiations of the treatment laser light are sequentially performed in a clockwise or counterclockwise direction.

In the present embodiment, the direction in which the treatment target site to which the treatment laser light is irradiated is actually located and the direction in which the reflective surface 27 of the contact lens 26 is directed to observe the treatment target site are opposite directions relative to the optical axis of the observation optical system 40. In this embodiment, the direction in which the treatment laser light should be irradiated is indicated by the direction in which the reflective surface 27 is directed. However, the direction in which the treatment laser light should be irradiated may be indicated by the direction in which the treatment target site is actually located. In addition, in another embodiment as described later, information regarding the contact lens used in treatment (for example, at least one of information indicating whether the contact lens is a partially rotatable lens, information on the specified angle for the partially rotatable lens, information indicating whether the interval indicators 28 are formed on the contact lens) may be included in the irradiation plan.

As an example, in this embodiment, the operator specifies an irradiation plan area and the number of irradiation spots at once by specifying either the "entire circumferential" mode or the "half circumferential" mode. Specifically, in this embodiment, there is a "entire circumferential" mode in which the treatment laser light is irradiated to multiple spots over the entire circumference of the angle A, and a "half circumferential" mode in which the treatment laser light is irradiated to multiple spots over the half of the circumference of the angle A. The default value of the number of spots to be irradiated in the "entire circumferential" mode is set to 100. The default value of the number of spots to be irradiated in the "half circumferential" mode is set to 50. When the "entire circumference" mode is designated by the operator, the control unit 60 sets the area covering the entire circumference of the corner A as the planned irradiation area, and sets "100" as the number of spots scheduled to be irradiated. In the example shown in FIG. 8, the "entire circumferential" mode is designated. Further, when the "half circumferential" mode is designated by the operator, the control unit 60 sets the area covering half the circumference of the corner A as the irradiation planed area, and sets "50" as the number of spots to be irradiated. In this embodiment, when the "half circumferential" mode is designated, the operator operates the touch panel to specify the "upper half", "lower half", "right half", "left half", etc. of the angle A. to specify the detailed position of the area to be irradiated at the corner A. The control unit 60 sets a planned irradiation area based on the input result.

It should be noted that the method for accepting the designation of the irradiation planned area and the number of irradiation spots may be changed. For example, the laser treatment device 1 may prepare in advance modes other than the "entire circumferential" mode and the "half circumferential" mode. The control unit 60 may accept a designation of an angle (within a range of 360 degrees) from the operator and set a region corresponding to the designated angle as the planned irradiation region. The control unit 60 may allow the operator to directly input the number of spots to be irradiated, and may set the input number of spots to be irradiated regardless of the selected mode. The control unit 60 may change the number of spots to be irradiated, which is predetermined for each mode, in accordance with an operation by the operator. The control unit 60 may allow the operator to input the interval between two adjacent spots, and also calculate the number of spots to be irradiated according to the parameters of the planned irradiation area (for example, the length in the circumferential direction of the treatment target location) and the input interval between the spots.

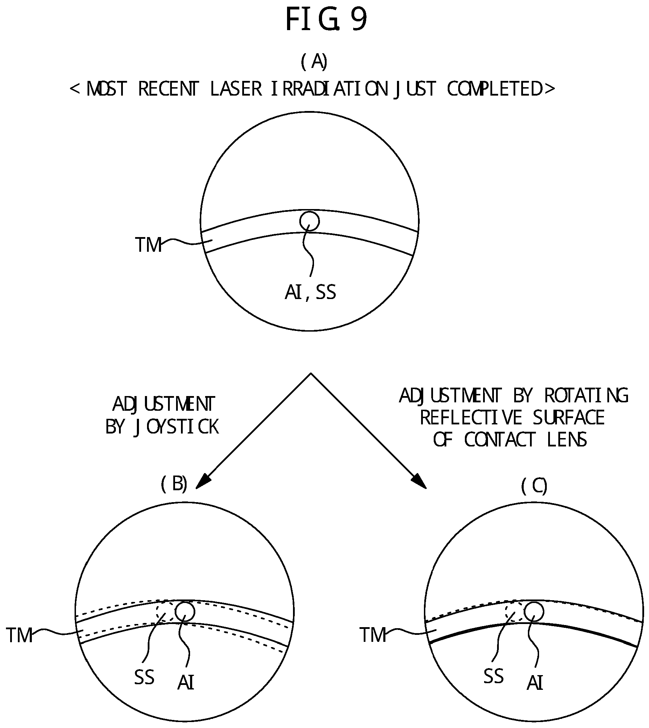

A method for adjusting the aiming point of the irradiation spot will be described with reference to FIG. 9. In this embodiment, as a method of adjusting the aiming point of the irradiation spot of the treatment laser light, there may be a method of adjusting the positional relationship between the patient's eye E and the laser irradiation optical system 10 by operating the joystick 5 and a method of performing at least one of a rotation operation of the reflective surface 27 of the contact lens 26 and a movement operation of the reflective surface 27 of the contact lens 26. FIG. 9 (A) shows a state in which the most recent irradiation of the treatment laser light has been completed. In the state of FIG. 9 (A), the position of the irradiated spot SS where an irradiation with the treatment laser light has been completed matches the position of the aiming light Al. From the state shown in FIG. 9 (A), the operator changes the position of the aiming light Al (that is, the aiming point of the irradiation spot) to a position of the next irradiation spot (in the example shown in FIG. 9, a position right next to the position of the irradiated spot SS).

FIG. 9 (B) shows a state where the aim position is changed from the state shown in FIG. 9 (A) to the position for the next irradiation spot by operating the joystick 5 (that is, with the angle of the reflective surface 27 of the contact lens 26 fixed). In FIG. 9 (B), the positions of the irradiated spot SS at the time of completion of the irradiation at an immediately previous timing and the treatment target location (the trabecular meshwork TM in this embodiment) are schematically shown by dotted lines. However, in reality, since there is no treatment mark in the irradiated spot SS at the time of completion of the irradiation at an immediately previous timing, it is difficult to accurately adjust the aim position by operating the joystick 5.

**In** addition, FIG. 9 (C) shows a state where the aim position is changed from the state shown in FIG. 9 (A) to the next irradiation spot position by rotating the reflective surface 27 of the contact lens 26 (that is, without operating the joystick 5). Also in FIG. 9 (C), the positions of the irradiated spot SS at the time of completion of the irradiation at the immediately previous timing and the treatment target location are schematically shown by dotted lines. However, in reality, even in FIG. 9 (C), there is no treatment scar in the irradiated spot SS at the time of completion of the immediately previous irradiation, and it is also difficult to accurately adjust the aiming point by rotating the reflective surface 27. In the present disclosure, a guide is displayed on the internal display unit 50 to assist the operator in adjusting the aim position.

### <First embodiment>

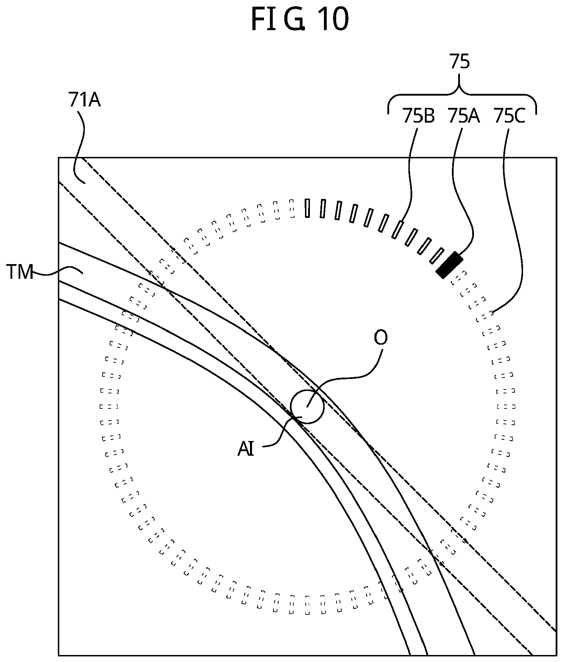

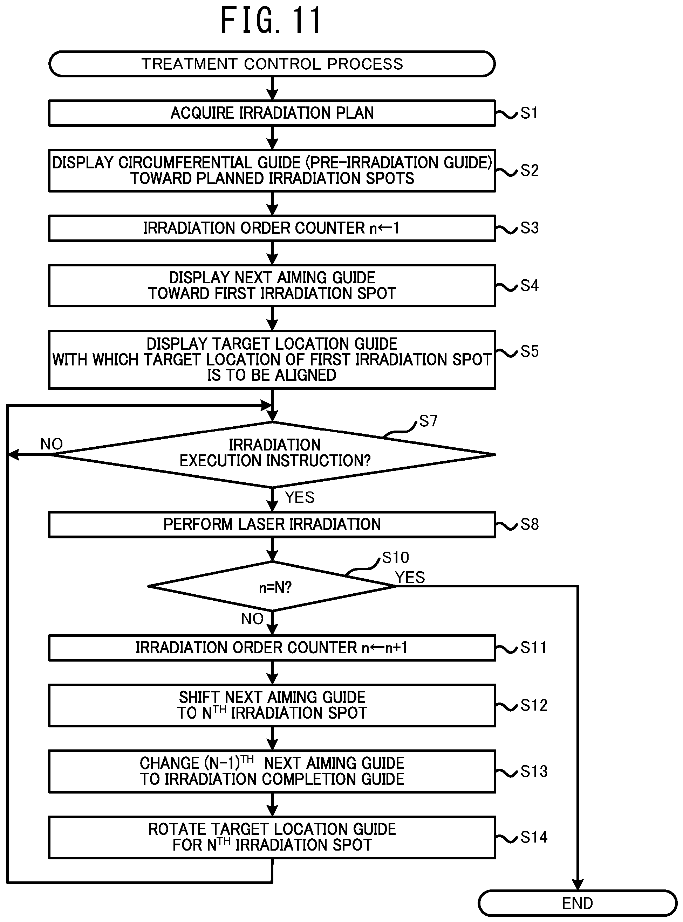

With reference to FIGS. 10 and 11, a treatment control process executed by the ophthalmic laser treatment device 1 in the first embodiment will be described. First, with reference to FIG. 10, the aiming guide (in the first embodiment, the target location guide 71A and the outer circumferential guide 75) displayed on the internal display unit 50 by the ophthalmic laser treatment device 1 of the first embodiment will be described.. The aiming guide is displayed on the internal display unit 50 in order to assist the operator in adjusting the aiming point of the treatment laser light to an appropriate position. As described above, the method for adjusting the aiming point includes a method by adjusting the positional relationship between the patient's eye E and the laser irradiation optical system 10 by operating the joystick 5 or a method by at least one of rotating and moving the reflective surface 27 of the contact lens 26. The operator checks the aiming guide while observing the tissue of the patient's eye E through the eyepiece 46 (that is, without taking his eyes off the eyepiece 46), and uses the confirmed aiming guide as a reference to adjust the irradiation point of the treatment laser light. Although details will be described later, the control unit 60 (CPU 61) of the ophthalmic laser treatment device 1 displays an aiming guide on the internal display unit 50 according to the progress of the irradiation plan.

In the example shown in FIG. 10, the arc-shaped or annular-shaped treatment target location (in the example shown in FIG. 10, the trabecular meshwork TM) and the spot Al of the aiming light irradiated on the treatment target location are observed by the operator. The observation images of the treatment target location and the aiming light are a reflected image reflected by the reflective surface of the contact lens. Note that FIG. 10 illustrates a situation where a contact lens that is not provided with the above-mentioned interval indicators 28 (see FIG. 7) and is not a partially rotatable lens is used. As described above, in this embodiment, the center of the display area in the internal display unit 50, the observation optical axis of the observation optical system 40, the optical axis of the treatment laser light, and the optical axis of the aiming light are all aligned with each other at the center O. Therefore, the internal display unit 50 can display an image at an appropriate angle in an appropriate direction centered on the optical axis of the treatment laser light and the aiming light. In other words, the operator visually recognizes the aiming light at the center of the observation visual field and the center of the display area of the internal display unit 50. Therefore, it becomes easier to adjust the aiming light and the irradiation position of the treatment laser light based on the displayed images on the internal display unit 50. When an instruction to perform laser irradiation is input, the treatment laser light is applied to the same spot as the aiming light spot Al.

As shown in FIG. 10, the ophthalmic laser treatment device 1 of the first embodiment displays a target location guide 71A on the internal display unit 50 as an aiming guide. The target location guide 71A is aligned with the arc-shaped or annular-shaped treatment target location (the trabecular meshwork TM in the example shown in FIG. 10) of the patient's eye E, which is observed by the operator via the observation optical system 40. The operator can adjust the irradiation position of the treatment laser light to an appropriate position by performing a various types of adjustments so as to cause the arc-shaped or annular-shaped treatment target location observed via the observation optical system 40 to be aligned with the displayed target location guide 71A.

The target location guide 71A illustrated in FIG. 10 includes a line in parallel with the tangential direction that is in contact with the appropriate position of the irradiation spot that is determined by the irradiation plan as a next spot when viewed in a sight direction of the operator in the observation optical system. Due to the characteristics of a circle, there are two points on the circumference where straight-lines with a same particular inclination touch the circle, and these two points are located exactly opposite to each other with respect to the center of the circle. Since the arc-shaped or annular-shaped treatment target location that is being observed by the operator is reflected on the reflective surface 27, a range of the fan-shaped portion of the treatment target site observed through the reflective surface 27 falls within an angular range of less than 180 degrees with the center of the arc-shaped or annular-shaped treatment target location as a reference. As a result, the two points as described above do not fall within the observation field of view at the same time, and the treatment target location having a tangent line parallel to the inclination of the target location guide 71A is uniquely determined. Therefore, the operator operates at least one of the joystick 5 and the contact lens 26 to move the treatment target location so that the tangential direction of the arc-shaped or annular-shaped treatment target location is aligned with the direction of the straight-line portion of the target location guide 71A. Accordingly, the aiming point of the treatment laser light can be accurately adjusted. Note that FIG. 10 shows a state in which the aiming point (the position of the aiming light spot Al) is accurately adjusted to the appropriate position of the next irradiation spot.

In addition, when the operator observes an arc-shaped or annular-shaped treatment target location through the observation optical system 40, the curvature of the treatment target location to be observed is changed according to the size of the patient's eye E, the observation magnification of the observation optical system 40, etc. This is because the observation range changes, and the treatment target site observed at the end of the observation range is affected by the curvature change. When adjustment is made to cause the target location guide 71A to be aligned with the tangential direction of the treatment target location, the treatment target location will be observed near the center of the observation range, which is the position of the aiming light. Therefore, the operator can align the tangential direction of the treatment target location with the direction of the straight-line portion of the target location guide 71A, regardless of the size of the patient's eye E, observation magnification, etc. Therefore, the operator can more easily adjust the aiming point to an appropriate position.

The target location guide 71A illustrated in FIG. 10 includes two straight-lines in parallel with the tangential direction as described above. The positions of the optical axis of the treatment laser light and the optical axis of the aiming light (the position of the center O in FIG. 10) are located between the two straight-lines. As a result, the center of the aiming light spot Al observed by the operator is located between the two straight-lines in the target location guide 71A. Therefore, the operator can align the tangential direction of the treatment target location with the direction of the straight-line portion of the target location guide 71A, and at the same time aligns the aiming point of the treatment laser light (i.e., the spot Al of the aiming light) with the appropriate position of the treatment target location. Note that the control unit 60 adjusts the distance between the two straight-lines in the target location guide 71A according to the observation magnification of the observation optical system 40. As a result, the two straight-lines of the target location guide 71A are displayed at an appropriate distance according to the observation magnification so that adjustment of the aim position is appropriately assisted regardless of the observation magnification. However, the number of straight-lines of the target location guide 71A may be one. In this case, if the straight-line passes through the positions of the optical axis of the treatment laser light and the optical axis of the aiming light, the aiming point can be easily aligned with the appropriate position.

The control unit 60 determines the angle of the target location guide 71A to be displayed on the internal display unit 50 according to the progress of the irradiation plan, and then displays the target location guide 71A at the determined angle. Therefore, the target location guide 71A is displayed on the internal display unit 50 at an appropriate angle according to the progress of the irradiation plan. The details will be described later.

Further, as shown in FIG. 10, the ophthalmic laser treatment device 1 of the first embodiment displays an outer circumferential guide 75 as an aiming guide on the internal display unit 50. The outer circumferential guide 75 indicates at least one of the rotation angle of the reflective surface of the contact lens suitable for the progress of the irradiation plan (that is, the direction in which the reflective surface 27 is disposed relative to the central axis AX1 of the contact lens 26) and the direction in which the irradiation spot to which the treatment laser light is to be emitted is located (i.e., the direction in which the irradiation spot in the observation image using the contact lens 26). The outer circumferential guide 75 is displayed along the outer circumference of the observation field of view by the operator via the observation optical system 40. As described above, the observation optical axis of the observation optical system 40 and the center of the display area of the internal display unit 50 are aligned with each other. Therefore, by displaying the outer circumferential guide 75 along the outer circumference of the observation field with the center of the display area of the internal display unit 50 as a reference, the operator can easily grasp the appropriate direction.

Specifically, in this embodiment, the plurality of outer circumferential guides 75 are arranged in an arc-shape or an annular-shape along the outer circumference of the observation field. Further, when moving one of the outer circumferential guides 75, the control unit 60 moves it on an arc-shape or annular-shape line along the outer circumference of the observation field. The center of the arc-shape or annular-shape line on which the outer circumferential guide 75 is displayed (in other words, the center of the outer circumferential guide 75 or the center of curvature of the outer circumferential guide 75) is aligned with the observation optical axis of the observation optical system 40. Therefore, the operator can appropriately grasp the direction indicated by the outer circumferential guide 75.

The outer circumferential guide 75 includes a next aiming guide 75A. The next aiming guide 75A indicates the appropriate direction of the next irradiation spot to be irradiated with the treatment laser light among the plurality of irradiation spots determined in the irradiation plan. Each time the laser irradiation is performed, the control unit 60 shifts the position of the next aiming guide 75A to a position corresponding to an irradiation spot that is determined in the irradiation plan and is located adjacent in a forward direction. For example, in the case of an irradiation plan in which 100 irradiations are performed in a clockwise direction over the entire circumference of the trabecular meshwork TM, the next aiming guide 75A may be shifted by 3.6 degrees relative to the center O. Therefore, the operator can appropriately grasp the direction of the irradiation spot whose aiming point is to be adjusted next using the next aiming guide that is shifted along the outer circumference of the observation field.

The outer circumferential guide 75 includes an completed irradiation guide 75B. The completed irradiation guide 75B indicates the direction of the irradiation spot that has already been irradiated with the treatment laser light, among the plurality of irradiation spots determined in the irradiation plan. Each time the laser irradiation is completed, the control unit 60 replaces the next aiming guide 75A, which was displayed just before the irradiation, with the completed irradiation guide 75B. Therefore, the operator can appropriately adjust the next aiming point using the next aiming guide 75A by grasping the direction in which the laser irradiation has been completed. It also becomes easier to recognize the progress of treatment.

The outer circumferential guide 75 includes a pre-irradiation guide 75C. The pre-irradiation guide 75C indicates the direction of an irradiation spot that is scheduled to be irradiated with the treatment laser light subsequent to the next time, among the plurality of irradiation spots defined in the irradiation plan. Each time the laser irradiation is completed, the control unit 60 replaces the pre-irradiation guide 75C displayed in the direction of the irradiation spot in the next irradiation order determined in the irradiation plan with the next aiming guide 75A. Therefore, transition of the next irradiation guide 75A is performed. In this case, the operator can appropriately adjust the next aiming point using the next aiming guide 75A by grasping the direction of the irradiation spot that is scheduled to be irradiated with the treatment laser light after the next time. It also becomes easier to recognize the progress of treatment.

Note that the control unit 60 displays each of the next aiming guide 75A, the completed irradiation guide 75B, and the pre-irradiation guide 75C in a distinguishable manner (that is, in a different displaying manner). Therefore, the operator can easily recognize the type of the outer circumferential guide 75 being displayed. Note that the control unit 60 may selectively display one or two of the next aiming guide 75A, the completed irradiation guide 75B, and the pre-irradiation guide 75C. In this case, it is desirable to display at least the next aiming guide 75A. Furthermore, it is more desirable to display both the next aiming guide 75A and the completed irradiation guide 75B. The next aiming guide 75A may be, for example, a straight-line extending to the center O through which the optical axis of the observation optical system 40 passes. It is only necessary that the next aiming guide 75A guides the direction in which the reflective surface 27 of the contact lens 26 should be directed.

With reference to FIG. 11, a treatment control process in the first embodiment will be described. Note that all treatment control processing described below is executed by the CPU (controller) 61 of the control unit 60 when an instruction for starting treatment is input via a touch panel or the like. The CPU 61 executes the treatment control process according to a control program stored in the ROM 62 or nonvolatile memory 65.

First, the control unit 60 obtains an irradiation plan on treatment laser light for the patient's eye E (S1). As described above, in the irradiation plan, when the patient's eye E is irradiated with the treatment laser light using the contact lens 26 having the reflective surface 27, the irradiation order etc. for multiple irradiation spots scheduled to be irradiated with the treatment laser light are determined. In the irradiation plan of this embodiment, the irradiation range of the treatment laser light (that is, the range in a circumferential direction in which a plurality of irradiation spots are arranged) in the annular treatment target location (the trabecular meshwork TM in this embodiment), the irradiation spot to be irradiated first, the number of irradiation spots, and the order of irradiation (including the irradiation direction) of the treatment laser light for each irradiation spot are determined. In this embodiment, each time a single irradiation with the treatment laser light is performed, the irradiation spot adjacent to the irradiation spot for which the irradiation has been completed becomes the next irradiation spot to be irradiated with the treatment laser light. The multiple irradiations of the treatment laser light are sequentially performed in a clockwise or counterclockwise direction. In addition, in another embodiment as described later, information regarding the contact lens used in treatment (for example, at least one of information indicating whether the contact lens is a partially rotatable lens, information on the specified angle for the partially rotatable lens, information indicating whether the interval indicators 28 are formed on the contact lens) may be included in the irradiation plan. As an example, in this embodiment, the irradiation plan is formed by operating the control box 6.

The control unit 60 displays the pre-irradiation guide 75C in a direction for each of the plurality of irradiation spots that are planed to be irradiated with the treatment laser light under the irradiation plan, among the outer circumference of the display area in the internal display unit 50 (that is, the outer circumference of the observation field of view via the observation optical system 40) (S2).

The control unit 60 sets, to "1", the value of the irradiation order counter "n" that specifies an irradiation order of the treatment laser lights to the plurality of irradiation spots defined in the irradiation plan (S3). The control unit 60 displays the next aiming guide 75A in the direction of the first irradiation spot defined in the irradiation plan among the outer circumference of the display area in the internal display unit 50 (S1). Furthermore, the control unit 60 determines the angle of the target location guide 71A according to the position or direction of the first irradiation spot defined in the irradiation plan, and displays the target location guide 71A on the internal display unit 50 at the determined angle (S5).

Next, the control unit 60 determines whether the operator has input an instruction to perform irradiation with the treatment laser light (S7). If the irradiation execution instruction has not been input (S7: NO), the determination in S7 is repeated to be in a standby state. During this time, the aiming point of the treatment laser light is adjusted by the operator. As an example, when guiding irradiation to a site in the 6 o'clock direction in the actual trabecular meshwork TM, the control unit 60 may display a straight-line extending in the horizontal direction (i.e., a unique angle corresponding to the aforementioned site) as the target site guide 71A. Further, the target location guide 71A illustrated in FIG. 10 guides irradiation to a region diagonally downward to the left in the actual trabecular meshwork.

When an instruction to perform laser irradiation is input (S7: YES), the treatment laser light is irradiated (S8). Next, the value of the irradiation order counter "n" is determined to reach the number "N" of total irradiation spots determined to be irradiated with treatment laser light under the irradiation plan (S10). If the irradiation of the treatment laser light to all irradiation spots is not completed (that is, if "n" has not reached "N") (S10: NO), "1" is added to the value of the irradiation order counter "n" (S11). The control unit 60 shifts the position of the next aiming guide 75A to a position corresponding to an irradiation spot that is located adjacent in the forward direction and is determined by the irradiation plan (S12). Further, the control unit 60 changes the displayed next aiming guide 75A to the completed irradiation guide 75B (S13). Further, the control unit 60 rotates the angle of the displayed target location guide 71A according to the forward direction and the advancing angle of the irradiation spot (S14). As an example, in the case of an irradiation plan in which 100 adjacent irradiations are performed to the entire circumference of the trabecular meshwork TM, the control unit 60 may rotate, 3.6 degrees in one direction, the target location guide 71A displayed on the internal display unit 50 each time the irradiation is performed. After that, the process returns to S7. When irradiation of all irradiation spots with the treatment laser light is completed (that is, when "n" has reached "N") (S10: YES), the process ends.

### <Second embodiment>

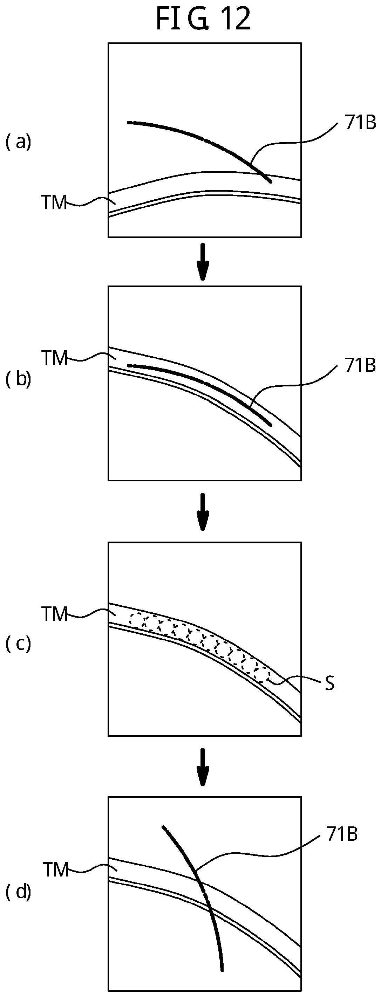

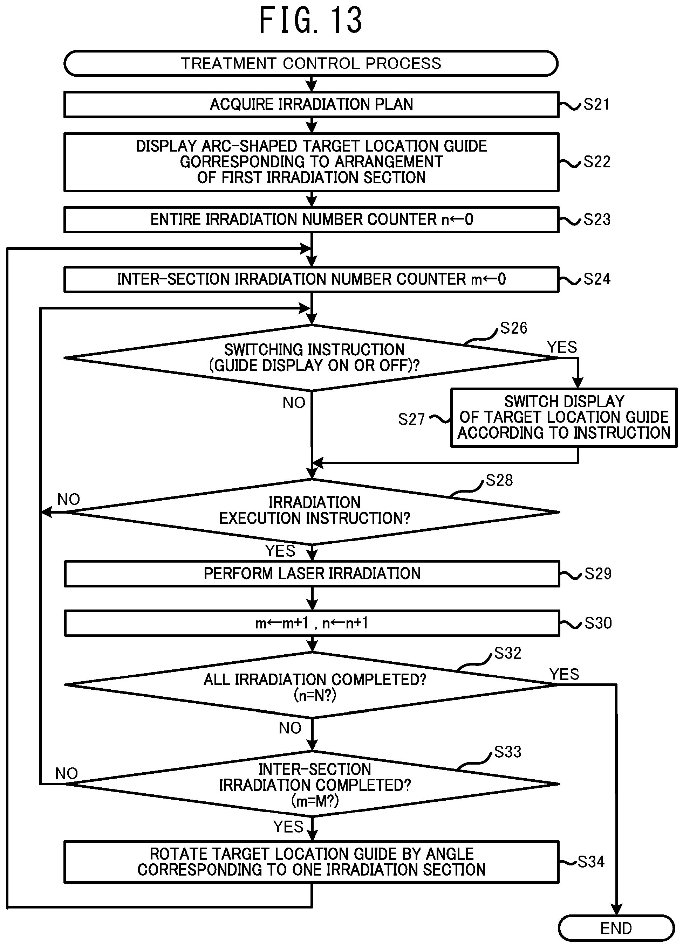

With reference to FIGS. 12 and 13, a treatment control process executed by the ophthalmic laser treatment device 1 according to a second embodiment will be described. Note that the configuration and control described in the first embodiment can be applied to at least part of the configuration, control, etc. in the second to fourth embodiments described below. Therefore, among the embodiments to be described below, the description may be omitted or simplified for parts where the configuration and control described in the first embodiment can be used. In the second embodiment, the shape and the like of the target location guide displayed on the internal display unit 50 are different from those in the first embodiment. Note that, similarly to the first embodiment, the second embodiment will be described by exemplifying a case where a contact lens that is not provided with the interval indicators 28 (see FIG. 7) and is not a partially rotatable lens is used..

First, with reference to FIG. 12, an aiming guide (i.e., a target location guide 71B in the second embodiment) displayed on the internal display unit 50 by the ophthalmic laser treatment device 1 of the second embodiment will be described. As described above, the target location guide serves as a reference with which the arc-shaped or annular-shaped treatment target location (the trabecular meshwork TM in the example shown in FIG. 12) of the patient's eye E, which is being observed by the operator through the observation optical system 40, is aligned. The target location guide 71B illustrated in FIG. 12 includes an arc-shaped portion corresponding to the arc-shaped arrangement of a plurality of irradiation spots determined under the irradiation plan. Therefore, by aligning the treatment target location with the target location guide 71B so that the arc shape drawn by the treatment target location matches the shape of the arc-shaped portion of the target location guide 71B, the rotation angle of the reflective surface of the contact lens and the aiming point of the treatment laser light can be easily, accurately adjusted.

The control unit 60 changes the curvature of the arc-shaped portion of the target location guide 71B displayed on the internal display unit 50 according to the magnification of the observation optical system 40. As a result, the arc-shaped portion of the target location guide 71B changes appropriately in accordance with the difference in appearance of the treatment target location that changes depending on the observation magnification. Therefore, the aiming point can be adjusted more easily.

In the second embodiment, a predetermined number of treatment laser lights are emitted within a fan-shaped irradiation section while the angle of the reflective surface of the contact lens is fixed. When irradiation by a predetermined number of treatment laser lights within one irradiation section is completed, the rotation angle of the reflective surface of the contact lens is changed (adjusted) by the operator, and next irradiation is performed a predetermined number of times within the next fan-shaped irradiation section. **In** the example shown in FIG. 12, the angular range of one irradiation section is 36 degrees. Therefore, when the treatment is performed on the entire circumference of the treatment target location, irradiation by a predetermined number of treatment laser lights is performed in each of the ten irradiation sections.

In FIG. 12 (a), the control unit 60 first displays, on the internal display unit 50, the arc-shaped target location guide 71B corresponding to the arc-shaped arrangement of a predetermined number of the irradiation spots (10 in FIG. 12) located in the first irradiation section under the irradiation plan. In the state shown in FIG. 12 (a), the arc shape of the observed treatment target location and the arc shape of the displayed target location guide 71B do not match. Therefore, as shown in FIG. 12 (b), the operator uses the contact lens and the joystick 5 to align the arc of the treatment target location with the arc of the target location guide 71B. As a result, the rotation angle of the reflective surface of the contact lens and the aiming point of the treatment laser light approach an appropriate angle and position for irradiating the irradiation spot determined by the irradiation plan with the treatment laser light. **In** the second embodiment, the operator can switch between displaying and non-displaying the target location guide 71B by operating a switch (not shown) disposed on the joystick 5 (see FIG. 12 (b) and (c)). As shown in FIG. 12 (c), the operator completes irradiation by a predetermined number of treatment laser lights within the irradiation zone while fixing the rotation angle of the reflective surface of the contact lens (in FIG. 12 (c), the spot S irradiated with the treatment laser light is schematically shown by a dotted line). When performing irradiation with a predetermined number of treatment laser lights, the operator may repeat the operation illustrated in FIG. 9 (B), for example. As shown in FIG. 12(d), when the irradiation of a predetermined number of treatment laser lights within the irradiation section is completed, the control unit 60 rotates the target location guide 71B in the forward direction toward the next irradiation spot by an angle corresponding to a single irradiation section (i.e., 36 degrees in this example of FIG. 12). The operator rotates the contact lens so that the arc shape of the observed treatment target location matches the arc shape of the rotated target location guide 71B. By repeating the above steps, treatment is appropriately performed on each of the plurality of irradiation sections. That is, in this embodiment, after repeating the procedure of shifting the observation image and of adjusting the aiming point using the joystick 5 multiple times, the reflective surface of the contact lens is rotated by a large amount (approximately 36 degrees in the example shown in FIG. 12) as one cycle. By repeating this cycle multiple times, a wide range of the annular-shaped or arc-shaped treatment target location is appropriately irradiated with the treatment laser light multiple times. The control unit 60 rotates the target location guide 71B based on a predetermined irradiation plan at a timing of largely rotating the reflective surface of the contact lens. As a result, even when a contact lens with a simple structure and the ophthalmic laser treatment device 1 with a simple structure are used in combination, the operator can adjust positions of multiple irradiation spots for treatment appropriately over a wide range of the treatment target area.

With reference to FIG. 13, a treatment control process in the second embodiment will be described. First, the control unit 60 obtains the irradiation plan for treatment laser light on the patient's eye E (S21). As mentioned above, the treatment plan acquired at S21 defines the angle for one irradiation section, the number of times M the treatment laser light is emitted within each irradiation section, the total number of times the treatment laser light is emitted to the entire treatment target location, the irradiation spot to which the treatment laser light is first applied, the irradiation order (including the irradiation direction) of the treatment laser light, etc.

The control unit 60 causes the internal display unit 50 to display an arc-shaped target location guide 71B corresponding to the arrangement of a predetermined number of irradiation spots arranged in the first irradiation section (S22). The control unit 60 sets the value of the total irradiation number counter "n", which specifies the cumulative number of times the treatment laser light is emitted to the entire treatment target location, to "0" (S23). Furthermore, the control unit 60 sets the value of a single-section irradiation number counter "m", which specifies the number of times the treatment laser light is emitted into a single irradiation section, to "0" (S24).

Next, the control unit 60 determines whether the operator has input an instruction for switching between displaying and non-displaying of the target location guide 71B on the internal display unit 50 (S26). When the display switching instruction is input (S26: YES), the control unit 60 switches between displaying and non-displaying of the target location guide 71B on the internal display unit 50 according to the input instruction (S27). After that, the process proceeds to S28.

The control unit 60 determines whether an instruction for executing irradiation with the treatment laser light has been input by the operator (S28). If the irradiation execution instruction has not been input (S28: NO), the process returns to S26. During this time, the aiming point of the treatment laser light is adjusted by the operator. When the irradiation execution instruction for the treatment laser light is input (S28: YES), the treatment laser light is emitted (S29). Next, the control unit 60 adds "1" to each of the total irradiation number counter "n" and the single-section irradiation number counter "m" (S30). The control unit 60 determines whether the value of the total irradiation number counter "n" has reached the total number "N" the treatment laser light is emitted to the entire treatment target location (S32). If irradiation by the treatment laser light to all irradiation spots is not completed (that is, if "n" has not reached "N") (S32: NO), the control unit 60 determines whether the single-section irradiation number counter "m" has reached the predetermined number of times "M" for irradiation with the treatment laser light within one irradiation section (S33). If the irradiation with the treatment laser light within one irradiation section is not completed (that is, if "m" has not reached "M") (S33: NO), the process returns to S26, and the processes of S26 to S33 are repeated. When irradiation with the treatment laser light on one irradiation section is completed (S33: YES), the control unit 60 rotates the target location guide 71B in the forward direction toward the next irradiation spot by an angle corresponding to one irradiation section. (S34), and the process returns to S24 and shifts to treatment on the next irradiation section. When irradiation on all irradiation spots with the treatment laser light is completed (S32: YES), the process ends.

Note that the process illustrated in the second embodiment may be changed. For example, when a partially rotatable lens is used, the control unit 60 may acquire information about the specified rotation angle (that is, the angle of rotation by one operation) of the used partially rotatable lens. The control unit 60 may rotate the displayed target location guide 71B by the specified rotation angle in the forward direction of the irradiation spot each time the predetermined number of times (corresponding to the predetermined number "M"), which are planned to perform in the specified rotation angle (corresponding to the above-described "single irradiation section angle"), irradiation with the treatment laser light is completed. In this case, each time the laser irradiation within one specified rotation angle range is completed, the target location guide 71B is rotated to an angle corresponding to the next specified rotation angle range. Therefore, even when treatment is performed using a partially rotatable lens, the aim position can be adjusted more appropriately.

Further, the control unit 60 may rotate the displayed target location guide 71B in accordance with the forward direction and the advancing angle of the irradiation spot each time the laser irradiation is performed. In this case, each time the laser irradiation is performed, the target location guide 71B is displayed with an appropriate angle according to the next irradiation spot.

The control unit 60 may change the displaying manner of the target location guides 71A and 71B based on information regarding the contact lenses being used. For example, if the contact lens being used is a contact lens that can be rotated as a whole by the operator, the control unit 60 may rotate the target location guide 71A by the advancing angle of the irradiation spot each time the laser irradiation is performed. In addition, when the contact lens being used is a partially rotatable lens, the control unit 60 may rotate the target location guide 71B by a specified rotation angle each time the laser irradiation is completed a predetermined number of times. In this case, since the display of the target location guides 71A and 71B is appropriately changed according to the contact lens being used, the aiming point of the treatment laser light can be easily adjusted.

### <Third embodiment>

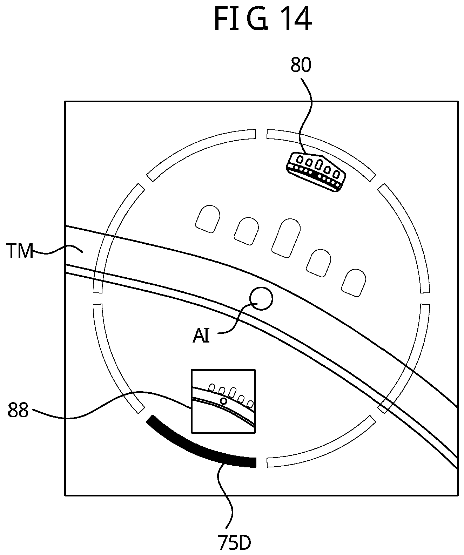

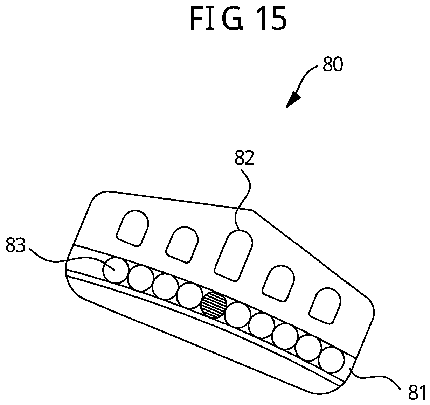

Next, a treatment control process executed by an ophthalmic laser treatment device 1 according to a third embodiment will be described with reference to FIGS. 14 to 16. With reference to FIGS. 14 and 15, an aiming guide (in the third embodiment, an angle guide 80 and a section aiming guide 75D) and a previous irradiation image 88 that are displayed on the internal display unit 50 by the ophthalmic laser treatment device 1 in the third embodiment will be explained first.

As shown in FIG. 14, the ophthalmic laser treatment device 1 in the third embodiment displays the angle guide 80 as an aiming guide on the internal display unit 50. The angle guide 80 indicates the rotation angle of the reflective surface of the contact lens in accordance with the progress of an irradiation plan. The operator refers to the angle indicated by the angle guide 80 and adjusts the rotation angle of the reflective surface of the contact lens, thereby adjusting the aiming point of the treatment laser light while the angle of the reflective surface is being appropriately adjusted.

As shown in FIG. 15, the angle guide 80 in the third embodiment includes a treatment target location schematic image 81. The shape of the treatment target location schematic image 81 is a shape along an appropriate angle of an arc-shaped or annular-shaped treatment target location of the patient's eye (in this embodiment, the trabecular meshwork TM shown in FIG. 14) on which an irradiation spot, for which a next laser irradiation is planned to be performed, is disposed. Therefore, the operator can easily adjust the rotation angle of the reflective surface of the contact lens by adjusting the angle of the observed treatment target site to match (or follow) the shape of the treatment target site schematic image 81.

Further, the angle guide 80 according to the third embodiment includes a shape following the contour shape of the reflective surface of the contact lens and a shape following a characteristic structure (in this embodiment, interval indicators 28) of the contact lens reflected on the reflective surface when the reflective surface of the contact lens is oriented in an appropriate direction. In the example shown in FIGS. 14 and 15, the control unit 60 displays the angle guide 80 in a direction in which the reflective surface is arranged with respect to the axis AX1 when viewed from the center of the display area of the internal display unit 50. Therefore, the operator can easily adjust the rotation angle of the reflective surface of the contact lens by adjusting the reflective surface of the contact lens to be oriented in a direction along the angle guide 80.

The control unit 60 determines the angle of the angle guide 80 to be displayed on the internal display unit 50 according to the progress of the irradiation plan, and displays the angle guide 80 at the determined angle. Therefore, by referring to the angle guide 80, the operator can adjust the angle of the reflective surface of the contact lens to an appropriate angle according to the progress of the irradiation plan.

Specifically, in the treatment by the ophthalmic laser treatment device 1 according to the third embodiment, a contact lens (a partially rotatable type lens) 26 shown in FIGS. 6 and 7 may be used. As described above, in the partially rotatable contact lens 26, each time the contact lens 26 is operated once by the user's finger, a portion including the reflective surface 27 rotates by a specified angle with respect to the grip 26A held by the user. The control unit 60 rotates the angle of the angle guide 80 by a specified angle each time a predetermined number of laser irradiations that were set to be performed within one specified rotation angle range (in other words, within one irradiation section) are completed. Therefore, even when performing treatment using a partially rotatable contact lens 26, the operator can adjust the angle of the reflective surface 27 to an appropriate angle by referring to the angle guide 80.

Note that the control unit 60 recommends the operator to rotate the reflective surface 27 by the specified angle each time the predetermined number of laser irradiations that were set to be performed within one specified rotation angle range (in other words, within one irradiation section) are completed (that is, each time adjustments of the aiming point to multiple positions by the joystick 5 while maintaining the orientation of the reflective surface 27 of the contact lens 26). Therefore, especially when performing treatment using the partially rotatable contact lens 26, the operator can appropriately grasp the timing at which the reflective surface 27 needs to be rotated by the specified angle. Note that in this embodiment, the control unit 60 recommends rotation of the reflective surface 27 by generating sound through a speaker. Therefore, the operator can grasp the timing at which the reflective surface 27 needs to be rotated while looking through the eyepiece lens 46.

As shown in FIG. 15, the angle guide 80 in the third embodiment includes a interval indicator schematic diagram 82 that imitates the interval indicators 28 (see FIG. 7) provided on the contact lens 26. As described above, the contact lens 26 used in treatment by the ophthalmic laser treatment device 1 according to the third embodiment is provided with the interval indicators 28. A plurality of interval indicators 28 are arranged at regular intervals to serve as a guideline for intervals between spots irradiated with treatment laser light. As shown in FIG. 14, at least one of the interval indicators 28 is observed by the operator via the reflective surface 27. In addition to easily adjusting the rotation angle of the reflective surface 27 of the contact lens 26 using the angle guide 80, the operator can also adjust a plurality of aiming points by adjusting the aiming points of the treatment laser light while grasping the interval indicator schematic diagram 82. In particular, when the contact lens 26 to be used is provided with the interval indicators 28, the operator can adjust the aiming point while comparing the interval indicators 28 of the contact lens 26 observed through the observation optical system 40 with the interval indicator schematic diagram 82 displayed on the internal display unit 50. Thus, the aiming point can be adjusted more easily.

A spot arrangement guide diagram 83 is added to the angle guide 80 of the third embodiment. The spot arrangement guide diagram 83 indicates an arrangement of consecutive irradiation spots, including the irradiation spot scheduled to be irradiated with the treatment laser light next, among the plurality of irradiation spots set under the irradiation plan. The control unit 60 shifts the displayed irradiation spot to which the treatment laser light is applied next time to, among the plurality of irradiation spots included in the spot arrangement guide diagram 83, the irradiation spot adjacent in the forward direction determined by the irradiation plan each time the laser irradiation is performed. Therefore, by grasping the position of the next irradiation spot displayed on the spot arrangement guide diagram 83, the operator can more appropriately adjust the next aiming point. In particular, in the angle guide 80 shown in FIGS. 14 and 15, the spot arrangement guide diagram 83 is displayed in a manner where the spot arrangement guide diagram 83 matches referential intervals of the irradiation spots indicated by the interval indicator schematic diagram 82. Therefore, the operator can more appropriately adjust the next aiming point by grasping the positional relationship of the next irradiation spot shown in the spot arrangement guide diagram 83 relative to the interval indicator schematic diagram 83.

As shown in FIG. 14, the ophthalmic laser treatment device 1 in the third embodiment displays a section aiming guide 75D on the internal display unit 50 as an outer circumferential guide displayed along the outer circumference of the observation field. The section aiming guide 75D illustrated in FIG. 14 indicates an area of the treatment target site according to the progress of the treatment plan (i.e., an area of the treatment target site when the patient's eye E is viewed from a front side without looking through the observation optical system 40). In the example shown in FIG. 14, the section aiming guide 75D is displayed by displaying a portion, among bar graphs each corresponding to one of eight of the irradiation sections, indicative of the direction of an area of the treatment target location corresponding to a laser irradiation to the current irradiation section in a different manner than other portions (that is, in a manner distinguishable from the other portions). In FIG. 14, the angle guide 80 guides the direction in which the reflective surface 27 of the contact lens 26 is oriented, and the section aiming guide 75D indicates a direction of the region of the treatment target location corresponding to the angle guide 80. In addition, in FIG. 14, considering that the observation image observed through the contact lens 26 is a reflected image (i.e., a virtual image), the section aiming guide 75D is arranged in an opposite direction to the angle guide 80. However, the section aiming guide 75D and the angle guide 80 may be arranged in the same direction. Each time the same number of laser irradiations as the number of the plurality of irradiation spots included in one irradiation section are completed, the control unit 60 shifts the position of the section aiming guide 75D to a position adjacent in the forward direction determined by the irradiation plan. Therefore, the operator can easily grasp the direction of the reflective surface 27 for irradiating the irradiation spot within the irradiation section with the treatment laser light using the section aiming guide 75D. Note that the section aiming guide 75D may indicate the direction of the irradiation section according to the progress of the treatment plan. In this case, the direction of the section aiming guide 75D is opposite to the direction of the appropriate rotation angle of the reflective surface 27.

The control unit 60 displays the section aiming guide 75D for each of specified angle ranges of the partially rotatable contact lens 26 (see FIGS. 6 and 7) being used. The control unit 60 shifts the position of the section aiming guide 75D to an adjacent position in the forward direction determined by the irradiation plan each time the same number of laser irradiations as the number of the plurality of irradiation spots included within one specified angle range are completed. Therefore, the section aiming guide 75D is appropriately displayed depending on the specifications of the partially rotatable contact lens 26 and the progress of treatment.

Further, the ophthalmic laser treatment device 1 in this embodiment includes a imaging optical system that captures an observation image of the patient's eye E. As shown in FIG. 14, the control unit 60 control the internal display unit 50 to display a previous irradiation image 88 captured by the imaging optical system. The previous irradiation image 88 is an image of the treatment target location irradiated with aiming light, which was captured by the imaging optical system when the treatment laser light was emitted last time. For example, a still image captured immediately before or after the previous laser irradiation may be displayed as the previous irradiation image 88. In the treatment according to the present embodiment, since no irradiation scars by the treatment laser light remain, an image of the treatment target location irradiated with the aiming light is used as the previous irradiation image 88. However, a graphic imitating a spot may be drawn on the captured image at a predetermined position (in this embodiment, a position that is aligned with the optical axis of the treatment laser light). The operator can adjust a next aiming point by grasping the location irradiated with the previous treatment laser light (that is, the region where the aiming light appears in the displayed previous irradiation image 88). Therefore, it becomes easier to irradiate the treatment laser light more appropriately.

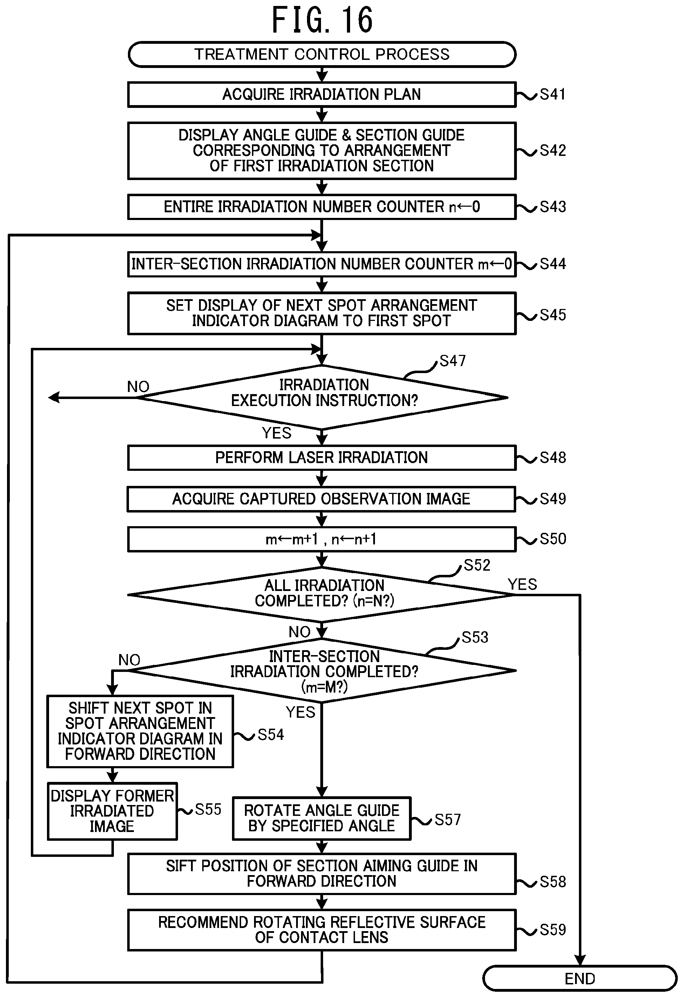

With reference to FIG. 16, a treatment control process according to the third embodiment will be described. First, the control unit 60 obtains an irradiation plan for treatment laser light on the patient's eye E (S41). The treatment plan acquired at S41 includes a specified angle of the partially rotatable contact lens 26 being used (that is, the angle for one irradiation section), the number of times M that the treatment laser light is emitted in each irradiation section, the total number N the treatment laser lights are emitted onto the entire treatment target location, the irradiation spot to which the treatment laser light is first applied, the irradiation order (including the irradiation direction) of the treatment laser light, etc. Note that information such as the specified angle of the contact lens 26 may be acquired based on information indicative of the type of the contact lens 26.

The control unit 60 controls the internal display unit 50 to display the angle guide 80 and the section aiming guide 75D at an angle, in a direction, and at a position corresponding to the arrangement of the first irradiation section (S42). The control unit 60 sets the value of the total irradiation number counter "n", which specifies the cumulative number of times the treatment laser light is emitted to the entire treatment target location, to "0" (S43). Furthermore, the control unit 60 sets the value of a single-section irradiation number counter "m", which specifies the number of times the treatment laser light is emitted into a single irradiation section, to "0" (S44). The control unit 60 sets the displayed irradiation spot to which the treatment laser light is applied next time (i.e., a next spot) to a spot having a first irradiation order of the treatment laser light among the plurality of spots in the spot arrangement guide diagram 83 (S45).

The control unit 60 determines whether the operator has input an instruction for performing irradiation with the treatment laser light (S47). If the irradiation execution instruction has not been input (S47: NO), the determination in S47 is repeated. During this time, the aiming point of the treatment laser light is adjusted by the operator. When the instruction to perform laser irradiation is input (S47: YES), the treatment laser light is emitted (S48). Further, the control unit 60 acquires an observation image of the treatment target location irradiated with the aiming light, which is captured by the imaging optical system when the treatment laser light is emitted (S49).

Next, the control unit 60 adds "1" to each of the total irradiation number counter "n" and the single-section irradiation number counter "m" (S50). The control unit 60 determines whether the value of the total irradiation number counter "n" has reached the total number "N" the treatment laser light is emitted to the entire treatment target location (S52). If irradiation by the treatment laser light to all irradiation spots is not completed (that is, if "n" has not reached "N") (S522: NO), the control unit 60 determines whether the single-section irradiation number counter "m" has reached the predetermined number of times "M" for irradiation with the treatment laser light within one irradiation section (S53). If the laser irradiation within one irradiation section is not completed (that is, if "m" has not reached "M") (S53: NO), the control unit 60 shifts the irradiation spot to which the treatment laser light will be applied next time (i.e., the next spot) to a spot adjacent in the forward direction of the irradiation. Further, the control unit 60 controls the internal display unit 50 to display the observation image captured at S49 as the previous irradiation image 88 (S55), and the process returns to S47.

When irradiation of the treatment laser light within one irradiation section is completed (S53: YES), the control unit 60 rotates the angle guide 80 by the specified angle of the partially rotatable type contact lens 26 (i.e., the angle for a single irradiation section). The control unit 60 shifts the position of the section aiming guide 75D to a position adjacent in the forward direction determined by the irradiation plan (S58). Furthermore, the control unit 60 recommends the operator to rotate the reflective surface 27 by the specified rotation angle (S59), and the process returns to S44. When irradiation on all irradiation spots with the treatment laser light is completed (S52: YES), the process ends.

Note that the process illustrated in the third embodiment may be changed. First, the displaying manner of the angle guide 80 may be changed. For example, a diagram or a photograph that assist the operator in grasping the appropriate angle of the observation image of the treatment target location observed through the observation optical system 40 may be displayed on the internal display unit 50 as an angle guide. Further, a schematic diagram or the like when viewing the contact lens 26 in a direction along the observation optical axis may be displayed as an angle guide.

The angle guide 80 in the third embodiment is rotated by the angular range of the irradiation section each time the predetermined number of laser irradiations set to be performed within one irradiation section are completed. However, the control unit 60 may rotate the displayed angle guide 60 in accordance with the forward direction and advancing angle of the irradiation spot each time the laser irradiation is executed.

The angle guide 80, the section aiming guide 75D, and the previous irradiation image 88 of the third embodiment are displayed on the internal display unit 50. However, in the ophthalmic laser treatment device 1, the operator can appropriately adjust the aiming point even when at least one of the angle guide 80, the section aiming guide 75D, and the previous irradiation image 88 is displayed on a display unit different from the internal display unit 50 (for example, an external display 7 of the control box 6 provided outside of the observation optical system 40).

### <Fourth embodiment>

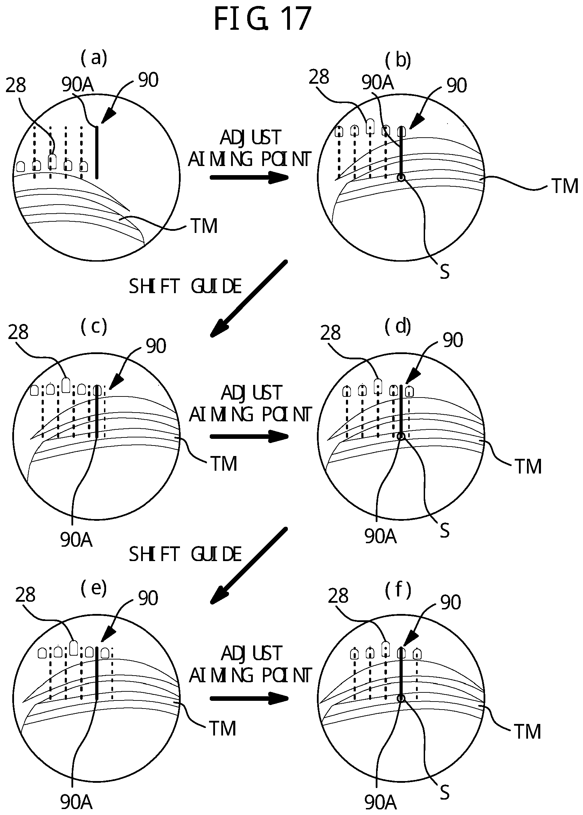

With reference to FIGS. 17 to 19, a treatment control process executed by an ophthalmic laser treatment device 1 according to a fourth embodiment will be described. FIGS. 17 to 19 illustrate a contact lens 26 (see FIGS. 6 and 7) that is of an internal rotatable type and in which internal indicators 28 are formed. As shown in FIGS. 15 and 16, an observation image observed by an observation optical system 40 includes a reflected image of the interval indicators 28 reflected by a reflective surface 27 of the contact lens 26. A spot interval guide 90 indicates, to an operator, an appropriate interval (i.e., a gap) between a plurality of irradiation spots that are scheduled to be irradiated with treatment laser light.

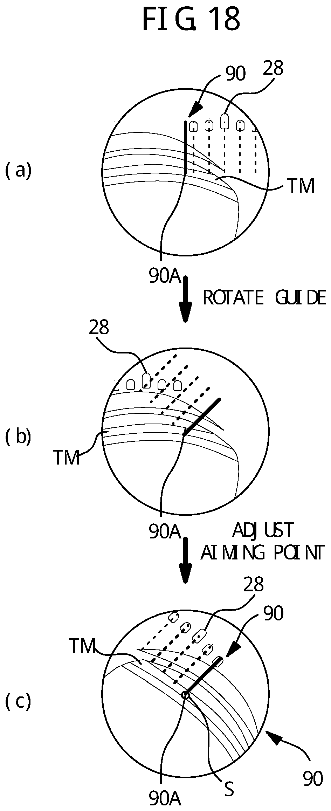

First, the flow of treatment using the spot interval guide 90 will be described using an example shown in FIGS. 17 and 18. FIGS. 17 and 18 show one example with an irradiation plan set in advance by the operator where irradiation is performed in a clockwise direction step by step on an actual trabecular meshwork area from the lower side (see FIG. 17) to the diagonally lower left side (see FIG. 18).

The irradiation plan in this embodiment includes rotating a rotation base 26B in the clockwise direction by the operator. In FIGS. 17 and 18, the lower trabecular meshwork is reflected by the reflective surface of the contact lens and enters into the operator's observation field. Therefore, in FIGS. 17 and 18, the top and bottom are reversed, and the lower trabecular meshwork is reflected (shown). When the treatment for the irradiation spot proceeds from right to left (that is, a counterclockwise direction) on the reflective surface in FIGS. 17 and 18, in the actual lower trabecular meshwork, the treatment for the irradiation spot proceeds from right to left. (i.e., a clockwise direction).

First, as shown in FIG. 17 (a), the control unit 60 determines the angle of the spot interval guide 90 to be displayed on the internal display unit 50 according to the progress of the irradiation plan (in detail, according to the direction of the irradiation section to which the treatment laser light is applied). Then, the control unit 60 shows the spot internal guide 90 at the determined angle. The indicator of the spot interval guide 90 extends along an appropriate direction of reflection of the treatment laser light to the next irradiation spot by the reflective surface 27 of the contact lens 26. FIG. 17 shows a guide for performing adjacent irradiations ten times on the area below the actual trabecular meshwork TM while maintaining the orientation of the reflective surface 27 of the contact lens 26. Based on the aforementioned area, the indicators of the spot interval guide 90 are arranged horizontally. The control unit 60 causes the next aiming indicator 90A, to which the next treatment laser light is aimed, to be identifiably displayed in the spot interval guide 90 In the example shown in FIG. 17A, the control unit 60 first cause the next aiming indicator 90A to be displayed at a position overlapping with the indicator located on an end side opposite to the forward direction of the treatment defined by the irradiation plan (in FIG. 17 (a), the indicator located on the right side end) among the plurality of indicators of the spot interval guide 90 (in the present embodiment, five indicators that are overserved via the reflective surface 27 of the contact lens 26 and with which the reflected image of five interval indicators 28 is aligned). Among the plurality of indicators of the spot interval guide 90, indicators other than the indicators located to be overlapped with the next aiming indicator 90A are pre-irradiation indicators corresponding to at least one of the spots to which the treatment laser light is applied subsequent to the next time. Further, the control unit 60 displays the plurality of indicators of the spot interval guide 90 and the next aiming indicator 90A so that the next aiming indicator 90A is aligned with the optical axis of the treatment laser light emitted by the laser irradiation optical system 10. Note that the position where the aiming indicator 90A is displayed next time does not need to be completely aligned with the optical axis of the treatment laser light. That is, the next aiming indicator 90A is displayed at a position corresponding to the optical axis of the treatment laser light so that the operator can recognize the position on the optical axis of the treatment laser light.

The operator adjusts at least one of the rotation angle of the contact lens 26 and a relative position, which is controllable by the joystick 5, between the patient's eye E and the laser irradiation optical system 10 so that the position of the plurality of indicators (in this embodiment, five indicators) of the spot interval guide 90 is aligned with the position of the plurality of the aiming indicators 28 reflected on the reflective surface 27 of the contact lens 26 in the observation field. As shown in FIG. 17 (b), when the position of the plurality of indicators of the spot interval guide 90 and the position of the aiming indicators 28 is aligned with each other, the spot S to which the treatment laser light is applied is located on a straight-line extending from the next aiming indicator 90A of the spot interval guide 90. The operator inputs an instruction to execute irradiation with the treatment laser light with the position of the aiming light being aligned with the treatment target location. As a result, the treatment laser light is applied to the spot S through which the optical axis of the laser irradiation optical system 10 passes.

As shown in FIG. 17 (c), when the laser irradiation is completed, the control unit 60 moves, by one appropriate interval of the aiming spot, the indicator of the spot interval guide 90 in a direction (i.e., a counterclockwise direction in FIG. 17) opposite to the forward direction of treatment determined under the irradiation plan (i.e., a clockwise direction in FIG. 17). As a result, the position of the plurality of indicators of the spot interval guide 90 are offset from the position of the aiming indicator 28. The control unit 60 moves the position of the next aiming indicator 90A by one appropriate interval of the irradiation spot in the forward direction determined by the irradiation plan. Furthermore, the control unit 60 changes the indicator on which the next aiming indicator 90A was superimposed during the previous irradiation by the treatment laser light to an irradiated indicator indicative of a spot to which the treatment laser light has been already applied. The irradiation plan in this embodiment is a plan in which ten adjacent irradiations are performed using the spot interval guide 90 formed of five indicators, and one appropriate interval of the irradiation spot means a half of the interval between the adjacent indicators of the spot interval guide 90. Note that the interval between the adjacent indicators of the spot interval guide 90 in this embodiment is 800 µm (i.e., twice the spot size of the treatment laser light and aiming light). The operator adjusts the aiming point of the treatment laser light by operating at least one of the joystick 5 and the contact lens (in FIG. 17, the joystick 5) such that the plurality of indicators of the spot interval guide 90 (in FIG. 17 (c), the five indicators other than the next aiming indicator 90A) is aligned with the position of the aiming indicator 28 within the observation field. As shown in FIG. 17 (d), when the position of the plurality of indicators of the spot interval guide 90 is aligned with the position of the aiming indicator 28, and the position of the aiming light is aligned with the position of the treatment target location, the operator inputs an instruction to execute laser irradiation. Note that this embodiment is merely one example, and the moving direction of the spot interval guide 90 may be reversed (i.e., guide to irradiate in a counterclockwise direction from the left end of the aiming indicator 28 to the right side).

As shown in FIG. 17 (e), when the laser irradiation is completed, the control unit 60 moves, by one appropriate interval of the irradiation spot, the indicator of the spot interval guide 90 in a direction opposite to the forward direction determined by the irradiation plan. Further, the control unit 60 moves the position of the next aiming indicator 90A by one appropriate interval of the irradiation spot in the forward direction determined by the irradiation plan. In the example shown in FIG. 17 (e), the position of the next aiming indicator 90A is aligned with the position of the second indicator from the end on the opposite side in the treatment forward direction among the plurality of indicators in the spot interval guide 90. The operator again aligns the position of the plurality of indicators of the spot interval guide 90 with the position of the aiming indicator 28 within the observation field, and inputs an instruction to execute laser irradiation (see FIG. 17 (f)). The above procedure is repeated until all the plurality of spots within one irradiation section are irradiated with the treatment laser light. In other words, the spot interval guide 90 assists the operator in repeatedly shifting the aiming point by one spot shown in FIG. 9 whereby the operator can grasp the interval between the plurality of irradiation spots.

FIG. 18 (a) shows a state when irradiation on one irradiation section with the treatment laser light (in the example shown in FIGS. 17 and 18, irradiation with the treatment laser lights 10 times) is completed. The control unit 60 automatically rotates the entire indicators of the spot interval guide 90 in the forward direction by the angle corresponding to the irradiation section. In the contact lens 26 used in this embodiment, a rotation base 26B rotates 36 degrees by a single rotation operation (see FIG. 7). Therefore, the control unit 60 rotates the entire indicators of the spot interval guide 90 by 36 degrees based on the irradiation plan. Furthermore, the control unit 60 displays the next aiming indicator 90A at a position to be interposed with the indicator, which is an indicator among the plurality of indicators that is located on an end in a direction opposite to the forward direction determined by the irradiation plan. Furthermore, the control unit 60 sets, as unirradiated indicators, all the indicators among the plurality of indicators of the spot interval guide 90 except the indicator at the position with which the next aiming indicator 90A is interposed. Further, the control unit 60 displays the plurality of indicators of the spot interval guide 90 and the next aiming indicator 90A so that the next aiming indicator 90A is aligned with the optical axis of the treatment laser light emitted by the laser irradiation optical system 10. FIG. 18 (b) shows a result of the observation image. By operating the partially rotatable contact lens 26 once, the operator rotates the reflective surface 27 of the contact lens 26 by a specified angle in a clockwise direction, which is the forward direction of the treatment (see FIG. 18 (c)). As described above, the specified angle of the partially rotatable contact lens 26 is the angle at which the spot interval guide 90 is rotated. After that, irradiation by the treatment laser light to all the plurality of spots in the next irradiation section is repeated.

As described above, the spot interval guide 90 is displayed to have the user recognize the appropriate interval between the plurality of irradiation spots scheduled to be irradiated with treatment laser light. The spot interval guide 90 is displayed on the internal display unit 50 according to the progress of the irradiation plan. Therefore, the operator checks the spot interval guide 90 while observing the patient's eye E through the eyepiece 46 (that is, without taking his eyes off the eyepiece 46), and adjusts the plurality of aiming points for the treatment laser light using the confirmed spot interval guide 90 as a reference. Therefore, the operator can easily cause the interval between the plurality of irradiation spots, to which treatment laser light is applied, to be an appropriate interval.

As described above, in this embodiment, the center of the display area in the internal display unit 50, the observation optical axis of the observation optical system 40, the optical axis of the treatment laser light, and the optical axis of the aiming light are all aligned with each other. Therefore, the internal display unit 50 can display the spot interval guide 90 at an appropriate angle in an appropriate direction centered on the optical axis of the treatment laser light and the aiming light. In other words, the operator visually recognizes the aiming light at the center of the observation visual field and the center of the display area of the internal display unit 50. Therefore, it becomes easier to adjust the aiming light and the irradiation position of the treatment laser light based on the displayed images on the internal display unit 50.

The control unit 60 changes the intervals between the plurality of indicators of the spot interval guide 90 displayed on the internal display unit 50 according to the magnification of the observation optical system 40. That is, the control unit 60 increases the interval between the indicators of the spot interval guide 90 as the magnification of the observation optical system 40 increases. In this case, even if the observation magnification is changed, the spot interval guide 90 in accordance with the appropriate interval between the plurality of irradiation spots (in this embodiment, in accordance with the five internal indicators 28 that serve as a guide for the appropriate interval between the plurality of irradiation spots) is displayed on the internal display unit 50.

The control unit 60 aligns the interval between the plurality of indicators of the spot interval guide 90 with the interval between the interval indicators 28 of the contact lens 26 being used. Therefore, the operator adjusts the aiming points while referring to the position and the direction of the interval indicators 28 observed through the observation optical system 40 and the position and the direction of the spot interval guide 90 displayed on the internal display 50. Thus, the operator can appropriately adjust the aiming points for the treatment laser light. As an example, in the fourth embodiment, the control unit 60 changes the displaying manner of the spot interval guide 90 (for example, the interval and the number of the plurality of indicators in the spot interval guide 90, and the rotation angle used when rotating the spot interval guide 90) based on information on the contact lens 26 being used. Therefore, since an appropriate spot interval guide 90 is displayed according to the interval indicator 28 of the contact lens 26 being used, the treatment can smoothly proceed.

As shown in FIGS. 17 and 18, the spot interval guide 90 has a shape extending along an appropriate direction of reflection of the treatment laser light to the next irradiation spot by the reflective surface 27 of the contact lens 26. The control unit 60 determines the angle of the spot interval guide 90 to be displayed on the internal display unit 50 according to the progress of the irradiation plan, and displays the spot interval guide 90 at the determined angle. Therefore, the operator can easily adjust the rotation angle of the reflective surface 27 of the contact lens 26 by adjusting the reflection angle of the treatment laser light to follow the direction indicated by the spot interval guide 90. Furthermore, since the spot interval guide 90 is displayed on the internal display unit 50 at an appropriate angle according to the progress of the irradiation plan, the aiming point can be more appropriately adjusted. Furthermore, the operator can easily adjust the rotation angle of the reflective surface 27 of the contact lens 26 by adjusting the rotation angle so that the interval indicator 28 is located ahead of the direction indicated by the shape of the spot interval guide 90.

As shown in FIGS. 17 and 18, in addition to a plurality of indicators arranged at regular intervals in the spot interval guide 90, the control unit 60 also displays the next aiming indicator 90A used for setting the position of the next irradiation spot at a position aligned with the optical axis of the treatment laser light emitted by the laser irradiation optical system 10 in a different manner than that of the other indicators. Each time the laser irradiation is executed, the control unit 60 shifts the position of the next aiming indicator 90A by one appropriate interval between the plurality of irradiation spots in the forward direction determined by the irradiation plan. Therefore, the operator sets the position on the tissue to which the next treatment lase light is applied while considering the next irradiation indicator 90A indicative of the position of the optical axis of the treatment laser light (if the aiming light is emitted, the optical axis of the aiming light).

Among the plurality of indicators in the spot interval guide 90, the indicators other than the next aiming indicator 90A are post-irradiated indicators corresponding to at least one of the spots to which the treatment laser light has been applied or pre-irradiated indicators corresponding to at least one of the spots to which the treatment laser light has not been applied. The control unit 60 displays each of the next aiming indicator 90A, the post-irradiated indicator, and the pre-irradiated indicator in a different manner (that is, in a manner that makes each of these indicators distinguishable for the operator). Therefore, the operator can easily grasp the positional relationship between the next aiming indicator 90A, the post-irradiated indicator, and the pre-irradiated indicator, making it easier to proceed with the treatment more smoothly.

As shown in FIG. 17, the control unit 60 shifts, by one appropriate interval between the plurality of irradiation spots, a plurality of indicators arranged at equal intervals in the spot interval guide 90 (in the present embodiment, the same intervals of the interval indicators 28 of the contact lens 26) in a direction opposite to the forward direction determined by the irradiation plan each time the laser irradiation is performed. The operator adjusts the aiming points every time so that the position of the plurality of indicators that is shifted each time the laser irradiation is performed is aligned with a fixed position on the observation image observed through the observation optical system 40 (in the present embodiment, the position where the plurality of indicators of the spot interval guide 90 are aligned with the interval indicators 28 on the observation image). Thus, the aiming point can be easily adjusted.

As described above, the shape of the treatment target location of the patient's eye (the trabecular meshwork TM in FIGS. 17 and 18) that is irradiated with the treatment laser light is arc-shaped or annular-shaped. The control unit 60 entirely rotates the plurality of indicators arranged at regular intervals in the spot interval guide 90. By rotating the entire indicators of the spot interval guide 90 according to the shape of the arc-shaped or annular-shaped treatment target location, adjustment of the aiming point can be appropriately performed using the spot interval guide 90 regardless of the difference in the circumferential position of the irradiation spot.

Specifically, the control unit 60 rotates the spot interval guide 90 in the forward direction determined by the treatment plan by aligning one rotation angle of the spot interval guide 90 with the angle for the irradiation section (that is, an angle range where treatment lase light irradiation based on the spot interval guide 90 can be performed multiple times while fixing the spot interval guide 90 at an angle). As a result, treatment with the treatment laser light for each irradiation section can be smoothly performed on the arc-shaped or annular-shaped treatment target location. In the present embodiment, the rotation angle of the spot interval guide 90 according to the irradiation section corresponds to the specified angle at which the reflective surface 27 of the partially rotatable contact lens 26 rotates in one operation.

The control unit 60 continues to shift the indicators of the spot interval guide 90 by one appropriate interval between the irradiation spots each time the laser irradiation is performed until laser irradiation for all the spots in the single irradiation section is completed. Further, the control unit 60 automatically shifts the entire indicators of the spot interval guide 90 in the forward direction by the angle for the irradiation section each time laser irradiation for all the spots in the single irradiation section is completed. As a result, not only the irradiation by the treatment laser light within one irradiation section, but also the irradiation by the treatment laser light to each of the plurality of irradiation sections is automatically and appropriately assisted according to the progress of the irradiation plan.

Furthermore, each time the plurality of indicators is rotated, the control unit 60 displays the next aiming indicator 90A at a position interposed with the position of the indicator, among the indicators, that is located at an end opposite to the forward direction of the treatment determined by the irradiation plan (i.e., a position where the aiming point of the irradiation spot is aligned with first after rotation of the indicator). As a result, the position of the next aiming indicator 90A is appropriately changed according to the progress of the irradiation plan, making it easier for the treatment to proceed more smoothly. Further, each time the control unit 60 rotates the plurality of indicators, all the indicators other than the indicator at the position where the aiming indicator 90A will be superimposed next time are the pre-irradiated indicators.

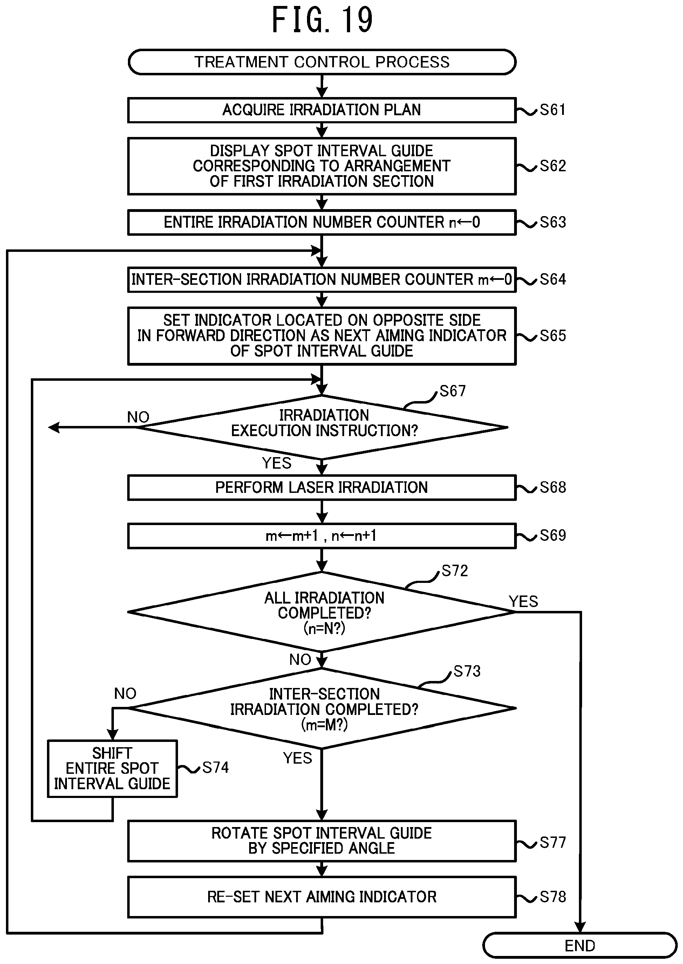

With reference to FIG. 19, a treatment control process in the fourth embodiment will be described. First, the control unit 60 acquires the irradiation plan for treatment laser light on the patient's eye E (S61). The treatment plan acquired at S61 includes a specified angle of the partially rotatable contact lens 26 being used (that is, the angle for one irradiation section), the number of times M that the treatment laser light is emitted in each irradiation section, the total number N the treatment laser lights are emitted onto the entire treatment target location, the irradiation spot to which the treatment laser light is first applied, the irradiation order (including the irradiation direction) of the treatment laser light, etc. Note that information such as the specified angle of the contact lens 26 may be acquired based on information indicative of the type of the contact lens 26.

The control unit 60 controls the internal display unit 50 to display the spot interval guide 90 at an angle corresponding to the arrangement of the first irradiation section (S62). The control unit 60 sets the value of the total irradiation number counter "n", which specifies the cumulative number of times the treatment laser light is emitted to the entire treatment target location, to "0" (S63). Furthermore, the control unit 60 sets the value of a single-section irradiation number counter "m", which specifies the number of times the treatment laser light is emitted into a single irradiation section, to "0" (S64). The control unit 60 sets a displaying position of the next aiming indicator 90A, which is the indicator for next irradiation with the treatment laser light, among the plurality of indicators of the spot interval guide 90, so that the next aiming indicator 90A is overlapped with an indicator located at a position on an end side opposite to the forward direction in the irradiation order.

The control unit 60 determines whether the operator has input an instruction for performing irradiation with the treatment laser light (S67). If the irradiation execution instruction has not been input (S677: NO), the determination in S67 is repeated. During this time, the aiming point of the treatment laser light is adjusted by the operator. When the instruction to perform laser irradiation is input (S67: YES), the treatment laser light is emitted (S68).

Next, the control unit 60 adds "1" to each of the total irradiation number counter "n" and the single-section irradiation number counter "m" (S69). The control unit 60 determines whether the value of the total irradiation number counter "n" has reached the total number "N" the treatment laser light is emitted to the entire treatment target location (S72). If irradiation with the treatment laser light to all irradiation spots is not completed (that is, if "n" has not reached "N") (S72: NO), the control unit 60 shifts the entire spot interval guide 90 in a direction opposite to the forward direction of the irradiation spots by a single appropriate interval between the irradiation spots (S74). In addition, at S74, control is also executed to shift the next aiming indicator 90A by one appropriate interval. After that, the process returns to S67.

When irradiation of the treatment laser light within one irradiation section is completed (S73: YES), the control unit 60 rotates the spot interval guide 90 by the specified angle of the partially rotatable type contact lens 26 (i.e., the angle for a single irradiation section) (S77). The control unit 60 re-sets the next aiming indicator 90A and the pre-irradiated indicator (S78), and the process returns to S64. When irradiation on all irradiation spots with the treatment laser light is completed (S72: YES), the process ends.

### <Fifth embodiment>

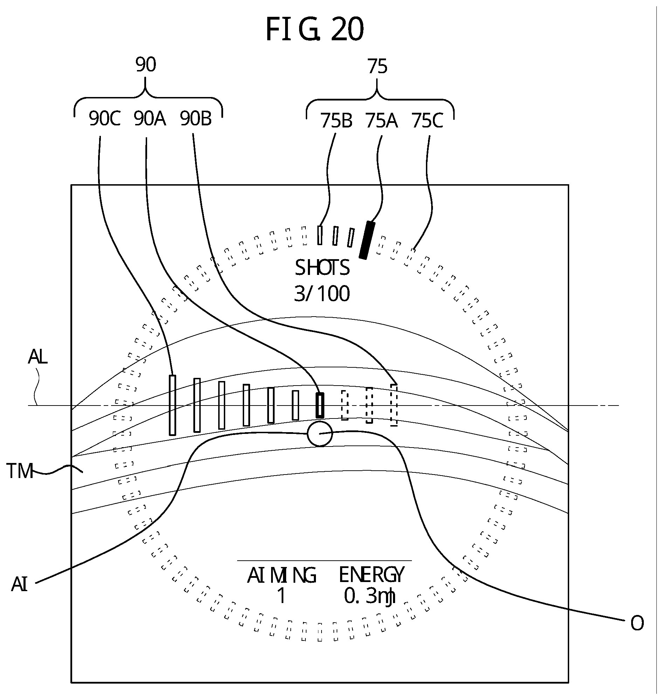

A treatment control process executed by an ophthalmic laser treatment device 1 according to a fifth embodiment will be described with reference to FIGS. 20 to 23. In the fifth embodiment, unlike the third and fourth embodiments, the interval indicators 28 are not formed and treatment is performed using a contact lens which is not a partially rotatable lens. However, at least part of the technique exemplified in the fifth embodiment can be used when treatment is performed using the contact lens 26 provided with the interval indicators 28. In the fifth embodiment, the relative position of the ophthalmic laser treatment device 1 with respect to the patient's eye is shifted when the aiming point is adjusted for each of the plurality of irradiation spots within a single irradiation section. Hereinafter, this adjustment pattern according to the fifth embodiment may be referred to as a "shifting adjustment pattern". However, in the shifting adjustment pattern, in addition to shifting the relative position of the ophthalmic laser treatment device 1 with respect to the patient's eye, the contact lens may also be rotated. In the fifth embodiment, the irradiation section refers to an angle range of an arc area (i.e., a fan-shaped are) where laser irradiation will be performed specified times M (M>=2) based on the spot interval guide 90 (that is, laser irradiation for each of a specified number of the irradiation spots). In the fifth embodiment, the center of the display area in the internal display unit 50, the observation optical axis of the observation optical system 40, the optical axis of the treatment laser light, and the optical axis of the aiming light are also all aligned with each other at the center O. In other words, the operator visually recognizes the spot Al of the aiming light at the center of the observation visual field and the center of the display area of the internal display unit 50. The treatment laser light is applied onto the same spot as the spot Al for the aiming light.

First, with reference to FIG. 20, one example of the operator's observation field of view during treatment by the ophthalmic laser treatment device 1 according to the fifth embodiment will be described. Similar to the fourth embodiment, the ophthalmic laser treatment device 1 in the fifth embodiment also displays the spot interval guide 90 on the internal display unit 50, whereby the operator can grasp the intervals between the plurality of irradiation spots. A spot interval guide 90 indicates, to an operator, an appropriate interval (i.e., a gap) between a plurality of irradiation spots that are scheduled to be irradiated with treatment laser light.

As shown in FIG. 20, the ophthalmic laser treatment device 1 in the fifth embodiment aligns the intervals between the plurality of indicators included in the spot interval guide 90 with the appropriate intervals between the plurality of irradiation spots. Therefore, the operator can easily cause the intervals of the irradiation spot for the treatment laser light to be appropriate intervals by aligning movement distance from the former irradiation spot to the next irradiation spot with the interval of the indicators included in the spot interval guide 90. Therefore, it becomes easier to perform treatment in accordance with the treatment plan more appropriately.

The ophthalmic laser treatment device 1 in the fifth embodiment displays the spot interval guide 90 at a position (in FIG. 20, away in an upper direction) away from the aiming point for the treatment laser light in the display region of the internal display unit 50 (in FIG. 20, a position to which the aiming light Al is emitted). The ophthalmic laser treatment device 1 displays an end of each of the indicators in the spot interval guide 90 close to the aiming point for the laser light (in FIG. 20, a lower end of each of the indicators) along a curve line following the curve of the treatment location of the patient's eye E (in the present embodiment, the trabecular meshwork) having an arc or annular shape. In the fifth embodiment, the spot interval guide 90 is displayed at a position different from the aiming point for the treatment laser light (the position where the aiming light Al is projected) (that is, the spot interval guide 90 does not overlap with the aiming point). Thus, the operator can set the irradiation position of the treatment laser light by appropriately recognizing the state of the tissue at the aiming point. Furthermore, by arranging the end of each of the plurality of indicators close to the aiming point along a curve line (i.e., in an arc shape), when the spot interval guide 90 is placed at an appropriate position relative to the next aiming point, the distance between the aim point side end (the lower end in FIG. 20) of each of the plurality of indicators and the arc-shaped treatment location decreases. As a result, by referring to the spot interval guide 90, the operator can more appropriately adjust the next aiming point of the treatment laser light. **In** the example shown in FIG. 20, the spot interval guide 90 is displayed on a side opposite to the iris when viewed from the treatment site (the trabecular meshwork in this embodiment). However, the spot interval guide may be displayed closer to the iris than the treatment location.

Note that although the shape of the curve of the treatment site of the patient's eye E may be different from other patient's eyes, it does not have a significant change in each patient's eye. Therefore, the aiming point side end of each of the plurality of indicators may be displayed along a predetermined curve that is determined based on the average shape of the treatment area. As one example, in this embodiment, an experiment has been performed in advance where a portion of the model eye that imitates the trabecular meshwork (a simulated trabecular meshwork) is observed under predetermined conditions. A program for displaying the aiming point side end of each of the plurality of indicators is stored in advance in a nonvolatile memory 65 in accordance with the shape of the simulated trabecular meshwork observed in the experiment. Details of the process for displaying the aiming point side end of each of the plurality of indicators will be described later.

Similarly to the fourth embodiment, the ophthalmic laser treatment device 1 displays a next aiming indicator 90A for aligning the next irradiation spot to a position corresponding to the optical axis of the treatment laser light in the spot interval guide 90. The ophthalmic laser treatment device 1 shifts the plurality of indicators included in the spot interval guide 90 in a direction opposite to the forward direction determined by the irradiation plan by a single appropriate interval between the irradiation spots each time irradiation with the treatment laser light is performed. Moreover, the ophthalmic laser treatment device 1 shifts the position of the next aiming indicator 90A in the spot interval guide 90 by one appropriate interval between the plurality of irradiation spots in the forward direction determined by the irradiation plan. As a result, the position of the next aiming indicator 90A is held at the position corresponding to the aiming point. The operator sets the position on the tissue to which the next treatment lase light is applied while considering the position of the next irradiation indicator 90A on the optical axis of the treatment laser light (if the aiming light is emitted, the optical axis of the aiming light).

Among the plurality of indicators in the spot interval guide 90, the indicators other than the next aiming indicator 90A are post-irradiated indicators 90B corresponding to the spots to which the treatment laser light has been applied or pre-irradiated indicators 90C corresponding to the spots to which the treatment laser light has not been applied. The ophthalmic laser treatment device 1 displays each of the next aiming indicator 90A, the post-irradiated indicator 90B, and the pre-irradiated indicator 90C in a different manner (that is, in a manner distinguishable by the operator). Therefore, the operator can easily grasp the positional relationship between the next aiming indicator 90A, the post-irradiated indicator 90B, and the pre-irradiated indicator 90C, making it easier to proceed with the treatment more smoothly.

The ophthalmic laser treatment device 1 shifts the entire spot interval guide 90 in a direction opposite to the forward direction determined by the irradiation plan by a single appropriate interval between the irradiation spots each time irradiation with the treatment laser light is performed. The operator adjusts the aiming point every time so that the position of the plurality of indicators that are shifted each time the treatment laser light is emitted is fixed on the observation image observed through the observation optical system 40. Thus, the aiming point can be adjusted more easily. As described above, in the fifth embodiment, the interval indicators 28 are not formed and treatment is performed using a contact lens which is not a partially rotatable lens. Even in this case, the operator can appropriately adjust each of the plurality of aiming points by appropriately adjusting the positional relationship between the characteristic parts present in the tissue included in the observation image and the plurality of indicators of the spot interval guide 90. For example, the operator identifies one characteristic region on the observation image, and each time the spot interval guide 90 is shifted by one appropriate interval of the irradiation spot (that is, each time the laser irradiation is performed), a shifting instruction to the device may be performed such that a specific indicator among the indicators (for example, the most left side indicator or the second left side indicator) is aligned with the specified characteristic location. In other words, if the spot interval guide 90 includes a plurality of indicators, each of the plurality of aiming points can be adjusted appropriately by aligning at least one of the indicators (i.e., a specific indicators) with the characteristic location.

The ophthalmic laser treatment device 1 inf the fifth embodiment displays elements in each of the indicators included in the spot interval guide 90 by arranging the elements a straight-line. In this embodiment, the ophthalmic laser treatment device 1 displays the indicators 90A, 90B, 90C included in the spot interval guide 90 along a virtual linear angle reference line AL (which is not actually displayed on the internal display unit 50). In this embodiment, the center of gravity of each of the plurality of indicators is displayed along the angle reference line AL. Each time laser irradiation is performed, the ophthalmic laser treatment device 1 shifts the spot interval guide 90 along a linear direction in which the plurality of indicator elements are arranged. In this case, the operator can proceed with the treatment more appropriately by aligning the direction in which the plurality of indicator elements are arranged with the shifting direction in which the aiming point of the treatment laser light is shifted to the next irradiation spot. For example, the reflective surface of a contact lens may include an edge or the like perpendicular to a direction extending outward from the central axis of the lens. In this case, the operator aligns the direction of the edge of the contact lens reflected in the observation image with the linear direction in which multiple index elements are arranged, and aims the treatment laser light along the direction of the edge. It is also possible to move it. In addition, even if the contact lens that was in contact with the patient's eye is once removed from the patient's eye, the operator can orient the edge of the contact lens in the observation image to a straight-line on which multiple index elements are lined up. It is also possible to easily restore the position and direction of the contact lens by adjusting the contact lens to the correct direction.

Specifically, in this embodiment, each of the plurality of indicators is orthogonal to the angle reference line AL, and the center (more specifically, the center of gravity) of each indicator is located on the angle reference line AL. When the plurality of indicators of the spot interval guide 90 are arranged at appropriate positions with respect to the next irradiation spot, each indicator of the spot interval guide 90 extends from the angle reference line AL to a position close to the treatment area (trabeculae) in this embodiment. As a result, the positional relationship between the spot interval guide 90 and the treatment site (for example, the positional relationship between a specific indicator and a characteristic portion in the tissue) can be more easily recognized. Further, the length of each of the plurality of indicators of the spot interval guide 90 is symmetrical about the angle reference line AL. Therefore, the operator can easily grasp the direction in which elements of the plurality of indicators are arranged (that is, the direction in which the angle reference line AL extends), so the operator can easily adjust the angle of the reflective surface of the contact lens to an appropriate angle. For example, by adjusting the angle of the reflective surface of the contact lens so that the direction of the angle reference line AL is in contact with the annular-shaped or arc-shaped treatment location, it is possible to set the angle of the reflective surface to an appropriate angle. Note that the plurality of indicators of the spot interval guide 90 do not have to extend from the angle reference line AL to the vicinity of the treatment site. Further, the indicator displayed near the treatment location may have an appropriate width to absorb individual differences in the curve of the treatment location.

The ophthalmic laser treatment device 1 in the fifth embodiment displays an outer circumferential guide 75 on the internal display unit 50 as an aiming guide. The outer circumferential guide 75 includes a next aiming guide 75A, a completed irradiation guide 75B, and a pre-irradiation guide 75C. The outer circumferential guide 75 may have the same configuration as the outer circumferential guide 75 described in the first embodiment (see FIG. 10). Therefore, a detailed explanation of the outer circumferential guide 75 will be omitted.

Moreover, the ophthalmic laser treatment device 1 in the fifth embodiment displays the total number of irradiation spots determined by the irradiation plan. In the example shown in FIG. 20, the denominator number of "SHOTS" is the total number of irradiation spots. Furthermore, the number of irradiation spots for which treatment has been completed (that is, the total number of irradiation spots for which irradiation with the treatment laser light has been completed and the number of irradiation spots for which irradiation has been skipped) is also displayed. In the example shown in FIG. 20, the molecule number of "SHOTS" is the number of irradiation spots for which treatment has been completed. Each time laser irradiation or irradiation skip is executed, "1" is added to the molecule number of "SHOTS". Further, in the example shown in FIG. 20, the mode of the aiming light Al being used and the energy of the treatment laser light are also displayed.

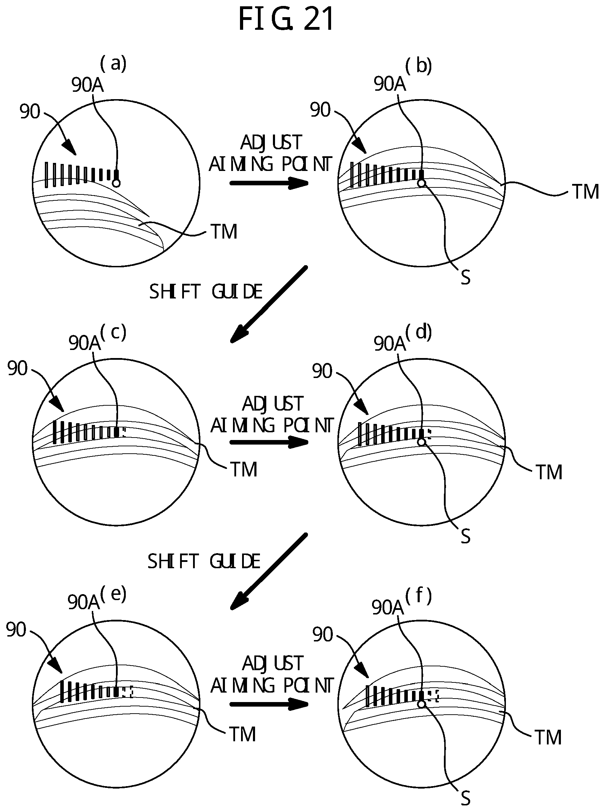

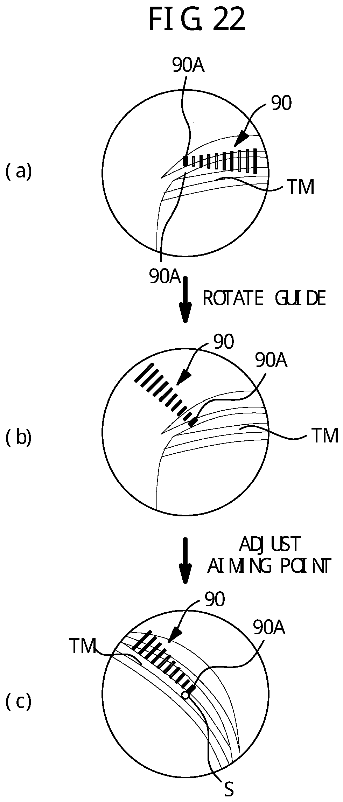

The flow of treatment using the spot interval guide 90 will be described using examples shown in FIGS. 21 and 22. In FIGS. 21 and 22, in order to facilitate understanding of the transition of the display of the spot interval guide 90, a portion of the operator's observation field other than the vicinity of the spot interval guide 90 displayed on the internal display unit 50 is omitted. FIGS. 21 and 22 show one example with an irradiation plan set in advance by the operator where irradiation is performed in a clockwise direction step by step on an actual trabecular meshwork area from the lower side (see FIG. 21) to the diagonally lower left side (see FIG. 22). In FIGS. 21 and 22, the lower trabecular meshwork is reflected by the reflective surface of the contact lens and enters into the operator's observation field. Therefore, in FIGS. 21 and 22, the top and bottom are reversed, and the lower trabecular meshwork is reflected (shown). When the treatment for the irradiation spot proceeds from right to left (that is, a counterclockwise direction) on the reflective surface in FIGS. 21 and 22, in the actual lower trabecular meshwork, the treatment for the irradiation spot proceeds from right to left. (i.e., a clockwise direction).

As shown in FIG. 21 (a), the control unit 60 determines the angle of the spot interval guide 90 to be displayed on the internal display unit 50 according to the progress of the irradiation plan, and displays the spot interval guide 90 at the determined angle. The spot interval guide 90 shown in FIG. 21 is a guide for irradiating a lower area of the actual trabecular meshwork TM 10 times (a specified number of times, that is, 10 times at most). The indicators of the spot interval guide 90 shown in FIG. 21 (a) are arranged in a horizontal direction in order to treat the lower area of the trabecular meshwork TM. The control unit 60 causes the next aiming indicator 90A, to which the next treatment laser light is aimed, to be identifiably displayed in the spot interval guide 90 In the example shown in FIG. 21 (a), the control unit 60 first displays a next aiming indicator 90A at a position of an indicator among plural indicators that is located on an end side of the spot interval guide 90 (in FIG. 21 (a), an indicator located on a right end side of the spot interval guide 90) opposite to a forward direction of treatment that is determined by the irradiation plan. Among the plurality of indicators of the spot interval guide 90, indicators other than the indicators located to be overlapped with the next aiming indicator 90A are pre-irradiation indicators corresponding to at least one of the spots to which the treatment laser light is applied subsequent to the next time. Further, the control unit 60 displays the plurality of indicators of the spot interval guide 90 so that the next aiming indicator 90A is aligned with the optical axis of the treatment laser light emitted by the laser irradiation optical system 10. Note that the position where the aiming indicator 90A is displayed next time does not need to be completely aligned with the optical axis of the treatment laser light. That is, the next aiming indicator 90A is displayed at a position corresponding to the aiming point of the treatment laser light so that the operator can recognize the aiming point to which the treatment laser light is emitted.

The operator adjusts at least one of a rotation angle of the contact lens or a relative position between the patient's eye E that is moved by the joystick 5 and the laser irradiation optical system 10 so that the next aiming indicator 90A included in the spot interval guide 90 is aligned with the first irradiation spot for the treatment laser light in the treatment location of the patient's eye E (in the present embodiment, the trabecular meshwork). Here, if any characteristic location exists in the tissue of the patient's eye E included in the observation image, it would be preferable for the operator to recognize the positional relationship between at least one of the indicators of the spot interval guide 90 (i.e., a specific indicator) and the characteristic location of the tissue. In this case, in subsequent treatment steps, the operator can advance treatment on each of the irradiation spots smoothly by adjusting the positional relationship between the specific indicator and the characteristic location of the tissue to the same positional relationship. As shown in FIG. 21 (b), the operator inputs a irradiation execution instruction for the treatment laser light after setting the position of the next aiming indicator 90A to the position corresponding to the first irradiation spot for the treatment laser light in the treatment location of the patient's eye E (in the present embodiment, the trabecular meshwork). As a result, the treatment laser light is applied to the spot S through which the optical axis of the laser irradiation optical system 10 passes.

As shown in FIG. 21 (c), when the laser irradiation is completed, the control unit 60 moves, by one appropriate interval of the aiming spot, all the indicators of the spot interval guide 90 in a direction (i.e., a left direction which is a counterclockwise direction in FIG. 21) opposite to the forward direction of treatment determined under the irradiation plan (i.e., a right direction in FIG. 21). Further, the control unit 60 moves the position of the next aiming indicator 90A by one appropriate interval of the irradiation spot in the forward direction determined by the irradiation plan. As a result, the position of the next aiming indicator 90A is held at the position corresponding to the aiming point. Further, the control unit 60 changes, to the irradiated indicator, the indicator on which the next aiming indicator 90A was superimposed during the previous laser irradiation. As shown in FIG. 21(d), the operator mainly operates the joystick 5 to move the tissue shown in the observation image in the forward direction of treatment determined by the irradiation plan by the interval between the indicators of the spot interval guide 90, whereby the operator can adjust the next aiming point for the laser light. In addition, the operator adjusts the positional relationship between at least one of the indicators (the specific indicator) and the characteristic location of the tissue to the same positional relationship that was formed at the previous time, whereby the operator can adjust the next aiming point for the laser light. Therefore, the aiming point can be easily adjusted appropriately. The operator inputs an instruction to execute laser irradiation while aligning the position of the next aiming indicator 90A to the position corresponding to the aiming point of the next treatment laser light (i.e., the state shown in FIG. 21(d)).

As shown in FIG. 21 (e), when the laser irradiation on the second irradiation spot is completed, the control unit 60 moves, by one appropriate interval of the irradiation spot, all the indicators of the spot interval guide 90 in a direction opposite to the forward direction determined by the irradiation plan. Further, the control unit 60 moves the position of the next aiming indicator 90A by one appropriate interval of the irradiation spot in the forward direction determined by the irradiation plan. As a result, the position of the next aiming indicator 90A is held at the position corresponding to the aiming point. The operator inputs an instruction to execute laser irradiation while aligning the position of the next aiming indicator 90A with the position corresponding to the next irradiation spot for the laser light (the state shown in FIG. 21 (f)). The above procedure is repeated until all the plurality of spots (10 spots in the example shown in FIGS. 21 and 22) within one irradiation section are irradiated with the treatment laser light. As mentioned above, the irradiation section is an angle range of an arc-shaped area in which laser irradiation will be performed a specified number of times M times (M>=2, M=10 in this embodiment) based on the spot interval guide 90.

FIG. 22 (a) shows a state when the specified times M irradiation on one irradiation section with the treatment laser light (in the example shown in FIGS. 21 and 2, irradiation with the treatment laser lights 10 times) is completed. The control unit 60 automatically rotates the entire indicators of the spot interval guide 90 in the forward direction by the angle corresponding to one irradiation section. Furthermore, the control unit 60 displays the next aiming indicator 90A at a position to be interposed with the indicator, which is an indicator among the plurality of indicators that is located on an end in a direction opposite to the forward direction determined by the irradiation plan. Furthermore, the control unit 60 sets, as unirradiated indicators, all the indicators among the plurality of indicators of the spot interval guide 90 except the indicator at the position with which the next aiming indicator 90A is interposed. Further, the control unit 60 displays the plurality of indicators of the spot interval guide 90 and the next aiming indicator 90A so that the next aiming indicator 90A is aligned with the optical axis of the treatment laser light emitted by the laser irradiation optical system 10. FIG. 22 (b) shows a result of the observation image. The operator rotates the reflective surface of the contact lens in a clockwise direction, which is a forward direction for treatment (see FIG. 22(c)). After that, irradiation by the treatment laser light to all the plurality of spots in the next irradiation section is repeated.

Note that the ophthalmic laser treatment device 1 in the fifth embodiment is configured to receive an instruction for rotating the entire spot interval guide 90 from a user before completing irradiation for all the plurality of irradiation spots in the irradiation section with the treatment laser light (that is, during the treatment on the irradiation spots in the irradiation section). If an instruction to rotate the entire spot interval guide 90 is input before the treatment for all the plurality of irradiation spots in the irradiation section under treatment is completed, the ophthalmic laser treatment device 1 rotates the entire spot interval guide 90 by an angle corresponding to an area in the irradiation section where the treatment has been completed. Therefore, even if the treatment for each irradiation section is not completely completed, the operator can move forward to treatment for the next irradiation section by rotating the entire spot interval guide 90.

Further, the ophthalmic laser treatment device 1 in the fifth embodiment is configured to receive an instruction (hereinafter referred to as a "skip instruction") from a user to omit irradiation with the treatment laser light for the next irradiation spot as defined in the irradiation plan. When the skip instruction is input, the ophthalmic laser treatment device 1 moves, by an appropriate interval between the irradiation spots, the spot interval guide 90 in a forward direction determined by the irradiation plan or in a direction opposite to the forward direction without performing irradiating with the treatment laser light. Therefore, if a non-irradiation area where irradiation with the treatment laser light should not be performed, the operator inputs the skip instruction to skip the irradiation with the treatment laser light for the non-irradiation area. Thus, the operator restarts treatment from the irradiation spot that is planned to be irradiated next. Therefore, it becomes easier for the treatment to proceed more smoothly. That is, the ophthalmic laser treatment device 1 includes a skip means that is configured to skip the step of irradiating, with the treatment laser light, at least one of the plurality of irradiation spots as defined in the treatment plan. Therefore, both benefits provided from the treatment plan (for example, guiding the irradiation with treatment laser light) and flexible treatment realized by the skip means (for example, a step of skipping irradiation with the laser light for the non-irradiated area that was found after starting the treatment in accordance with the treatment plan) can be obtained.

As described above, also in the fifth embodiment, the spot interval guide 90 for allowing the user to grasp the appropriate interval between the plurality of irradiation spots is displayed on the internal display unit 50 according to the progress of the irradiation plan. Therefore, the operator checks the spot interval guide 90 while observing the patient's eye E through the eyepiece 46 (that is, without taking his eyes off the eyepiece 46), and adjusts the plurality of aiming points for the treatment laser light using the confirmed spot interval guide 90 as a reference.

The control unit 60 changes the intervals between the plurality of indicators of the spot interval guide 90 displayed on the internal display unit 50 according to the magnification of the observation optical system 40. Therefore, even if the observation magnification is changed, the spot interval guide 90 corresponding to the appropriate interval between the plurality of irradiation spots can be displayed on the internal display unit 50.

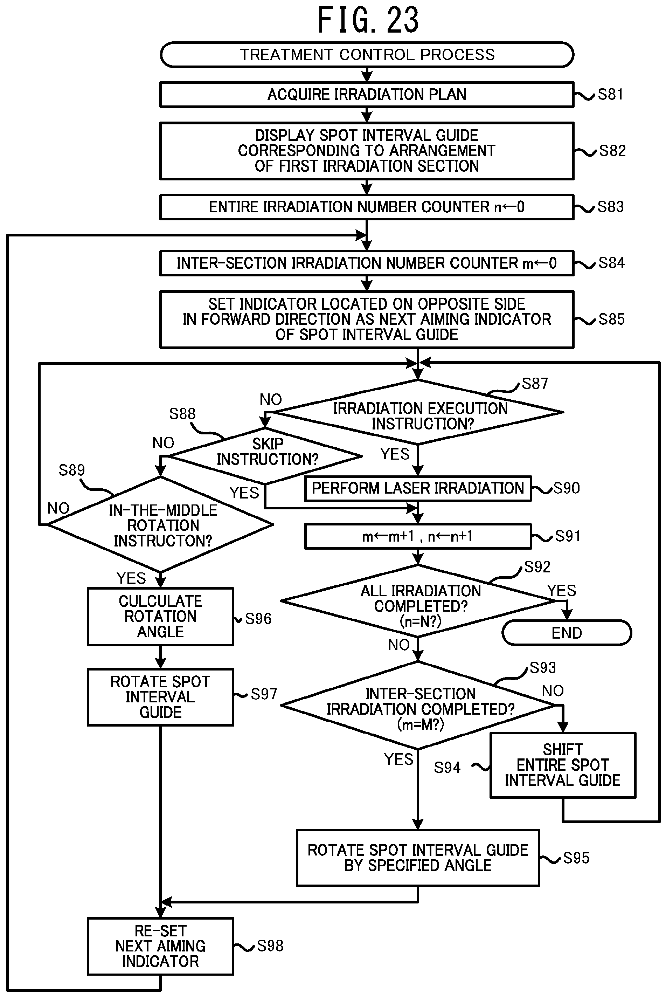

With reference to FIG. 23, a treatment control process in the fifth embodiment will be described. First, the control unit 60 acquires the irradiation plan for treatment laser light on the patient's eye E (S81). The treatment plan acquired at S81 defines an angle between the two adjacent irradiation spots, the angle for one irradiation section, the number of times M the treatment laser light is emitted within each irradiation section, the total number of times the treatment laser light is emitted to the entire treatment target location, the irradiation spot to which the treatment laser light is first applied, the irradiation order (including the irradiation direction) of the treatment laser light, etc. Note that if the angle range of the annular-shaped or arc-shaped treatment target location is R degrees (R<=360) and the number of irradiation spots on which irradiation with the laser light in the treatment target location is performed is N, then the angle between two adjacent irradiation spots is "R/N" degrees. Furthermore, when dividing the range of R degrees in a ringshaped or arc-shaped treatment target location into S irradiation sections, the angle of one irradiation section is "R/S" degrees.

The control unit 60 controls the internal display unit 50 to display the spot interval guide 90 at an angle corresponding to the arrangement of the first irradiation section (S82). The control unit 60 sets the value of the total irradiation number counter "n", which specifies the cumulative number of times the treatment laser light is emitted to the entire treatment target location, to "0" (S83). Furthermore, the control unit 60 sets the value of the single-section irradiation number counter "m" that specifies the number of times the treatment laser light is irradiated within one irradiation section to "0" (S84). The control unit 60 sets a displaying position of the next aiming indicator 90A, which is the indicator for next irradiation with the treatment laser light, among the plurality of indicators of the spot interval guide 90, so that the next aiming indicator 90A is overlapped with an indicator located at a position on an end side opposite to the forward direction in the irradiation order (S85).

Here, one example of a method of displaying ends of the plurality of indicators included in the spot interval guide 90 close to the aiming point along a curve line in S82 and S94 will be described. First, the control unit 60 sets, to a smallest initial value, the length of the indicator for next irradiation with the treatment laser light (i.e., the next aiming indicator 90A) (that is, the length in a direction perpendicular to the angle reference line AL as shown in FIG. 20). Among the plurality of indicators, the control unit 60 increases each of the lengths of indicators (other indicators) other than the next irradiation indicator 90A (the lengths in a direction perpendicular to the angle reference line AL) from the initial value for the length of the next irradiation indicator 90A as the distance between each of the other indicator and the next irradiation indicator 90A increases. The relationship between the distance from the next aiming indicator 90A and the length of each of other indicators may be set in advance such that the ends close to the aiming point (the aiming point side ends) of the other indicators are arranged along the shape of the simulated trabecular meshwork that was observed in the experiment Alternatively, the length of each of the other indicators may be calculated to increase from the initial value in proportion to the distance from the next aiming indicator 90A. Once the lengths of all the other indicators are set, each of the plurality of indicators included in the spot interval guide 90 is displayed on the internal display unit 50 with the set length.

The control unit 60 determines whether the operator has input an instruction for performing irradiation with the treatment laser light (S87). If the irradiation execution instruction is not input (S87: NO), the control unit 60 determines whether an irradiation skip instruction (that is, the "kip instruction") for skipping irradiation to the next irradiation spot as defined by the irradiation plan (S88). If the skip instruction has not been input (S88: NO), the control unit 60 then determines whether an instruction for rotating the entire spot interval guide 90 (hereinafter, may be referred to as an "in-the-middle rotation instruction") is input before laser light irradiation for all the irradiation spots in the irradiation section under treatment is completed (S89). If any instructions are not input at S87-S89, the process repeats S87-S89. During this time, the aiming point of the treatment laser light is adjusted by the operator.

When the instruction to perform laser irradiation is input (S87: YES), the treatment laser light is emitted (S90). Next, the control unit 60 adds "1" to each of the total irradiation number counter "n" and the single-section irradiation number counter "m" (S91). The control unit 60 determines whether the value of the total irradiation number counter "n" has reached the total number "N" the treatment laser light is emitted to the entire treatment target location (S92). If the specified number of irradiations with the treatment laser light to all the irradiation spots (including the number of skipped irradiations in this embodiment) has not been completed (in other words, if "n" has not reached "N") ) (S92: NO), the control unit 60 determines whether the specified number of irradiations with the treatment laser light to the irradiation section during treatment has been completed (that is, if the value of the single-section irradiation number counter "m" has reached the irradiation number "M" for the single irradiation section) (S93). If the irradiation with the treatment laser light to the irradiation section under treatment is not completed (S93: NO), the control unit 60 shifts the entire spot interval guide 90 in a direction opposite to the forward direction of the irradiation spots by one appropriate interval between the irradiation spots (S94). In addition, at S94, control is also executed to shift the next aiming indicator 90A by one appropriate interval. After that, the process returns to S87.

When irradiation with the treatment laser light to one irradiation section is completed (S93: YES), the control unit 60 rotates the entire spot interval guide 90 by the angle for the single irradiation section (a specified angle) (S95). The control unit 60 re-sets the next aiming indicator 90A and the pre-irradiated indicator (S98), and the process returns to S84. When irradiation on all irradiation spots with the treatment laser light is completed (S92: YES), the process ends.

Further, if the skip instruction is input before the instruction to execute laser irradiation is input (S88: YES), the control unit 60 adds "1" to each of the total irradiation number counter "n" and the single irradiation number counter "m" (S91), and executes the processes at S92 to S95 and S98. That is, if irradiation with the treatment laser light to all irradiation spots is not completed (i.e., if "n" has not reached "N") (S92: NO), the control unit 60, without performing laser light irradiation, shifts the entire spot interval guide 90 in the forward direction of the irradiate spots or a direction opposite to the forward direction by a single appropriate interval between the irradiation spots (S94). The direction in which the spot interval guide 90 is shifted may be determined according to an instruction input by the operator. Further, if "n" has reached "N" (S92: YES), the control unit 60 rotates the entire spot interval guide 90 by the specified angle (S95), and re-sets the next aiming indicator 90A and the pre-irradiated indicator (S98).

Furthermore, if the in-the-middle rotation instruction is input before an instruction to execute irradiation with the treatment laser light is input (S89: YES), the control unit 60 calculates an angle corresponding to an area in the irradiation section under treatment where treatment has been done as an angle at which the spot interval guide 90 is rotated (S96). The specific method for calculating the rotation angle at S96 can be selected as appropriate. For example, an angle between the two adjacent irradiation spots when viewed from the center of a virtual circle through which multiple planned irradiation spots pass is defined as A degrees, and the number of the irradiation spots in the irradiation section under treatment where the treatment has been done is defined as "m" (in the present embodiment, the number m is equal to the single section irradiation number counter m). In this embodiment, the control unit 60 calculates the total angle corresponding to the area where the treatment has been done in the irradiation section under the treatment by "A (degrees) x m (number)". The number of irradiation spots "m" for which treatment has been done also includes the number of irradiation spots for which irradiation with the treatment laser light was skipped in accordance with the skip instruction. The control unit 60 rotates the entire spot interval guide 90 by the angle calculated at S96 in the forward direction defined by the treatment plan (S97). Note that if the number "m" of irradiation spots for which the treatment has been done is "0", the angle calculated at S96 is also "0 degree", so the spot interval guide 90 is not rotated at S97. Thereafter, a re-setting process for the next aiming indicator 90A and the pre-irradiated indicator (S98) is performed, and the process returns to S84.

### <Sixth embodiment>

A treatment control process executed by an ophthalmic laser treatment device 1 according to a sixth embodiment will be described with reference to FIGS. 20 and 24 to 27. In the sixth embodiment, as with the fifth embodiment, the interval indicators 28 are not formed and treatment is performed using a contact lens which is not a partially rotatable lens. However, at least part of the technique exemplified in the sixth embodiment can be used when treatment is performed using the contact lens 26 provided with the interval indicators 28. In the sixth embodiment, an adjustment pattern for adjusting the aiming spot for the laser light is a pattern for rotating mainly the contact lens when the aiming point is adjusted for each of the plurality of irradiation spots within a single irradiation section. Hereinafter, this adjustment pattern according to the sixth embodiment may be referred to as a "rotating adjustment pattern". However, in the rotating adjustment pattern, in addition to rotating the contact lens, the relative position of the ophthalmic laser treatment device 1 with respect to the patient's eye may be shifted. In the sixth embodiment, the irradiation section refers to an angle range of an arc area (i.e., a fan-shaped are) where laser irradiation will be performed specified times M (M>=2) based on the spot interval guide 90 (that is, laser irradiation for each of a specified number of the irradiation spots). In the sixth embodiment, the center of the display area in the internal display unit 50, the observation optical axis of the observation optical system 40, the optical axis of the treatment laser light, and the optical axis of the aiming light are also all aligned with each other at the center O. In other words, the operator visually recognizes the spot AI of the aiming light at the center of the observation visual field and the center of the display area of the internal display unit 50. The treatment laser light is applied onto the same spot as the spot AI for the aiming light.

An observation field of view of the operator during treatment at the ophthalmic laser treatment device 1 in the sixth embodiment is similar to the observation field of view described in the fifth embodiment as shown in FIG. 20. AS shown in FIG. 20, in the sixth embodiment, the ophthalmic laser treatment device 1 also displays the spot interval guide 90 on the internal display unit 50, whereby the operator can grasp the intervals between the plurality of irradiation spots. The ophthalmic laser treatment device 1 aligns the intervals between the plurality of indicators included in the spot interval guide 90 with the appropriate intervals between the plurality of irradiation spots. The ophthalmic laser treatment device 1 displays the spot interval guide 90 at a position (in FIG. 20, away in an upper direction) away from the aiming point for the treatment laser light in the display region of the internal display unit 50 (in FIG. 20, a position to which the aiming light AI is emitted). The ophthalmic laser treatment device 1 displays an end of each of the indicators in the spot interval guide 90 close to the aiming point for the laser light (in FIG. 20, a lower end of each of the indicators) along a curve line following the curve of the treatment location of the patient's eye E (in the present embodiment, the trabecular meshwork) having an arc or annular shape. The spot interval guide 90 is displayed at a position different from the aiming point of the treatment laser light (i.e., the position onto which the aiming light Al is projected).

As shown in FIG. 20, the ophthalmic laser treatment device 1 according to the sixth embodiment displays a next aiming indicator 90A for aligning the next irradiation spot to a position corresponding to the optical axis of the treatment laser light in the spot interval guide 90. The ophthalmic laser treatment device 1 shifts and rotates a plurality of indicators included in the spot interval guide 90 each time irradiation with the treatment laser light is performed (details will be described later). Moreover, the ophthalmic laser treatment device 1 shifts the position of the next aiming indicator 90A in the spot interval guide 90 by one appropriate interval between the plurality of irradiation spots in the forward direction determined by the irradiation plan. The operator can more easily adjust the next aiming point by setting the position on the tissue to be irradiated with the next treatment laser light while considering the position of the next aiming indicator 90A on the optical axis of the treatment laser light.

Among the plurality of indicators in the spot interval guide 90, the indicators other than the next aiming indicator 90A are post-irradiated indicators 90B corresponding to the spots to which the treatment laser light has been applied or pre-irradiated indicators 90C corresponding to the spots to which the treatment laser light has not been applied. The ophthalmic laser treatment device 1 displays each of the next aiming indicator 90A, the post-irradiated indicator 90B, and the pre-irradiated indicator 90C in a different manner (that is, in a manner distinguishable by the operator).

As shown in FIG. 20, the ophthalmic laser treatment device 1 according to the sixth embodiment displays elements in each of the indicators included in the spot interval guide 90 by arranging the elements a straight-line. In this embodiment, the ophthalmic laser treatment device 1 displays the indicators 90A, 90B, 90C included in the spot interval guide 90 along a virtual linear angle reference line AL (which is not actually displayed on the internal display unit 50). In this embodiment, the center of gravity of each of the plurality of indicators is displayed along the angle reference line AL. In this case, the operator can proceed with the treatment more appropriately by matching the direction in which the plurality of index elements are arranged with the movement direction in which the aiming point of the treatment laser light is moved to the next aiming point. Specifically, each of the plurality of indicators is orthogonal to the angle reference line AL, and the center (more specifically, the center of gravity) of each indicator is located on the angle reference line AL. The length of each of the plurality of indicators of the spot interval guide 90 is symmetrical about the angle reference line AL. Therefore, the operator can easily grasp the direction in which elements of the plurality of indicators are arranged (that is, the direction in which the angle reference line AL extends), so the operator can easily adjust the angle of the reflective surface of the contact lens to an appropriate angle.

The ophthalmic laser treatment device 1 in the sixth embodiment displays an outer circumferential guide 75 on the internal display unit 50 as an aiming guide. The outer circumferential guide 75 includes a next aiming guide 75A, a completed irradiation guide 75B, and a pre-irradiation guide 75C. The outer circumferential guide 75 may have the same configuration as the outer circumferential guide 75 described in the first embodiment (see FIG. 10). Moreover, the ophthalmic laser treatment device 1 in the sixth embodiment displays the total number of irradiation spots determined by the irradiation plan. Furthermore, the number of irradiation spots for which treatment has been completed (that is, the total number of irradiation spots for which irradiation with the treatment laser light has been completed and the number of irradiation spots for which irradiation has been skipped) is also displayed. Further, in the example shown in FIG. 20, the mode of the aiming light AI being used and the energy of the treatment laser light are also displayed.

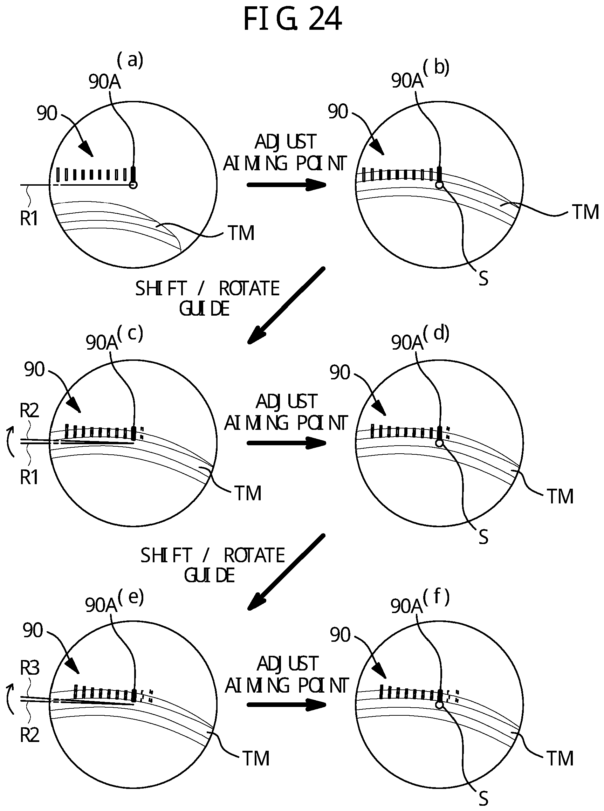

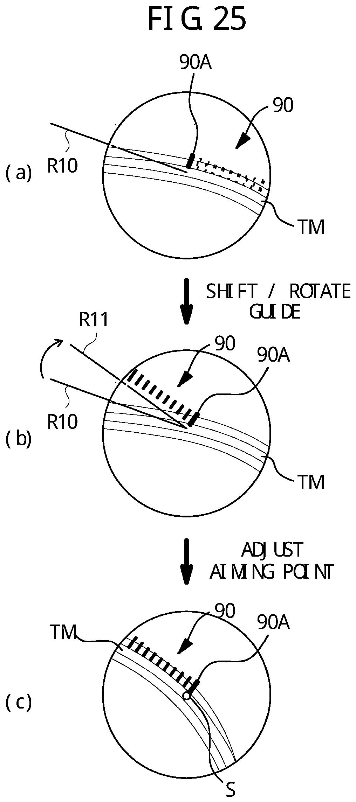

The flow of treatment using the spot interval guide 90 in the sixth embodiment will be described with reference to FIGS. 24 to 26. In FIGS. 24 and 25, in order to facilitate understanding of the transition of the display of the spot interval guide 90, a portion of the operator's observation field other than the vicinity of the spot interval guide 90 displayed on the internal display unit 50 is omitted. FIGS. 24 and 25 show one example with an irradiation plan set in advance by the operator where irradiation is performed in a clockwise direction step by step on an actual trabecular meshwork area from the lower side (see FIG. 24) to the diagonally lower left side (see FIG. 25). In FIGS. 24 and 25, the lower trabecular meshwork is reflected by the reflective surface of the contact lens and enters into the operator's observation field. Therefore, in FIGS. 24 and 25, the top and bottom are reversed, and the lower trabecular meshwork is reflected (shown). When the treatment for the irradiation spot proceeds from right to left (that is, a counterclockwise direction) on the reflective surface in FIGS. 24 and 25, in the actual lower trabecular meshwork, the treatment for the irradiation spot proceeds from right to left. (i.e., a clockwise direction).

First, as shown in FIG. 24 (a), the control unit 60 determines the angle of the spot interval guide 90 to be displayed on the internal display unit 50 (more specifically, the angle at which a plurality of indicators included in the spot interval guide 90 are arranged), and displays the spot internal guide 90 at the determined angle. The spot interval guide 90 shown in FIG. 24 is a guide for irradiating a lower area of the actual trabecular meshwork TM 10 times (a specified number of times, that is, 10 times at most). The multiple indicators of the spot interval guide 90 shown in FIG. 24 (a) are arranged in a horizontal direction in order to treat the lower area of the trabecular meshwork TM. That is, in the state shown in FIG. 24 (a), the plurality of indicators included in the spot interval guide 90 are arranged along the first reference line R1 extending in the horizontal direction.

The control unit 60 causes the next aiming indicator 90A, to which the next treatment laser light is aimed, to be identifiably displayed in the spot interval guide 90 In the example shown in FIG. 24 (a), the control unit 60 first displays a next aiming indicator 90A at a position of an indicator that is located on an end of the spot interval guide 90 (in FIG. 24 (a), an indicator located on a right end of the spot interval guide 90) opposite to a forward direction of treatment that is determined by the irradiation plan. Among the plurality of indicators of the spot interval guide 90, indicators other than the indicators located to be overlapped with the next aiming indicator 90A are pre-irradiation indicators corresponding to at least one of the spots to which the treatment laser light is applied subsequent to the next time. Further, the control unit 60 displays the plurality of indicators of the spot interval guide 90 so that the next aiming indicator 90A is aligned with the optical axis of the treatment laser light emitted by the laser irradiation optical system 10. Note that the position where the aiming indicator 90A is displayed next time does not need to be completely aligned with the optical axis of the treatment laser light. That is, the next aiming indicator 90A is displayed at a position corresponding to the aiming point of the treatment laser light so that the operator can recognize the aiming point to which the treatment laser light is emitted.

The operator adjusts mainly at least one of a rotation angle of the contact lens or a relative position between the patient's eye E that is moved by the joystick 5 and the laser irradiation optical system 10 so that the next aiming indicator 90A included in the spot interval guide 90 is aligned with the first irradiation spot for the treatment laser light in the treatment location of the patient's eye E (in the present embodiment, the trabecular meshwork). As shown in FIG. 24 (b), the operator inputs a irradiation execution instruction for the treatment laser light after setting the position of the next aiming indicator 90A to the position corresponding to the aiming spot for the treatment laser light in the treatment location of the patient's eye E (in the present embodiment, the trabecular meshwork). As a result, the treatment laser light is applied to the spot S through which the optical axis of the laser irradiation optical system 10 passes. Note that in the state shown in FIG. 24 (b), the treatment laser light is reflected on the reflective surface of the contact lens at the right side position with respect to the center of the reflective surface.

As shown in FIG. 24 (c), when the laser irradiation is completed, the control unit 60 moves, by one appropriate interval of the aiming spot, all the indicators of the spot interval guide 90 in a direction (i.e., a left direction which is a counterclockwise direction in FIG. 24) opposite to the forward direction of treatment determined under the irradiation plan (i.e., a right direction in FIG. 24). Further, when the laser irradiation is completed, the control unit 60 rotates all the indicators of the spot interval guide 90 by a unit angle T in the forward direction determined by the irradiation plan (i.e., a clockwise direction in FIG. 24). The unit angle T is a rotation angle of the contact lens required to shift the aiming point for the treatment laser light from the previous irradiation spot to the next irradiation spot. In other words, the control unit 60 rotates the entire spot interval guide 90 arranged along the reference line R1 as illustrated in FIGS. 24 (a) and 24 (b) by the unit angle, thereby arranging the spot interval guide 90 along the second reference line R2 (see FIGS. 24 (c) and 24 (d)). As a result, irradiation with the laser light multiple times in the single irradiation section is appropriately performed according to the progress of the irradiation plan by adjusting the aiming point by rotating the contact lens.

The control unit 60 moves the position of the next aiming indicator 90A by one appropriate interval of the irradiation spot in the forward direction determined by the irradiation plan. As a result, the position of the next aiming indicator 90A is held at the position corresponding to the aiming point. Further, the control unit 60 changes, to the irradiated indicator, the indicator on which the next aiming indicator 90A was superimposed during the previous laser irradiation. As shown in FIG. 24 (d), the operator can adjust the aiming point for the treatment laser light from the previous irradiation spot to the next irradiation spot by rotating the contact lens at the reference angle as described above. In addition, the operator adjusts the positional relationship between at least one of the indicators (the specific indicator) and the characteristic location of the tissue to the same positional relationship that was formed at the previous time, whereby the operator can adjust the next aiming point for the laser light. Therefore, the aiming point can be easily adjusted appropriately. The operator inputs an instruction to execute laser irradiation while aligning the position of the next aiming indicator 90A to the position corresponding to the aiming point of the next treatment laser light (i.e., the state shown in FIG. 24 (d)). In addition, in the state shown in FIG. 24 (d), the treatment laser light is reflected at a position closer to the center of the reflective surface of the contact lens than in the state shown in FIG. 24 (b). In this embodiment, when irradiation with the treatment laser light is repeatedly performed within the same irradiation section, the treatment laser light is reflected at the right side position in the reflective surface of the contact lens in the first half, and then the treatment laser light is reflected at the left side position in the reflective surface in the second half. That is, in this embodiment, when reflecting the treatment laser light, the vicinity around the center of the reflective surface of the contact lens is likely to be used evenly on both sides.

As shown in FIG. 24 (e), when the laser irradiation on the second irradiation spot is completed, the control unit 60 moves, by one appropriate interval of the irradiation spot, all the indicators of the spot interval guide 90 in a direction opposite to the forward direction determined by the aiming plan. Further, when the laser irradiation on the second irradiation spot is completed, the control unit 60 rotates all the indicators of the spot interval guide 90 by a unit angle T in the forward direction determined by the irradiation plan. In other words, the control unit 60 rotates the entire spot interval guide 90 arranged along the reference line R1 as illustrated in FIGS. 24 (c) and 24 (d) by the unit angle, thereby arranging the spot interval guide 90 along the third reference line R3 (see FIGS. 24 (e) and 24 (f)). The control unit 60 moves the position of the next aiming indicator 90A by one appropriate interval of the irradiation spot in the forward direction determined by the aiming plan. As a result, the position of the next aiming indicator 90A is held at the position corresponding to the aiming point. The operator adjusts the position of the next aiming indicator 90A to a position corresponding to the next irradiation spot for the treatment laser light by rotating the contact lens by the reference angle (see FIG. 24 (f)). Thereafter, the operator inputs an instruction for executing laser irradiation. The above procedure is repeated until all the plurality of spots (10 spots in the example shown in FIGS. 24 and 25) within one irradiation section are irradiated with the treatment laser light. As mentioned above, the irradiation section is an angle range of an arc-shaped area in which laser irradiation will be performed a specified number of times M times (M>=2, M=10 in this embodiment) based on the spot interval guide 90.

FIG. 25 (a) shows a observation image immediately before the specified times M irradiation on the first irradiation section with the treatment laser light (in the example shown in FIGS. 24 and 25, irradiation with the treatment laser lights 10 times) is completed. In the state shown in FIG. 25 (a), the spot interval guide 90 is rotated by the unit angle T*(M-1) degrees from the state before treatment laser light on the first irradiation section is performed (see FIGS. 24 (a) and 24 (b)). That is, in the state shown in FIG. 25 (a), the entire spot interval guide 90 is arranged along the tenth reference line R10. When irradiation onto all M irradiation spots within the first irradiation section with the treatment laser light is completed (that is, when irradiation onto the 10th irradiation spot with the treatment laser light is completed), the control unit 60 controls rotates the entire spot interval guide 90 in the forward direction as determined by the irradiation plan, thereby adjusting the angle of the entire spot interval guide 90 to an angle corresponding to the first irradiation spot in the next irradiation section. For example, assume that the range of R degree (R<=360) in an annular or arcuate treatment region is divided into S irradiation sections, and each irradiation section is irradiated with the treatment laser light M times. During treatment in one irradiation section, when irradiation with the treatment laser light is completed M times, the total rotation angle of the spot interval guide 90 is (T*(M -1)) degrees. Further, the angular range of one irradiation section is R/S degrees. Therefore, when irradiation with the treatment laser light onto all the plurality of irradiation spots in the irradiation section under treatment is completed, the control unit rotates the spot interval guide 90 by (R/S-T*(M-1)) degrees. As a result, in the states shown in FIGS. 25 (b) and 25 (c), the entire spot interval guide 90 is arranged along the eleventh reference line R11. Furthermore, the control unit 60 displays the next aiming indicator 90A at a position to be interposed with the indicator, which is an indicator among the plurality of indicators that is located on an end in a direction opposite to the forward direction determined by the irradiation plan. Furthermore, the control unit 60 sets, as unirradiated indicators, all the indicators among the plurality of indicators of the spot interval guide 90 except the indicator at the position with which the next aiming indicator 90A is interposed. Further, the control unit 60 displays the plurality of indicators of the spot interval guide 90 and the next aiming indicator 90A so that the next aiming indicator 90A is aligned with the optical axis of the treatment laser light emitted by the laser irradiation optical system 10. FIG. 25 (b) shows a result of the observation image. The operator rotates the reflective surface of the contact lens and operates the joystick to shift the position of the optical axis of the treatment laser light to align the aiming point of the treatment laser light with the position of the next aiming indicator 90A(See FIG. 25 (c)). After that, irradiation by the treatment laser light to all the plurality of spots in the second irradiation section is repeated.

Note that the ophthalmic laser treatment device 1 in the sixth embodiment is configured to receive an instruction for rotating the entire spot interval guide 90 from a user before completing irradiation for all the plurality of irradiation spots in the irradiation section with the treatment laser light (that is, during the treatment on the irradiation spots in the irradiation section). If an instruction to rotate the entire spot interval guide 90 is input before the treatment for all the plurality of irradiation spots in the irradiation section under treatment is completed, the ophthalmic laser treatment device 1 rotates the entire spot interval guide 90 by an angle corresponding to an area in the irradiation section where the treatment has been completed.

Further, the ophthalmic laser treatment device 1 in the sixth embodiment is configured to receive an instruction (hereinafter referred to as a "skip instruction") from a user to omit irradiation with the treatment laser light for the next irradiation spot as defined in the irradiation plan. When the skip instruction is input, the ophthalmic laser treatment device 1 moves, by an appropriate interval between the irradiation spots, the spot interval guide 90 in a forward direction determined by the aiming plan or in a direction opposite to the forward direction without performing irradiating with the treatment laser light.

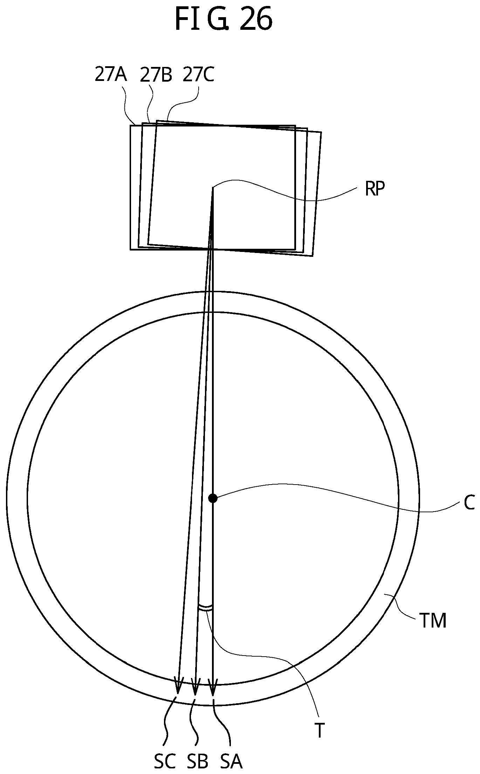

Referring to FIG. 26, a rotation angle of the contact lens (i.e., a unit angle T) will be described below. The unit angle T is used to move the aiming point of the treatment laser light to the next irradiation spot when the relative position of the device with respect to the patient's eye is not moved (that is, the optical axis of the treatment laser light is not moved), In the example shown in FIG. 26, it is assumed that irradiation with the laser light is performed N times (in the present embodiment, 100 times) at equal intervals within R degrees angle region of the annular-shaped or arc-shaped treatment target location (in the present embodiment, trabecular meshwork TM). In addition, it is assumed that a virtual approximate circle (that is, a circle through which a plurality of planned irradiation spots S passes) passing through a annular-shaped or arc-shaped treatment target location. When the patient's eye is viewed from a direction along the visual axis (i.e., the state shown in FIG. 26), if the treatment laser light reflected by the reflective surface 27 of the contact lens always intersects the center C of the approximate circle, the unit angle T is the R/N degrees (3.6 degrees in this embodiment). In this case, when the patient's eye is viewed from a direction along the visual axis, the radius of gyration of the treatment laser light is equal to the radius of the approximate circle (that is, the distance from the center C to the irradiation spot S).

However, if the aiming point is shifted to the next irradiation spot only by rotating the contact lens without moving the relative position of the device with respect to the patient's eye, even when viewed in the visual axis direction of the patient's eye, the laser light does not always intersect the center C of the approximate circle. That is, in the rotation adjustment pattern, the treatment laser light is rotated around a reflection position RP on the reflective surface 27 of the contact lens. In fact, in the example shown in FIG. 26, if the angle of the reflective surface 27 of the contact lens is the angle for irradiating the first irradiation spot SA with the treatment laser light (i.e., the angle indicated by 27A), the treatment laser light intersects the center C of the approximate circle when viewed is the visual axis direction of the patient's eye. However, if the angle of the reflective surface 27 of the contact lens is the angle for irradiating the second irradiation spot SB with the treatment laser light (i.e., the angle indicated by 27B), the treatment laser light does not intersect the center C of the approximate circle when viewed is the visual axis direction of the patient's eye. Further, if the angle of the reflective surface 27 of the contact lens is the angle for irradiating the third irradiation spot SC with the treatment laser light (i.e., the angle indicated by 27C), the treatment laser light does not intersect the center C of the approximate circle when viewed in the visual axis direction of the patient's eye. As shown in FIG. 26, when the angle of the reflective surface 27 of the contact lens 27 is rotated while maintaining the position of the optical axis of the treatment laser light, the treatment laser light turns around the reflection position RP on the reflective surface 27.

As shown in FIG. 26, when the angle of the reflective surface 27 of the contact lens 27 is rotated while maintaining the position of the optical axis of the treatment laser light, the turning radius of the laser light (i.e., a distance to the irradiation spots SA, SB, SC from the reflection position RP) is longer than the radius of the approximate circle (i.e., the distance to the irradiation spot SA, SB, SC from the center C) when viewed in the visual axis direction of the patient's eye. As a result, the unit angle T in the rotation adjustment pattern when the eye to be examined is viewed from the direction along the visual axis becomes smaller than R/N degrees. Therefore, the unit angle T in the rotation adjustment pattern may be set, within a range smaller than R/N degrees, in accordance with the turning radius of the laser light when viewed in the visual axis direction of the patient's eye (i.e., a distance between the reflective position RP in the reflective surface 27 of the contact lens and the irradiation spots SA,SB,SC onto which the laser light is applied.

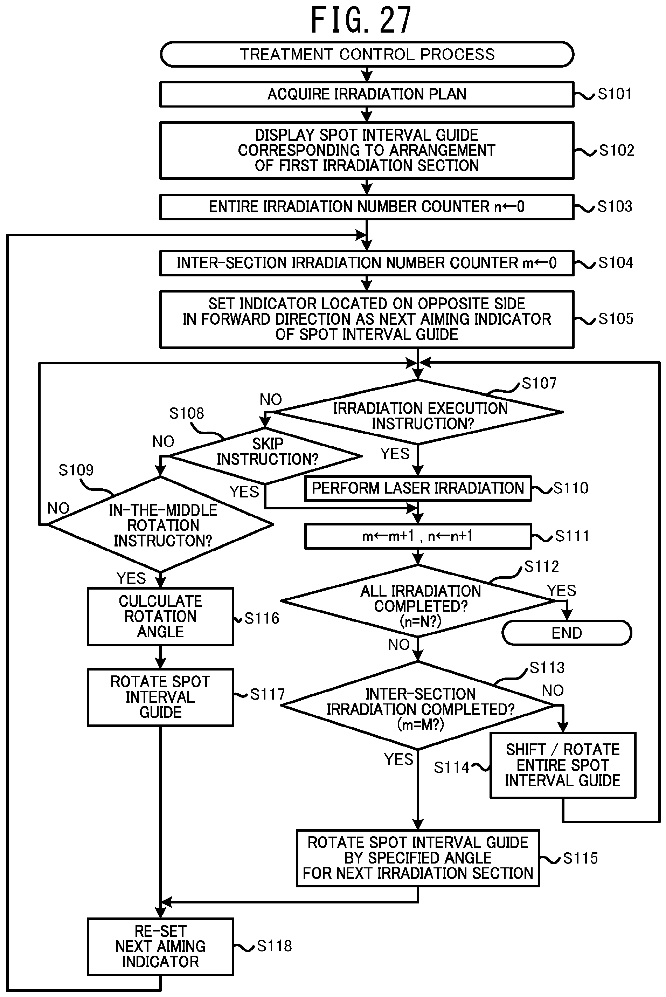

With reference to FIG. 27, a treatment control process in the sixth embodiment will be described. First, the control unit 60 acquires the irradiation plan for treatment laser light on the patient's eye E (S101). The treatment plan acquired at S101 defines an angle between the two adjacent irradiation spots, the angle for one irradiation section, the number of times M the treatment laser light is emitted within each irradiation section, the total number of times the treatment laser light is emitted to the entire treatment target location, the irradiation spot to which the treatment laser light is first applied, the irradiation order (including the irradiation direction) of the treatment laser light, etc.

The control unit 60 controls the internal display unit 50 to display the spot interval guide 90 at an angle corresponding to the arrangement of the first irradiation section (S102). The control unit 60 sets the value of the total irradiation number counter "n", which specifies the cumulative number of times the treatment laser light is emitted to the entire treatment target location, to "0" (S103). Furthermore, the control unit 60 sets the value of the single-section irradiation number counter "m" that specifies the number of times the treatment laser light is irradiated within one irradiation section to "0" (S104). The control unit 60 sets a displaying position of the next aiming indicator 90A, which is the indicator for next irradiation with the treatment laser light, among the plurality of indicators of the spot interval guide 90, so that the next aiming indicator 90A is overlapped with an indicator located at a position on an end side opposite to the forward direction in the irradiation order (S105). Note that a method similar to the method shown in the fifth embodiment can be adopted as a method for displaying ends of the plurality of indicators included in the spot interval guide 90 close to the aiming point along a curve line.

The control unit 60 determines whether the operator has input an instruction for performing irradiation with the treatment laser light (S107). If the irradiation execution instruction is not input (S107: NO), the control unit 60 determines whether an irradiation skip instruction (that is, the "skip instruction") for skipping irradiation to the next irradiation spot as defined by the irradiation plan (S108). If the skip instruction has not been input (S108: NO), the control unit 60 then determines whether an instruction for rotating the entire spot interval guide 90 (hereinafter, may be referred to as an "in-the-middle rotation instruction") is input before laser light irradiation for all the irradiation spots in the irradiation section under treatment is completed (S109). If any instructions are not input at S87-S89, the process repeats S107-S109. During this time, the aiming point of the treatment laser light is adjusted by the operator.

When the instruction to perform laser irradiation is input (S107: YES), the treatment laser light is emitted (S110). Next, the control unit 60 adds "1" to each of the total irradiation number counter "n" and the single-section irradiation number counter "m" (S111). The control unit 60 determines whether the value of the total irradiation number counter "n" has reached the total number "N" the treatment laser light is emitted to the entire treatment target location (S112). If the specified number of irradiations with the treatment laser light to all the irradiation spots (including the number of skipped irradiations in this embodiment) has not been completed (in other words, if "n" has not reached "N") ) (S112: NO), the control unit 60 determines whether the specified number of irradiations with the treatment laser light to the irradiation section during treatment has been completed (that is, if the value of the single-section irradiation number counter "m" has reached the irradiation number "M" for the single irradiation section) (S113). If the irradiation with the treatment laser light to the irradiation section under treatment is not completed (S113: NO), the control unit 60 shifts the entire spot interval guide 90 in a direction opposite to the forward direction of the irradiation spots by one appropriate interval between the irradiation spots. Further, the control unit 60 rotates the entire spot interval guide 90 by the unit angle T as described above (S114). In addition, at S114, control is also executed to shift the next aiming indicator 90A by one appropriate interval. After that, the process returns to S107.

When irradiation with the treatment laser light to one irradiation section is completed (S113: YES), the control unit 60 rotates the entire spot interval guide 90 to an angle corresponding to the next irradiation section (S115). The control unit 60 re-sets the next aiming indicator 90A and the pre-irradiated indicator (S118), and the process returns to S104. When irradiation on all irradiation spots with the treatment laser light is completed (S112: YES), the process ends.

Further, if the skip instruction is input before the instruction to execute laser irradiation is input (S108: YES), the control unit 60, without emitting the laser light, adds "1" to each of the total irradiation number counter "n" and the single irradiation number counter "m" (S111), and executes the processes at S112 to S115 and S118. Furthermore, if the in-the-middle rotation instruction is input before an instruction to execute irradiation with the treatment laser light is input (S109: YES), the control unit 60 calculates an angle corresponding to an area in the irradiation section under treatment where treatment has been done as an angle at which the spot interval guide 90 is rotated (S116). The specific method for calculating the rotation angle at S116 can be selected as appropriate. For example, an angle between the two adjacent irradiation spots when viewed from the center of a virtual circle through which multiple planned irradiation spots pass is defined as A degrees, the unit angle as described above is T degrees, and the number of the irradiation spots in the irradiation section under treatment where the treatment has been done is defined as "m" (in the present embodiment, the number m is equal to the single section irradiation number counter m). In this embodiment, the control unit 60 calculates the total angle corresponding to the area where the treatment has been done in the irradiation section under the treatment by "(A (degrees) - T (degrees)) x m (number)". The number of irradiation spots "m" for which treatment has been done also includes the number of irradiation spots for which irradiation with the treatment laser light was skipped in accordance with the skip instruction. The control unit 60 rotates the entire spot interval guide 90 by the angle calculated at S116 in the forward direction defined by the treatment plan (S117). Thereafter, a re-setting process for the next aiming indicator 90A and the pre-irradiated indicator (S118) is performed, and the process returns to S104.

### <Seventh embodiment>

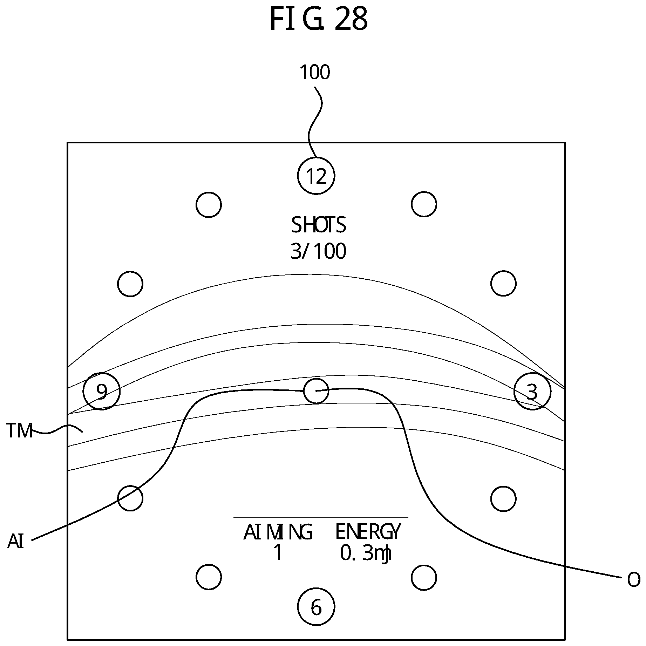

Referring to FIG. 28, an ophthalmic laser treatment device 1 according to a seventh embodiment will be described. The ophthalmic laser treatment device 1 in the seventh embodiment includes a laser irradiation optical system 10 (see FIG. 2) and a superimposing display section. The superimposing display unit displays a superimposed image by superimposing a circumferential chart 100 (details will be described later) on an observation image that includes at least a part of an annular-shaped or arc-shaped treatment target location (in this embodiment, the trabecular meshwork TM) in the patient's eye. The internal display unit 50 (see FIGS. 2 and 3) described in the first to sixth embodiments is used as the superimposing display unit in the seventh embodiment. However, it is also possible to use a configuration other than the internal display unit 50 as the superimposing display unit. For example, a transmissive electroluminescent (EL) panel (which may be organic or inorganic) may be used as the superimposing display unit. Furthermore, by forming the circumferential chart 100 on the surface of a plate glass by vapor deposition or the like and illuminating the formed vapor deposition location with a separate light source, the circumferential chart 100 may be displayed in a predetermined color (for example, green, etc.) in a superimposed manner. In addition, it is more preferable that the circumferential chart 100 can be illuminated (in other words, brightly) and presented to an operator. In this case, the operator can easily grasp the circumferential chart 100 even if, for example, part of or all of the observation image is dark depending on the observation conditions. Further, the ophthalmic laser treatment device 1 may include an observation and imaging unit that captures an observation image of the patient's eye. The superimposing display unit (for example, a monitor) may superimpose and display another image or the like on the observation image captured by the observation and imaging unit. The configuration of the superimposing display unit may be a simple configuration in which it is impossible to change the image displayed superimposed on the observation image.

As shown in FIG. 28, the superimposing display unit of the ophthalmic laser treatment device 1 in the seventh embodiment superimposes a circumferential chart 100 that indicates the angle in the circumferential direction about an optical axis O of the treatment laser light on an observation image. Therefore, by visually recognizing the circumferential chart 100 superimposed on the observation image, the operator can appropriately grasp the direction of the treatment target location with respect to the optical axis of the treatment laser light. For example, the contact lens may be temporarily removed from the patient's eye during treatment on multiple irradiation spots. In this case, the operator should circle the treatment target location in the direction in which the spot irradiated with the treatment laser light immediately before removing the contact lens (the angle in the circumferential direction centered on the optical axis of the treatment laser light) is located. It can be properly understood by referring to the circumferential chart. Therefore, when reattaching a contact lens that has been removed to the patient's eye, the contact lens should be adjusted while referring to the circumferential chart so that the aiming point of the treatment laser light can be adjusted appropriately in the direction where the next spot will be located. The angle can be adjusted. Therefore, the operator can easily and appropriately adjust the aiming point of the treatment laser light.

As shown in FIG. 28, the internal display unit aligns the center of the circumferential chart 100 displayed and superimposed on the observation image with the passing position of the optical axis O of the treatment laser light in the observation image. Therefore, the direction of the treatment target location with respect to the center of the optical axis O of the treatment laser light can be more appropriately recognized using the circumferential chart 100.

The circumferential chart 100 illustrated in FIG. 28 includes a plurality of indicators arranged at equal angles in a circumferential direction about the center of the optical axis O of the treatment laser light. Therefore, the direction of the treatment target location can be more easily grasped by the plurality of indicators arranged at equal angles. Specifically, the superimposing display unit of the seventh embodiment displays symbols (for example, numbers up to 12, etc.) that can be displayed in at least one of the directions of the dial of the analog clock at appropriate positions in the circumferential chart 100 (in the example shown in FIG. 28, at 12 o'clock, 3 o'clock, 6 o'clock, and 9 o'clock positions). In other words, the circumferential chart 100 is displayed in accordance with the clock position. Therefore, the operator can appropriately grasp the direction of the treatment target location with respect to the optical axis of the treatment laser light, in the same sense as when grasping the time using an analog clock. Note that it is also possible to change the specific design of the circumferential chart. For example, the indicator placed on the right side of the optical axis O of the treatment laser light may be set to 0 degree, and a plurality of indicators (for example, indicators in the form of angular values) may be displayed every 15 degrees in a counterclockwise direction. The shape of one or more indicators may be different from the shape of other indicators. Some or all of the indicators may be composed only of letters (or numbers), or may be composed of a combination of letters and shapes. When using letters as indicators, the angular intervals between the letters are preferably 15 degrees or more. Further, a plurality of indicators may be connected to each other. A plurality of circumferential charts having different aspects may be stored in the storage device. In this case, the operator may be able to select one of circumferential charts to be displayed on the superimposing display unit.

The superimposing display unit fixedly displays the circumferential chart 100 at a predetermined position within the image display area. Therefore, regardless of the state of the observation image, the treatment plan, the cumulative number of irradiations with the treatment laser light, etc., the circumferential chart 100 is fixedly displayed at a predetermined position, and therefore the direction of the treatment target location with the center of the optical axis O of the treatment laser light can be more accurately understood. In the seventh embodiment, the relative positional relationship between the optical axis of the optical system for causing the operator to visually recognize the observation image and the optical axis O of the treatment laser light is also fixed. In other words, the optical axis of the observation optical system and the optical axis O of the treatment laser light are always aligned with each other.

The superimposing display unit can switch between superimposed display mode and non-display mode. Therefore, when there is no need for the operator to grasp the direction of the treatment target location, the circumferential chart 100 is not displayed so that the operator can concentrate on observing the observation image. In the seventh embodiment, the control unit 60 determines whether the circumferential chart 100 is to be displayed based on whether a treatment mode set by the operator is an annular treatment mode whether treatment on an annular-shaped or arc-shaped treatment target location is performed. Therefore, a field of view in accordance to the set treatment mode is appropriately provided to the operator. Note that the control unit 60 may change the shape of the circumferential chart 100 based on whether the treatment mode selected by the operator is the annular treatment mode. For example, the control unit 60 may display the circumferential chart 100 in a superimposed manner in the annular treatment mode, while displaying a cross-shaped chart (for example, a reticle for aiming) in a superimposed manner in other treatment modes. Further, the control unit 60 may switch the circumferential chart 100 illustrated in FIG. 28 and the outer circumferential guide 75 illustrated in FIGS. 10 and 20.

At least any of the embodiments disclosed above may be executed according to a set irradiation plan. As mentioned above, the irradiation plan is formulated by setting at least one of the following: the irradiation area, the number of irradiations, the type of contact lens to be used, and the method of adjusting the aiming point to the adjacent irradiation spot (see FIG. 9). The control unit 60 may display information on the internal display unit 50 based on the irradiation plan. For example, if the contact lens shown in FIG. 6 is selected, the display control according to the fourth embodiment may be performed. When a contact lens that is entirely rotated to adjust the angle of the reflective surface is used, the display control according to the first embodiment or the fifth embodiment may be performed. Upon receiving a trigger signal, the control unit 60 emits the treatment laser light and changes displaying contents on the internal display unit 50 based on the irradiation plan.

The embodiments disclosed herein are illustrative in all respects and should not be considered restrictive. The scope of the present invention is indicated by the claims rather than the above description, and it is intended that all changes within the meaning and scope of the claims are included. For example, it is also possible to adopt only some of the techniques described in each of the first to fifth embodiments. Further, it is also possible to appropriately combine the techniques of a plurality of different embodiments and employ them in an ophthalmic laser treatment device.

The configuration of the spot interval guide in the fourth embodiment is one example. Therefore, it is also possible to change the configuration of the spot interval guide. That is, if the operator is properly guided to adjust the observation image (for example, adjustment by operating the joystick 5, and adjustment by rotating or shifting the contact lens) such that the spot interval guide displayed by the internal display unit 50 is located at a specific position in the observation image observed by the operator via the observation optical system 40 (for example, a specific position relative to the reflected image 28Z of the interval indicators 28 in the observation image, or a specific position relative to at least part of an outline of the contact lens in the observation image), a user can properly grasp an appropriate interval between the irradiation spots. Note that the spot interval guide may also be referred to as an alignment guide for aligning the aiming point with the desired portion of the trabecular meshwork TM.

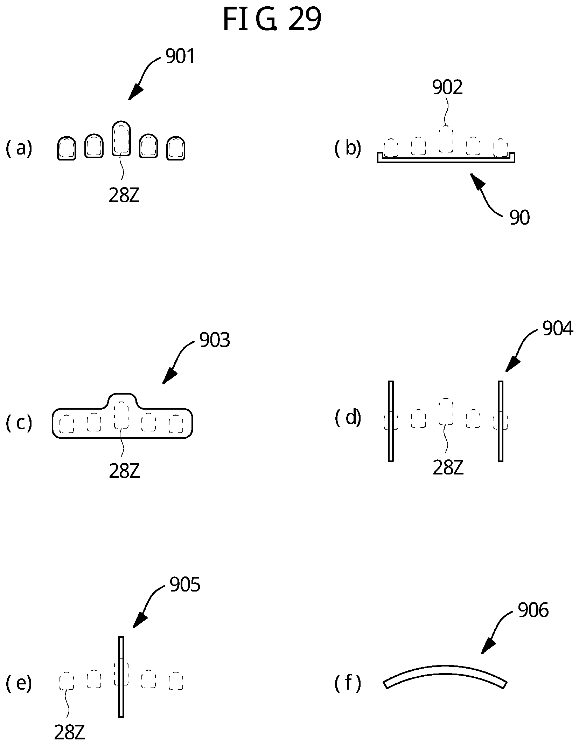

FIG. 29 shows a modification example to the fourth embodiment on the spot interval guide. In FIGS. 29 (a) to 29 (e), a reflected image 28Z of interval indicators 28 (see FIG. 7) provided on a contact lens 26 is schematically shown by a dotted line. As shown in FIG. 29 (a), the spot interval guide 901 may include a plurality of graphics each having a shape corresponding to each of the plurality of reflected images 28Z of the interval indicators 28. As shown in FIG. 29 (b), the spot interval guide 902 may include a graphic corresponding to the length from the left end to the right end of the reflected images 28Z of the interval indicators 28. As shown in FIG. 29 (c), the spot interval guide 903 may include a graphic that entirely surrounds the plurality of reflected images 28Z of the interval indicators 28. As shown in FIG. 29 (d), the spot interval guide 904 includes two graphics that correspond to the reflected images of the leftmost indicator and the reflected image of the rightmost indicator among the plurality of reflected images 28Z of the interval indicators 28. As shown in FIG. 29 (e), the spot interval guide 901 may be one graphic that corresponds to the reflected image of the middle indicator among the plurality of reflected images 28Z of the interval indexes 28. As described above, the configuration of the spot interval guide that is aligned with the reflected image 28Z of the interval indicator 28 can be selected as appropriate. Further, as shown in FIG. 29 (f), the spot interval guide may be aligned with a part of the contact lens 26 included in the observation image instead of the reflected images 28Z of the interval indicators 28. The spot interval guide 906 shown in FIG. 29 (f) includes an arc-shaped graphic that follows the arc-shaped contour of the contact lens 26 in the observation image.

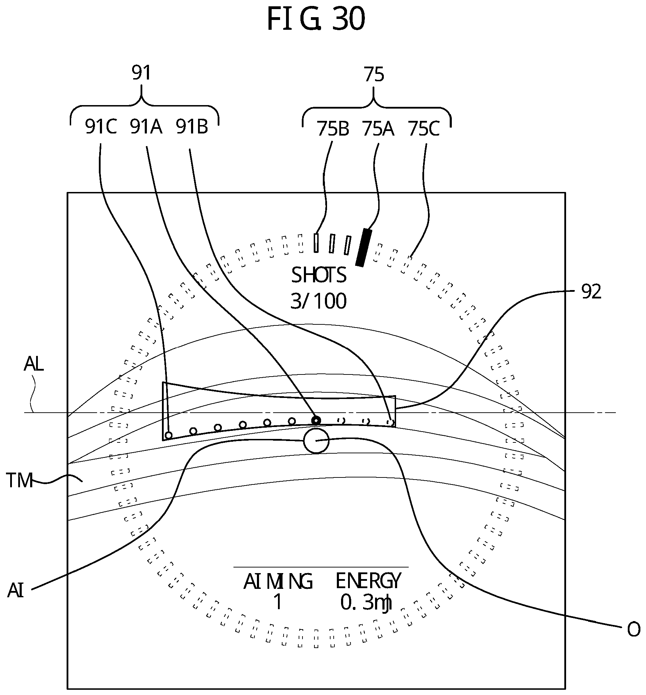

The configuration of the spot interval guide in the fifth embodiment and the sixth embodiment is also only one example. FIG. 30 shows a modification example of the spot interval guide to the fifth and sixth embodiments. Each of the plurality of indicators of the spot interval guide 90 described in the fifth and sixth embodiments has a rectangular shape extending in a direction perpendicular to the angle reference line AL (see FIG. 20). However, it is also possible to change the shape of each of the plurality of indicators included in the spot interval guide. For example, in the example shown in FIG. 30, each of the plurality of indicators (the next aiming indicator 91A, the post-irradiated indicator 91B, and pre-irradiated indicator 91C) included in the spot interval guide 91 is circular-shaped. Note that indicators such as line, regular polygons, and star shapes may be displayed as indicators of the spot interval guide.

Further, the spot interval guide 91 shown in FIG. 30 includes a frame portion 92 surrounding a plurality of indicators. The frame portion 92 shown in FIG. 30 surrounds the plurality of indicators and extends in the direction along the angle reference line AL. Therefore, the operator can easily adjust the angle of the reflective surface of the contact lens to an appropriate angle based on the direction in which the frame portion 92 extends. Furthermore, in the example shown in FIG. 30, at least some elements in the spot interval guide 91 (in FIG. 30, the end of the frame portion 92 close to the aiming point) are displayed along a curve line that follows the curve of an arc-shape or annular-shape treatment location of the patient's eye E. As a result, when the spot interval guide 91 is placed at an appropriate position relative to the next aiming point, an end of the frame portion 92 close to the aiming point becomes closer to the arc-shape treatment location. Therefore, by referring to the spot interval guide 91, the operator can more appropriately adjust the next aiming point of the treatment laser light.

## Claims

1. An ophthalmic laser treatment device that irradiates a tissue of a patient's eye with treatment laser light each time an instruction for performing laser irradiation is input, the device comprising:
a laser irradiation optical system that irradiates the patient's eye with treatment laser light;
an observation optical system that allows an operator to observe an observation image of the patient's eye through an eyepiece;
an internal display unit that is disposed in the observation optical system and is configured to display an image to the operator through the eyepiece; and
a control unit that is configured to perform, when irradiating the patient's eye with treatment laser light using a contact lens having a reflective surface that reflects the treatment laser light in a direction intersecting an optical axis:
an irradiation plan acquisition step of acquiring an irradiation plan that defines an order for irradiating a plurality of irradiation spots with the treatment laser light, the plurality of irradiation spots being scheduled to be irradiated with the treatment laser light; and
an aiming guide display step of controlling the internal display unit to display an aiming guide in accordance with a progress of the irradiation plan to assist the operator in adjusting an aiming spot for the treatment laser light in an appropriate position.

2. The ophthalmic laser treatment device according to claim 1, wherein
the internal display unit has a display area, and
the display area has a center that is aligned with the optical axis of the observation optical system.

3. The ophthalmic laser treatment device according to claim 1 or 2, wherein
in the aiming guide display step, the control unit is configured to perform a spot interval guide display step of controlling the internal display unit to display, as the aiming guide, a spot interval guide in accordance with the progress of the irradiation plan, and
the spot internal guide indicates an appropriate interval between the plurality of irradiation spots that are scheduled to be irradiated with the treatment laser light.

4. The ophthalmic laser treatment device according to claim 3, wherein
the control unit is configured to change a size of the spot interval guide displayed on the internal display unit according to magnification of the observation optical system.

5. The ophthalmic laser treatment device according to claim 3 or 4, wherein
the control unit is configured to adjust at least one of intervals between a plurality of indicators included in the spot interval guide to match the appropriate interval between the plurality of irradiation spots.

6. The ophthalmic laser treatment device according to any one of claims 3 to 5, wherein
the spot interval guide has a shape along an appropriate direction of reflection of the treatment laser light to a next one of the irradiation spots by the reflective surface of the contact lens, and
the control unit is further configured to:
determine an angle of the spot interval guide to be displayed on the internal display unit in accordance with the progress of the irradiation plan; and
control the internal display unit to display the spot interval guide at the determined angle.

7. The ophthalmic laser treatment device according to any one of claims 3 to 6, wherein
the spot interval guide includes a plurality of indicators having ends close to the aiming spot for the treatment laser light, and
the control unit is further configured to:
control the internal display unit to display the spot interval guide in the display area of the internal display unit at a position spaced away from the aiming point for the treatment laser light; and
control the internal display unit to display the ends of the plurality of indicators to be arranged along a cure line similar to a curve of an arc-shaped or annular-shaped treatment location of the patient's eye.

8. The ophthalmic laser treatment device according to any one of claims 3 to 7, wherein
the control unit is further configured to:
control the internal display unit to display a next aiming indicator at a position on the optical axis of the treatment laser light emitted by the laser irradiation optical system for a next one of the irradiation spots to be aligned with the next aiming indicator; and
shift a position of the next aiming indicator in the spot interval guide by one appropriate interval between the plurality of irradiation spots in a forward direction determined by the irradiation plan each time laser irradiation is performed.

9. The ophthalmic laser treatment device according to any one of claims 3 to 8, wherein
the control unit is further configured to shift the spot interval guide by one appropriate interval between the plurality of irradiation spots in a direction opposite to a forward direction determined by the irradiation plan each time laser irradiation is performed.

10. The ophthalmic laser treatment device according to any one of claims 3 to 9, wherein
a treatment target location of the patient's eye onto which the laser irradiation is performed has an arc shape or an annular shape, and
the control unit is further configured to rotate the entire spot interval guide.

11. The ophthalmic laser treatment device according to claim 9 or 10, wherein
a treatment target location of the patient's eye onto which the laser irradiation is performed has an arc shape or an annular shape,
an angular range of an arc-shaped region onto which the laser irradiation is scheduled to be performed a specified number of times based on the spot interval guide is defined as an irradiation section, and
the control unit is further configured to:
if the laser irradiation onto all the plurality of irradiation spots within the irradiation section currently undergoing treatment is not completed, shift the spot interval guide by one appropriate interval between the plurality of irradiation spots in the direction opposite to the forward direction determined by the irradiation plan each time the laser irradiation is performed; and
rotate the entire spot interval guide by an angle corresponding to an angle of the irradiation section in the forward direction determined by the irradiation plan each time the laser irradiation onto all the plurality of irradiation spots within the irradiation section currently undergoing treatment is completed.

12. The ophthalmic laser treatment device according to claim 9 or 10, wherein
a treatment target location of the patient's eye onto which the laser irradiation is performed has an arc shape or an annular shape,
an angular range of an arc-shaped region onto which the laser irradiation is scheduled to be performed a specified number of times based on the spot interval guide is defined as an irradiation section, and
the control unit is further configured to:
if the laser irradiation onto all the plurality of irradiation spots within the irradiation section currently undergoing treatment is not completed, shift the spot interval guide by one appropriate interval between the plurality of irradiation spots in the direction opposite to the forward direction determined by the irradiation plan and rotate the spot interval guide in the forward direction determined by the irradiation plan by a rotation angle of the contact lens required to shift the aiming spot for the treatment laser light to a next one of the irradiation spots, each time the laser irradiation is performed; and
rotate the entire spot interval guide in the forward direction determined by the irradiation plan to an angle corresponding to the next irradiation section each time the laser irradiation onto all the plurality of irradiation spots within the irradiation section currently undergoing treatment is completed.

13. The ophthalmic laser treatment device according to claim 11 or 12, wherein
the control unit is further configured to rotate the entire spot interval guide by an angle corresponding to a treatment completion area in the irradiation section where treatment has been done if an instruction for rotating the entire spot interval guide to an angle corresponding to the next irradiation section is input before the laser irradiation onto all the plurality of irradiation spots within the irradiation section currently undergoing treatment has not been completed.

14. The ophthalmic laser treatment device according to claim 1 or 2, wherein
the control unit is further configured to control the internal display unit to display, as the aiming guide, an outer circumferential guide along a circumference of an observation field of view by the operator through the observation optical system, and
the outer circumferential guide indicates at least one of a rotation angle of the reflective surface of the contact lens suitable for the progress of the irradiation plan or a direction toward one of the irradiation spots onto which the laser irradiation should be performed.

15. An ophthalmic laser treatment device that irradiates a tissue of a patient's eye with a treatment laser light each time an instruction for performing laser irradiation is input, the device comprising
a laser irradiation optical system that irradiates the patient's eye with treatment laser light;
an observation optical system that allows an operator to observe an observation image of the patient's eye through an eyepiece;
an internal display unit that is disposed in the observation optical system and is configured to display an image to the operator through the eyepiece; and
a control unit that is configured to perform:
an irradiation plan acquisition step of acquiring an irradiation plan for irradiating the patient's eye with a treatment laser light using a contact lens having a reflective surface that reflects the treatment laser light in a direction intersecting an optical axis; and
a spot interval guide display step of controlling the internal display unit to display a spot internal guide indicative of an appropriate interval between a plurality of irradiation spots that are scheduled to be irradiated with the treatment laser light.

## Patentansprüche

1. Augenlaserbehandlungsvorrichtung, die jedes Mal, wenn eine Anweisung zum Durchführen einer Laserbestrahlung eingegeben wird, ein Gewebe eines Auges eines Patienten mit Behandlungslaserlicht bestrahlt, wobei die Vorrichtung aufweist:
ein Laserbestrahlungsoptiksystem, das das Auge des Patienten mit Behandlungslaserlicht bestrahlt,
ein Beobachtungsoptiksystem, das es einer Bedienperson ermöglicht, ein Beobachtungsbild des Auges des Patienten durch ein Okular zu beobachten,
eine interne Anzeigeeinheit, die in dem Beobachtungsoptiksystem angeordnet ist und konfiguriert ist, um der Bedienperson durch das Okular ein Bild anzuzeigen, und
eine Steuereinheit, die konfiguriert ist, um beim Bestrahlen des Auges des Patienten mit Behandlungslaserlicht unter Verwendung einer Kontaktlinse mit einer reflektierenden Fläche, die das Behandlungslaserlicht in einer Richtung reflektiert, die eine optische Achse schneidet, durchzuführen:
einen Bestrahlungsplan-Erlangungsschritt zum Erlangen eines Bestrahlungsplans, der eine Reihenfolge zum Bestrahlen einer Mehrzahl von Bestrahlungspunkten mit dem Behandlungslaserlicht definiert, wobei die Mehrzahl von Bestrahlungspunkten mit dem Behandlungslaserlicht bestrahlt werden soll, und
einen Zielführungs-Anzeigeschritt zum Steuern der internen Anzeigeeinheit, um eine Zielführung entsprechend einem Fortschritt des Bestrahlungsplans anzuzeigen, um die Bedienperson dabei zu unterstützen, einen Zielpunkt für das Behandlungslaserlicht in einer geeigneten Position einzustellen.

2. Augenlaserbehandlungsvorrichtung gemäß Anspruch 1, wobei
die interne Anzeigeeinheit einen Anzeigebereich hat und
der Anzeigebereich ein Zentrum hat, das zu der optischen Achse des Beobachtungsoptiksystems ausgerichtet ist.

3. Augenlaserbehandlungsvorrichtung gemäß Anspruch 1 oder 2, wobei
in dem Zielführungs-Anzeigeschritt die Steuereinheit konfiguriert ist, um einen Punktintervallführungs-Anzeigeschritt durchzuführen, zum Steuern der internen Anzeigeeinheit, um als Zielführung eine Punktintervallführung entsprechend dem Fortschritt des Bestrahlungsplans anzuzeigen, und
die Punktintervallführung ein geeignetes Intervall zwischen der Mehrzahl von Bestrahlungspunkten angibt, die mit dem Behandlungslaserlicht bestrahlt werden sollen.

4. Augenlaserbehandlungsvorrichtung gemäß Anspruch 3, wobei
die Steuereinheit konfiguriert ist, um eine Größe der auf der internen Anzeigeeinheit angezeigten Punktintervallführung entsprechend einer Vergrößerung des Beobachtungsoptiksystems zu ändern.

5. Augenlaserbehandlungsvorrichtung gemäß Anspruch 3 oder 4, wobei
die Steuereinheit konfiguriert ist, um mindestens eines von Intervallen zwischen einer Mehrzahl von Indikatoren, die in der Punktintervallführung enthalten sind, so einzustellen, dass sie zu dem geeigneten Intervall zwischen der Mehrzahl von Bestrahlungspunkten passen.

6. Augenlaserbehandlungsvorrichtung gemäß irgendeinem der Ansprüche 3 bis 5, wobei
die Punktintervallführung eine Form entlang einer geeigneten Reflexionsrichtung des Behandlungslaserlichts zu einem nächsten der Bestrahlungspunkte durch die reflektierende Fläche der Kontaktlinse hat, und
die Steuereinheit ferner konfiguriert ist zum:
Ermitteln eines Winkels der Punktintervallführung zum Anzeigen auf der internen Anzeigeeinheit entsprechend dem Fortschritt des Bestrahlungsplans, und
Steuern der internen Anzeigeeinheit, um die Punktintervallführung in dem ermittelten Winkel anzuzeigen.

7. Augenlaserbehandlungsvorrichtung gemäß irgendeinem der Ansprüche 3 bis 6, wobei
die Punktintervallführung eine Mehrzahl von Indikatoren aufweist, die Enden nahe bei dem Zielpunkt für das Behandlungslaserlicht haben, und
die Steuereinheit ferner konfiguriert ist zum:
Steuern der internen Anzeigeeinheit, um die Punktintervallführung in dem Anzeigebereich der internen Anzeigeeinheit an einer Position anzuzeigen, die von dem Zielpunkt für das Behandlungslaserlicht im Abstand angeordnet ist, und
Steuern der internen Anzeigeeinheit, um die Enden der Mehrzahl von Indikatoren anzuzeigen, die entlang einer Heilungslinie anzuordnen sind, die einer Kurve einer bogenförmigen oder ringförmigen Behandlungsstelle des Auges des Patienten ähnelt.

8. Augenlaserbehandlungsvorrichtung gemäß irgendeinem der Ansprüche 3 bis 7, wobei
die Steuereinheit ferner konfiguriert ist zum:
Steuern der internen Anzeigeeinheit, um einen nächsten Zielindikator an einer Position auf der optischen Achse des von dem Laserbestrahlungsoptiksystem emittierten Behandlungslaserlichts für einen nächsten der zu dem nächsten Zielindikator auszurichtenden Bestrahlungspunkte anzuzeigen, und
Verschieben einer Position des nächsten Zielindikators in der Punktintervallführung um ein geeignetes Intervall zwischen der Mehrzahl von Bestrahlungspunkten in einer durch den Bestrahlungsplan ermittelten Vorwärtsrichtung jedes Mal, wenn eine Laserbestrahlung durchgeführt wird.

9. Augenlaserbehandlungsvorrichtung gemäß irgendeinem der Ansprüche 3 bis 8, wobei
die Steuereinheit ferner konfiguriert ist, um die Punktintervallführung jedes Mal, wenn eine Laserbestrahlung durchgeführt wird, um ein geeignetes Intervall zwischen der Mehrzahl von Bestrahlungspunkten in einer Richtung entgegengesetzt zu einer durch den Bestrahlungsplan ermittelten Vorwärtsrichtung zu verschieben.

10. Augenlaserbehandlungsvorrichtung gemäß irgendeinem der Ansprüche 3 bis 9, wobei
eine Behandlungszielstelle des Auges des Patienten, wo die Laserbestrahlung durchgeführt wird, eine Bogenform oder eine Ringform hat, und
die Steuereinheit ferner konfiguriert ist, um die gesamte Punktintervallführung zu drehen.

11. Augenlaserbehandlungsvorrichtung gemäß Anspruch 9 oder 10, wobei
eine Behandlungszielstelle des Auges des Patienten, wo die Laserbestrahlung durchgeführt wird, eine Bogenform oder eine Ringform hat,
ein Winkelbereich eines bogenförmigen Bereichs, wo die Laserbestrahlung basierend auf der Punktintervallführung eine bestimmte Anzahl von Malen durchgeführt werden soll, als Bestrahlungsabschnitt definiert ist, und
die Steuereinheit ferner konfiguriert ist zum:
Verschieben der Punktintervallführung um ein geeignetes Intervall zwischen der Mehrzahl von Bestrahlungspunkten in der Richtung entgegengesetzt zu der durch den Bestrahlungsplan ermittelten Vorwärtsrichtung jedes Mal, wenn die Laserbestrahlung durchgeführt wird, wenn die Laserbestrahlung auf alle aus der Mehrzahl von Bestrahlungspunkten innerhalb des derzeit behandelten Bestrahlungsabschnitts nicht abgeschlossen ist, und
Drehen der gesamten Punktintervallführung um einen Winkel, der mit einem Winkel des Bestrahlungsabschnitts in der durch den Bestrahlungsplan ermittelten Vorwärtsrichtung korrespondiert, jedes Mal, wenn die Laserbestrahlung auf alle aus der Mehrzahl von Bestrahlungspunkten innerhalb des derzeit behandelten Bestrahlungsabschnitts abgeschlossen ist.

12. Augenlaserbehandlungsvorrichtung gemäß Anspruch 9 oder 10, wobei
eine Behandlungszielstelle des Auges des Patienten, wo die Laserbestrahlung durchgeführt wird, eine Bogenform oder eine Ringform hat,
ein Winkelbereich eines bogenförmigen Bereichs, wo die Laserbestrahlung basierend auf der Punktintervallführung eine bestimmte Anzahl von Malen durchgeführt werden soll, als ein Bestrahlungsabschnitt definiert ist, und
die Steuereinheit ferner konfiguriert ist zum:
wenn die Laserbestrahlung auf alle aus der Mehrzahl von Bestrahlungspunkten innerhalb des derzeit behandelten Bestrahlungsabschnitts nicht abgeschlossen ist, Verschieben der Punktintervallführung um ein geeignetes Intervall zwischen der Mehrzahl von Bestrahlungspunkten in der Richtung entgegengesetzt zu der durch den Bestrahlungsplan ermittelten Vorwärtsrichtung und Drehen der Punktintervallführung in der durch den Bestrahlungsplan ermittelten Vorwärtsrichtung um einen Drehwinkel der Kontaktlinse, der erforderlich ist, um den Zielpunkt für das Behandlungslaserlicht zu einem nächsten der Bestrahlungspunkte zu verschieben, jedes Mal, wenn die Laserbestrahlung durchgeführt wird, und
Drehen der gesamten Punktintervallführung in der durch den Bestrahlungsplan ermittelten Vorwärtsrichtung um einen Winkel, der mit dem nächsten Bestrahlungsabschnitt korrespondiert, jedes Mal, wenn die Laserbestrahlung auf alle aus der Mehrzahl von Bestrahlungspunkten innerhalb des derzeit behandelten Bestrahlungsabschnitts abgeschlossen ist.

13. Augenlaserbehandlungsvorrichtung gemäß Anspruch 11 oder 12, wobei
die Steuereinheit ferner konfiguriert ist zum Drehen der gesamten Punktintervallführung um einen Winkel, der mit einem Behandlungsabschlussbereich in dem Bestrahlungsabschnitt korrespondiert, wo eine Behandlung durchgeführt wurde, wenn eine Anweisung zum Drehen der gesamten Punktintervallführung um einen Winkel, der mit dem nächsten Bestrahlungsabschnitt korrespondiert, eingegeben wird, bevor die Laserbestrahlung auf alle aus der Mehrzahl von Bestrahlungspunkten innerhalb des derzeit behandelten Bestrahlungsabschnitts abgeschlossen ist.

14. Augenlaserbehandlungsvorrichtung gemäß Anspruch 1 oder 2, wobei
die Steuereinheit ferner konfiguriert ist zum Steuern der internen Anzeigeeinheit, um als Zielführung eine Außenumfangsführung entlang eines Umfangs eines Beobachtungsfeldes durch die Bedienperson durch das Beobachtungsoptiksystem anzuzeigen, und
die Außenumfangsführung mindestens eines von einem Drehwinkel der reflektierenden Fläche der Kontaktlinse, der für den Fortschritt des Bestrahlungsplans geeignet ist, oder einer Richtung zu einem der Bestrahlungspunkte, auf den die Laserbestrahlung durchgeführt werden soll, angibt.

15. Augenlaserbehandlungsvorrichtung, die ein Gewebe eines Auges eines Patienten jedes Mal mit einem Behandlungslaserlicht bestrahlt, wenn eine Anweisung zum Durchführen einer Laserbestrahlung eingegeben wird, wobei die Vorrichtung aufweist:
ein Laserbestrahlungsoptiksystem, das das Auge des Patienten mit Behandlungslaserlicht bestrahlt,
ein Beobachtungsoptiksystem, das es einer Bedienperson ermöglicht, ein Beobachtungsbild des Auges des Patienten durch ein Okular zu beobachten,
eine interne Anzeigeeinheit, die in dem Beobachtungsoptiksystem angeordnet ist und konfiguriert ist, um der Bedienperson durch das Okular ein Bild anzuzeigen, und
eine Steuereinheit, die konfiguriert ist, um durchzuführen:
einen Bestrahlungsplan-Erlangungsschritt zum Erlangen eines Bestrahlungsplans zum Bestrahlen des Auges des Patienten mit einem Behandlungslaserlicht unter Verwenundg einer Kontaktlinse mit einer reflektierenden Fläche, die das Behandlungslaserlicht in einer Richtung reflektiert, die eine optische Achse schneidet, und
einen Punktintervallführungs-Anzeigeschritt zum Steuern der internen Anzeigeeinheit, um eine Punktintervallführung anzuzeigen, die ein geeignetes Intervall zwischen einer Mehrzahl von Bestrahlungspunkten angibt, die mit dem Behandlungslaserlicht bestrahlt werden sollen.

## Revendications

1. Dispositif de traitement ophtalmique au laser qui irradie un tissu d'un œil d'un patient avec une lumière laser de traitement chaque fois qu'une instruction d'irradiation laser est entrée, le dispositif comprenant :
un système optique d'irradiation laser qui irradie l'œil du patient avec une lumière laser de traitement ;
un système optique d'observation qui permet à un opérateur d'observer une image d'observation de l'œil du patient à travers un oculaire ;
une unité d'affichage interne qui est disposée dans le système optique d'observation et qui est configurée pour afficher une image à l'opérateur à travers l'oculaire ; et
une unité de commande qui est configurée pour exécuter, lors de l'irradiation de l'œil du patient avec une lumière laser de traitement en utilisant une lentille de contact ayant une surface réfléchissante qui réfléchit la lumière laser de traitement dans une direction coupant un axe optique :
une étape d'acquisition de plan d'irradiation consistant à acquérir un plan d'irradiation qui définit un ordre d'irradiation d'une pluralité de points d'irradiation avec la lumière laser de traitement, la pluralité de points d'irradiation étant prévus pour être irradiés avec la lumière laser de traitement ; et
une étape d'affichage de guide de visée consistant à commander l'unité d'affichage interne pour afficher un guide de visée en fonction d'une progression du plan d'irradiation afin d'aider l'opérateur à ajuster un point de visée pour la lumière laser de traitement dans une position appropriée.

2. Dispositif de traitement ophtalmique au laser selon la revendication **1,** dans lequel
l'unité d'affichage interne comporte une zone d'affichage, et
la zone d'affichage comporte un centre qui est aligné avec l'axe optique du système optique d'observation.

3. Dispositif de traitement ophtalmique au laser selon la revendication 1 ou 2, dans lequel
dans l'étape d'affichage de guide de visée, l'unité de commande est configurée pour exécuter une étape d'affichage de guide d'intervalle de points consistant à commander l'unité d'affichage interne pour afficher, en tant que guide de visée, un guide d'intervalle de points en fonction de la progression du plan d'irradiation, et
le guide d'intervalle de points indique un intervalle approprié entre la pluralité de points d'irradiation qui sont prévus pour être irradiés avec la lumière laser de traitement.

4. Dispositif de traitement ophtalmique au laser selon la revendication 3, dans lequel
l'unité de commande est configurée pour modifier une taille du guide d'intervalle de points affiché sur l'unité d'affichage interne en fonction d'un grossissement du système optique d'observation.

5. Dispositif de traitement ophtalmique au laser selon la revendication 3 ou 4, dans lequel
l'unité de commande est configurée pour ajuster au moins un parmi des intervalles entre une pluralité d'indicateurs inclus dans le guide d'intervalle de points afin de faire correspondre l'intervalle approprié entre la pluralité de points d'irradiation.

6. Dispositif de traitement ophtalmique au laser selon l'une quelconque des revendications 3 à 5, dans lequel
le guide d'intervalle de points a une forme le long d'une direction appropriée de réflexion de la lumière laser de traitement vers un suivant parmi les points d'irradiation par la surface réfléchissante de la lentille de contact, et
l'unité de commande est en outre configurée pour :
déterminer un angle du guide d'intervalle de points à afficher sur l'unité d'affichage interne en fonction de la progression du plan d'irradiation ; et
commander l'unité d'affichage interne pour afficher le guide d'intervalle de points à l'angle déterminé.

7. Dispositif de traitement ophtalmique au laser selon l'une quelconque des revendications 3 à 6, dans lequel
le guide d'intervalle de points comprend une pluralité d'indicateurs ayant des extrémités proches du point de visée pour la lumière laser de traitement, et
l'unité de commande est en outre configurée pour :
commander l'unité d'affichage interne pour afficher le guide d'intervalle de points dans la zone d'affichage de l'unité d'affichage interne à une position espacée du point de visée pour la lumière laser de traitement ; et
commander l'unité d'affichage interne pour afficher les extrémités de la pluralité d'indicateurs à disposer le long d'une ligne de traitement similaire à une courbe d'un emplacement de traitement en forme d'arc ou en forme annulaire de l'œil du patient.

8. Dispositif de traitement ophtalmique au laser selon l'une quelconque des revendications 3 à 7, dans lequel
l'unité de commande est en outre configurée pour :
commander l'unité d'affichage interne pour afficher un indicateur de visée suivant à une position sur l'axe optique de la lumière laser de traitement émise par le système optique d'irradiation laser pour un suivant des points d'irradiation à aligner avec l'indicateur de visée suivant ; et
décaler une position de l'indicateur de visée suivant dans le guide d'intervalle de points d'un intervalle approprié entre la pluralité de points d'irradiation dans une direction vers l'avant déterminée par le plan d'irradiation chaque fois qu'une irradiation laser est effectuée.

9. Dispositif de traitement ophtalmique au laser selon l'une quelconque des revendications 3 à 8, dans lequel
l'unité de commande est en outre configurée pour décaler le guide d'intervalle de points d'un intervalle approprié entre la pluralité de points d'irradiation dans une direction opposée à une direction vers l'avant déterminée par le plan d'irradiation chaque fois qu'une irradiation laser est effectuée.

10. Dispositif de traitement ophtalmique au laser selon l'une quelconque des revendications 3 à 9, dans lequel
un emplacement cible de traitement de l'œil du patient sur lequel l'irradiation laser est effectuée a une forme d'arc ou une forme annulaire, et
l'unité de commande est en outre configurée pour faire tourner l'ensemble du guide d'intervalle de points.

11. Dispositif de traitement ophtalmique au laser selon la revendication 9 ou 10, dans lequel
un emplacement cible de traitement de l'œil du patient sur lequel l'irradiation laser est effectuée a une forme d'arc ou une forme annulaire,
une plage angulaire d'une région en forme d'arc sur laquelle l'irradiation laser est prévue pour être effectuée un nombre spécifié de fois sur la base du guide d'intervalle de points est définie comme une section d'irradiation, et
l'unité de commande est en outre configurée pour :
si l'irradiation laser sur tous parmi la pluralité de points d'irradiation dans la section d'irradiation actuellement en cours de traitement n'est pas terminée, décaler le guide d'intervalle de points d'un intervalle approprié entre la pluralité de points d'irradiation dans la direction opposée à la direction vers l'avant déterminée par le plan d'irradiation chaque fois que l'irradiation laser est effectuée ; et
faire tourner l'ensemble du guide d'intervalle de points d'un angle correspondant à un angle de la section d'irradiation dans la direction vers l'avant déterminée par le plan d'irradiation chaque fois que l'irradiation laser sur tous parmi la pluralité de points d'irradiation dans la section d'irradiation actuellement en cours de traitement est terminée.

12. Dispositif de traitement ophtalmique au laser selon la revendication 9 ou 10, dans lequel
un emplacement cible de traitement de l'œil du patient sur lequel l'irradiation laser est effectuée a une forme d'arc ou une forme annulaire,
une plage angulaire d'une région en forme d'arc sur laquelle l'irradiation laser doit être effectuée un nombre spécifié de fois sur la base du guide d'intervalle de points est définie comme une section d'irradiation, et
l'unité de commande est en outre configurée pour :
si l'irradiation laser sur tous parmi la pluralité de points d'irradiation dans la section d'irradiation actuellement en cours de traitement n'est pas terminée, décaler le guide d'intervalle de points d'un intervalle approprié entre la pluralité de points d'irradiation dans la direction opposée à la direction vers l'avant déterminée par le plan d'irradiation et faire tourner le guide d'intervalle de points dans la direction vers l'avant déterminée par le plan d'irradiation d'un angle de rotation de la lentille de contact nécessaire pour décaler le point de visée de la lumière laser de traitement vers un suivant parmi les points d'irradiation, chaque fois que l'irradiation laser est effectuée ; et
faire tourner l'ensemble du guide d'intervalle de points dans la direction vers l'avant déterminée par le plan d'irradiation d'un angle correspondant à la section d'irradiation suivante chaque fois que l'irradiation laser sur tous parmi la pluralité de points d'irradiation dans la section d'irradiation actuellement en cours de traitement est terminée.

13. Dispositif de traitement ophtalmique au laser selon la revendication 11 ou 12, dans lequel
l'unité de commande est en outre configurée pour faire tourner l'ensemble du guide d'intervalle de points d'un angle correspondant à une zone d'achèvement de traitement dans la section d'irradiation où le traitement a été effectué si une instruction pour faire tourner l'ensemble du guide d'intervalle de points d'un angle correspondant à la section d'irradiation suivante est entrée avant que l'irradiation laser sur tous parmi la pluralité de points d'irradiation dans la section d'irradiation en cours de traitement ne soit terminée.

14. Dispositif de traitement ophtalmique au laser selon la revendication 1 ou 2, dans lequel
l'unité de commande est en outre configurée pour commander l'unité d'affichage interne afin d'afficher, en tant que guide de visée, un guide circonférentiel extérieur le long d'une circonférence d'un champ de vision d'observation par l'opérateur à travers le système optique d'observation, et
le guide circonférentiel extérieur indique au moins l'un parmi un angle de rotation de la surface réfléchissante de la lentille de contact adapté à la progression du plan d'irradiation ou une direction vers l'un des points d'irradiation sur lesquels l'irradiation laser doit être effectuée.

15. Dispositif de traitement ophtalmique au laser qui irradie un tissu d'un œil d'un patient avec une lumière laser de traitement chaque fois qu'une instruction d'exécution d'une irradiation laser est entrée, le dispositif comprenant :
un système optique d'irradiation laser qui irradie l'œil du patient avec une lumière laser de traitement ;
un système optique d'observation qui permet à un opérateur d'observer une image d'observation de l'œil du patient à travers un oculaire ;
une unité d'affichage interne qui est disposée dans le système optique d'observation et qui est configurée pour afficher une image à l'opérateur à travers l'oculaire ; et
une unité de commande qui est configurée pour exécuter :
une étape d'acquisition de plan d'irradiation consistant à acquérir un plan d'irradiation pour irradier l'œil du patient avec une lumière laser de traitement en utilisant une lentille de contact ayant une surface réfléchissante qui réfléchit la lumière laser de traitement dans une direction coupant un axe optique ; et
une étape d'affichage de guide d'intervalle de points consistant à commander l'unité d'affichage interne pour afficher un guide d'intervalle de points indiquant un intervalle approprié entre une pluralité de points d'irradiation qui sont prévus pour être irradiés avec la lumière laser de traitement.
